# EUROPEAN PATENT APPLICATION

(11) **EP 0 594 868 A1**
(43) Date of publication of application: **04.05.1994**
(21) Application number: 93910344.6
(22) Date of filing: 14.05.1993
(51) Int. Cl.: C12N 15/52

(54) **GENE WHICH CODES FOR EICOSAPENTAENOIC ACID SYNTHETASE GROUP AND PROCESS FOR PRODUCING EICOSAPENTAENOIC ACID**

(30) Priority: 15.05.1992 JP 147945/92
(71) Applicant: SAGAMI CHEMICAL RESEARCH CENTER, Tokyo 100 (JP)
(72) Inventor: YAZAWA, Kazunaga, Sagamihara-shi, Kanagawa 228 (JP); YAMADA, Akiko, Sagamihara-shi, Kanagawa 229 (JP); KATO, Seishi, Sagamihara-shi, Kanagawa 228 (JP); KONDO, Kiyosi, Yamato-shi, Kanagawa 242 (JP)
(74) Representative: Stuart, Ian Alexander
(86) International application number: JP9300641
(87) International publication number: WO9323545

(57) **Abstract**

An advantageous process for producing eicosapentaenoic acid (EPA) useful as medicine, pesticide, food, feed and the like by the genetic recombination process by taking a gene coding for an EPA biosynthetase group from a microorganism. EPA is produced by taking a gene coding for an EPA biosynthetase group, preparing a plasmid by linking the gene with a vector, transforming *Escherichia coli* with the plasmid, and culturing the transformed *Escherichia coli*.

## Description

### FIELD OF THE ART

The present invention relates to genes coding for eicosapentaenoic acid (designated EPA hereinafter) synthesizing enzymes, plasmids containing these genes, microorganisms transformed with the plasmid, and a process for production of eicosapentaenoic acid using the microorganism. The EPA is useful as a starting material for pharmaceuticals, foods, feeds and the like.

### BACKGROUND ART

Polyunsaturated fatty acids represented by eicosapentaenoic acid (EPA) play an important roll as a component of a biomembrane. So far, the following pharmacological actions of EPA are known. (1) Platelet coagulation inhibitory action (thrombolytic action), (2) blood neutral fat-lowering action, (3) actions for lowering blood VLDL-cholesterol and LDL-cholesterol and increasing HDL-cholesterol (anti-arterial sclerosis action), (4) blood viscosity-lowering action, (5) blood pressure lowering action, (7) anti-inflammatory action, (8) anti-tumor action.

In addition, EPA is a substrate for biosynthesis of prostaglandins and exhibits an essential function in vivo in higher mammals including humans. In particular EPA is important as a substrate for production of three types of prostaglandins, has platelet coagulation inhibitory action and is studied for applications to treatment and prophylactic agents for thrombosis. In addition EPA has especially high activity for lowering plasma cholesterol level among polyunsaturated fatty acids having said action, and is highly effective in comparison with linoleic acid and the like usually contained in plant oil. Additionally, EPA is known as an essential nutrient for fish.

Thus, epidemiological research by Dyerberg, Denmark (Am. J. Clin. Nutur, 28, 959, 1975) showed a possibility for use as health foods or pharmaceuticals on the basis of thrombosis inhibitory action or lipid-lowering actions of EPA. However, as can be seen from its chemical structure, the chemical synthesis of EPA is very difficult. Accordingly, in Japan, it is recommended to eat blue back fish such as sardine, salmon, saury, and the like.

At present, most commercially available EPA products are fractionation products from fish oil obtained by a boiling process, and the EPA content thereof is about 10 to 30%. Fish oil extracted by a boiling process is a mixed glyceride containing various kind of fatty acids as component fatty acids, and not only is isolation and purification of each component difficult, but also since EPA is a polyunsaturated fatty acid having 20 carbon atoms and five double bonds all of which are cis-type, EPA is also a unstable and highly oxidation-liable fatty acid. Therefore, it is necessary for EPA to be concentrated from fish oil with prevention of oxygen, light, heat and the like. In addition, since fish oil contains various fatty acids in addition to EPA, their fractionation is difficult. Moreover, although various organic solvents used for fractionation of EPA are eliminated under a reduced pressure, complete elimination of the organic solvents is difficult from a technical and economical point of view.

Most EPA preparations used for pharmaceuticals are those having at least 90% EPA concentration, produced by extracting fish oil by various processes, hydrolysing the fish oil enzymatically or under an alkaline condition to generate free fatty acids, optionally converting the free fatty acids to corresponding methyl or ethyl esters, and further purifying by fractional crystallization at a low temperature, applying a urea-addition method, distillation under a reduced pressure, reverse phase chromatography, or the like. However, since these processes use many organic solvents and heating to near 200°C, it is possible that an EPA concentrate obtained by using such process may be denaturated by residual organic solvent, and polymerization, isomerization or oxidation of EPA. Moreover, where a fish oil is used as a starting material for production of EPA, it is difficult to eliminate docosenoic acid or the like which is considered to be a cause of cardiodiseases, and therefore problems remain in the use for health foods, pharmaceuticals or the like.

On the other hand, recently, processes for production of EPA using microorganisms such as chlorella, unicellular algae Monodus, Eugrena or Diatomaceae have been studied in place of extraction methods from fish oil having drawbacks such as residual fish odor due to incomplete purification and concentration, and the production of EPA using microorganisms has been considered. Recently, fungi producing EPA were reported by Gellerman and Schlenk (J. L. Gellerman and H. Schlenk, BBA, 573, 23, 1979) and Yamada et al. (Meeting of The society of Fermentation Technology, Japan, 1986).

The present inventors sought marine bacteria having an ability to produce EPA to find a new fermentation process for production of EPA using bacteria from which genes can be easily obtained, and which can be cultured in a short time and be easily controlled, and as a result, the present inventors found a new bacteria belonging to the genus Pseudomonas, Alteromonas or Shewanella (K. Yazawa et al., J. Biochem., 103, 5 (1988); K. Yazawa et al., Nippon Suisan Gakkai shi, 54, 1835 (1988)).

It has been suggested that biosynthesis of polyunsaturated fatty acids including EPA works by site-specific aerobic unsaturation of corresponding saturated fatty acids (for example, R. Jeffcoat and A. T. James (1984) in: S. Numa (Ed.): Fatty acid metabolism and its regulation, Elsevier, Amsterdam, pp85 - 112). However, there is no report relating to biosynthetic enzymes which participate in EPA synthesis, and gene coding therefor.

### DISCLOSURE OF THE INVENTION

Generally, the ability of a wild strain to produce a useful substance is low, and therefore where it is intended that the ability of the microorganism is industrially used, an improvement, i.e., an increase of productivity of the microorganism is carried out by various methods. The present inventors intended to carry out research for increasing an EPA productivity by finding genes not described in literature for EPA biosynthetic enzymes using gene recombination techniques and introducing the same into another organism, to impart an EPA biosynthesis ability to an organism not having an EPA biosynthesis ability, and eventually to establish an advantageous process for production of EPA.

Accordingly, the present invention provides genes for EPA biosynthetic enzymes, expression plasmids containing said genes, organisms transformed with said plasmid, and a process for production of EPA using said organism.

### BRIEF EXPLANATION OF THE DRAWINGS

Figure 1 represents the structure of the plasmid pEPA containing a group of the present genes.

Fig. 2 represents a restriction enzyme map of a DNA fragment containing a group of the present genes.

### Best Mode for Carrying Out the Invention

According to the present invention, an EPA producing strain is constructed by extracting DNA from a microorganism having an ability to produce EPA (microbial origin of genes), cutting this DNA with restriction enzymes to excise genes coded for a group of EPA biosynthesis enzymes, introducing the genes into an appropriate vector to construct an expression plasmid, and transforming a host organism with the plasmid to construct an EPA producing strain. EPA can be produced by this organism.

### Gene source

Although organisms which can be used as a gene source according to the present invention are not limited to specific genera, species or strains, usually, microorganisms classified to the genus Pseudomonas, Alteromonas, Shewanella or the like can be used. These microorganisms can be easily obtained from official or public depository institutes for microorganisms.

As examples of microorganisms belonging to the genus Pseudomonas, Pseudomonas putrefaciens SCRC-2181 (FERM BP-2917), SCRC-2201 (FERM BP-2916), SCRC-2271 (FERM BP-2195), SCRC-2341 (FERM BP-2918), SCRC-2451 (FERM BP-2919), SCRC-2642 (FERM BP-2920), SCRC-2792 (FERM BP-2921), SCRC-2878 (FERM BP-1623), SCRC-3011 (FERM BP-2913), and SCRC-3022 (FERM BP-2914) may be mentioned.

As example of microorganisms belonging to the genus Alteromonas, Alteromonas putrefaciens SCRC-2871 (FERM BP-1624) and Alteromonas putrefaciens subspecies sagamifaciens SCRC-1162 (FERM BP-1626) may be mentioned.

As an example of microorganism belonging to the genus Shewanella, Shewanella putrefaciens SCRC-2874 (FERM BP-1625) may be mentioned.

### Cloning of Genes Coding for a Group of EPA Biosynthesis Enzymes and Construction of Expression Plasmid

In the present invention, the case wherein Shewanella putrefaciens SCRC-2874 (FERM BP-1625) was used as a source of genes for a group of EPA biosynthesis enzymes is concretely explained. However, as described above, various EPA producing microorganisms can be similarly used as a gene source. A process for cloning genes is described in the Examples of the present invention.

According to the present invention, EPA producing strains can be artificially generated by transforming an heterogeneous host such as Escherichia coli or a homogeneous host such as Shewanella, or further yeast, fungus or the like. In addition, EPA producing plants can be generated by introducing the present genes into a higher plant such as soybean, sunflower, rape, or the like. As a region for expression of a group of EPA biosynthesis genes, although a control region natively accompanying these enzymes can be used, it is advantageous to prepare another promoter/operator system for increasing an amount of expression or allowing inducible expression. Where E. coli is used as a host, as the promoter/operator system, trp, tac, lavUV5, P_{L}, P_{R} or 1pp promoter/operator system and the like can be used, and as an SD sequence, an SD sequence of trp leader peptide, lacZ, metapyrocatechase or cII gene can be used. In addition, a transcriptional terminator, for example, rrnBT₁T₂ terminator of E. coli ribosome gene or the like can be provided downstream of a coding region. In addition, for expression of the above-mentioned gene, a host/vector system of Saccharomyces cerevisiae can be used, wherein as a promoter there can be used a promoter of alcohol dehydrogenase gene, a promoter of acid phosphatase gene, a promoter of glycelaldehyde-3-phosphate dehydrogenase gene, a promoter of enolase gene or the like can be used, wherein a plasmid preferably contains a sequence for replication in yeast, and an auxotrophic marker as a selectable maker for selection of yeast containing said plasmid, such as Leu, Trp, His or the like.

For introduction of the genes into a plant, there are a method using a vector, and a direct introduction method. As vectors, Ti plasmid; DNA viruses such as cauliflower mosaic virus (CaMV), Geminivirus, cassava roten virus, tomato golden mosaic virus, and the like; and RNA viruses such as brome mosaic virus (BMV), and tobacco mosaic virus (TMV) can be used, wherein as a promoter, 35S promoter of CaMV or the like may be mentioned. On the other hand, as direct protoplast introduction methods there can be mentioned a calcium phosphate method, polyethylene glycol method, microinjection, electroporation, liposome method and the like. Moreover, as a direct plant cell introduction method, there may be mentioned a particle gun method.

In addition, a fatty acid composition in a host plant can be changed by using a part of the group of the genes.

Note, that generally, an amount of expression of a particular protein in E. coli is affected by the number of copies of the genes, an efficiency of transcription, stability of mRNA, efficiency of translation, and stability of the protein. To modify control regions such as a promoter, SD region, terminator and the like, a smaller plasmid can be easily treated. The number of copies of the genes depends on the size of the plasmid, and there is a tendency for the plasmid to be smaller as the number of copies increases. For this purpose, a smaller plasmid can be obtained by inserting a DNA fragment containing a group of genes for EPA biosynthesis described in the Examples of the present invention into a plasmid, and repeating subcloning of the plasmid to cut off unnecessary portions of the gene DNA fragment. Smaller EPA biosynthesis enzyme genes thus obtained are included in the present invention. In addition, to enhance stability or activity of the enzyme, the nucleotide sequence of the gene (amino acid sequence) can be modified by a known technique, and the present invention includes such a modified gene.

As a host E. coli of the present invention, any strain derived from E. coli K12 may be used. For example, JM83, JM101, JM103, JM105, JM109, RR1, RB791, W3110, C600, HB101, DH1, AG1, NM554 or the like can be used. As a yeast host, AH22, DC5, D-13-1A, YNN144 or the like can be mentioned.

Note, that a group of the enzymes encoded by the present genes can convert higher fatty acids synthesized by a biosynthetic system natively possessed by the host organism to eicosapentaenoic acid.

In practicing the present invention, an organism such as a microorganism transformed with the present genes is cultured in a medium according to a conventional procedure to obtain microbial cells. In this case, for example, a medium having a composition shown in Table 1 is prepared.

**Table 1**

| | |
|---|---|
| Yeast extract | 0.5% |
| Pepton | 1.0% |
| Sea water | 1/2 concentration |
| pH 7.0 | |

From the microbial cells thus obtained, EPA can be obtained according to a conventional procedure such as extraction with an organic solvent. The details are described in the following Examples.

### EXAMPLES

Next, the present invention is explained in more detail by way of Examples.

### Example 1-1 Preparation of Genomic DNA Containing Genes Coding for a Group of PEA Biosynthesis Enzymes

Shewanella putrefaciens SCRC-2874 (FERM BP-1625) was inoculated in 125 ml of a medium (1% pepton, 0.5% yeast extract, 1/2 concentration artificial sea water), and cultured at 15°C for 18 hours with shaking (OD610 = 8.6). The resulting microbial cells were washed once with 1M NaCl, and suspended in 20 ml of 1M NaCl. The suspension was allowed to stand at 55°C for 30 minutes, 20 ml of 0.1M EDTA was added thereto, and after being allowed to stand at 55°C for 15 minutes, the suspension was centrifuged at 10,000 rpm for 10 minutes. To the precipitate, was added 10 ml of TES buffer (1 mM EDTA, 0.1 mM NaCl, 10 mM Tris-HCl, pH 8.0) containing 100 mg of lysozyme, and the cells were suspended. After being allowed to stand at 37°C for an hour, 1 ml of 10% SDS was added to the suspension, which was then allowed to stand at 60°C for an hour. 11 ml of neutralized phenol was added to the suspension, which was then gently shaken for 5 minutes and centrifuged at 6,500 rpm for 5 minutes to obtain the upper layer. 20 ml of ethanol was added to the layer and the mixture was gently shaken. Precipitated DNA was wound onto a glass bar, washed in ethanol, dissolved in 10 ml of TES buffer, and the solution was allowed to stand at 4°C overnight. 0.5 mg of RNase A was added to the solution, which was then gently shaken at 37°C for 3 hours, and after 1 mg of proteinse K was added thereto, the solution was further shaken for 4.5 hours; 5 ml each of neutralized phenol and chloroform were gradually added to the mixture, which was then gently shaken for 5 minutes and centrifuged to recover the upper layer. 10 ml of chloroform was added to the layer, which was then gently shaken and centrifuged to obtain the upper layer. 20 ml of ethanol was added to the layer, which was then gently shaken, and precipitated DNA was wound onto a glass bar. The DNA was washed in ethanol, and dissolved in 3 ml of TES buffer. An amount of DNA thus obtained was about 2.8 mg. Next, 200 µg of the DNA was partially digested with restriction enzyme Sau3A1 and subjected to electrophoresis on 0.3% agarose, and DNA fragments larger than about 20 Kb were isolated by electroelusion. The DNA fragments were extracted with phenol/chloroform, and precipitated with ethanol, and the precipitate was dissolved in 500 µl of TE buffer (1 mM EDTA, 10 mM Tris-HCl, pH 7.4).

### Example 1-2 Insertion of Chromosomal DNA Fragments into Vector

As a vector, cosmid pWE15 (STRATAGENE) was used. 10 µg of pWE15 was completely digested with restriction enzyme BamHI, treated with calf intestine alkaline phosphatase at 37°C for an hour, extracted with phenol/chloroform, and precipitated with ethanol, and the precipitate was dissolved in 10 µl of TE buffer. 1.5 µg of the vector DNA thus obtained was mixed with 1 µg of the chromosomal DNA prepared in Example 1 and partially digested with restriction enzyme Sau3A1, and these DNA were ligated using T4 DNA ligase at 26°C for 10 minutes. One fourth of the reaction mixture was packaged according to a conventional method to form phage, which was then infected to E. coli K12/AG-1.

### Example 1-3 Screening of Recombinant EPA Producing Strain

The E. coli suspension infected with the phage of Example 1-2 was plated on an LB agar medium (trypton 1%, yeast extract 0.5%, NaCl 1%, Agar 2%) containing 50 µg/ml ampicillin and cultured at 37°C overnight. The developed colony was inoculated in 1.5 ml of LB medium containing 50 µg/ml ampicillin, and cultured at 25°C for 1 to 7 days with shaking. The culture was centrifuged to collect the microbial cells, and after removing the medium, the cells were suspended in 0.5 ml of methanol saturated with hydrogen chloride. The cell suspension was sealed and incubated at 80°C for an hour to methyl-esterify fatty acids. After allowing the suspension to cool, it was extracted three times with 0.3 ml hexane, the hexane layer was dried and the residue was dissolved in 20 µl of methanol. 2 µl of the solution was spotted on a silica gel plate which was then developed three times with a developing solvent of hexane and ether 19:1, dried in air, and colored with iodine.

In this way, as a result of tests of about 390 recombinant clones, one clone showing a thin layer chromatography spot at the same position as a standard methyl ester of EPA was obtained. From the clone, cosmid was extracted using an alkali/SDS method. This cosmid was designated as pEPA. The pEPA is a cosmid wherein a San3A1 fragment of about 37 Kbp was inserted into BamHI site of pWE15.

### Example 1-4 Preparation of Restriction Enzyme Map of pEPA

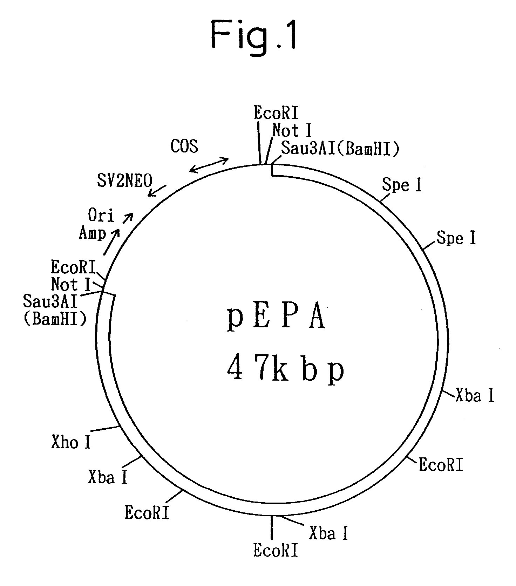

Comsmid pEPA was prepared from transformant AG-1/pEPA. The pEPA was cleaved with various restriction enzymes, and a restriction enzyme map was prepared (Fig. 1).

### Example 1-5 Analysis of Sequence

An entire nucleotide sequence of a Sau3A1-Sau3A1 fragment containing a genomic DNA insert in the cosmid pEPA is shown in SEQ ID NO: 1. In the nucleotide sequence 8 open reading frames, ORFs 2 to 9, can be identified, and these nucleotide sequences and corresponding amino acid sequences are shown in SEQ ID NOs: 2 to 9 respectively. The relationship between the entire nucleotide sequence (SEQ ID NO: 1) and ORFs 2 to 9 (SEQ ID NOs: 2 to 9, respectively) is shown in Table 2.

**Table 2**

| SEQ ID NO | Length of sequence | Positions on SEQ ID NO: 1 |
|---|---|---|
| 2 | 1983 | 6121- 8103 |
| 3 | 2910 | 9681-12590 |
| 4 | 4902 | 13905-18806 |
| 5 | 3174 | 18997-22170 |
| 6 | 2340 | 22173-24512 |
| 7 | 5637 | 24515-30151 |
| 8 | 1629 | 30727-32355 |
| 9 | 1596 | 32750-34345 |

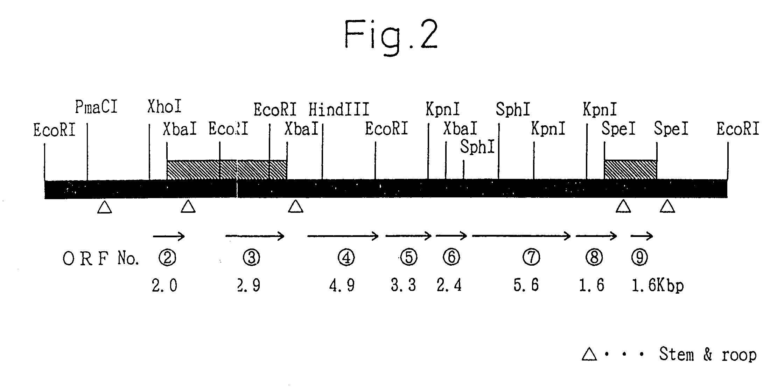

A restriction enzyme map of the above-mentioned genomic DNA insert and positions of the ORFs 2 to 9 on the map are shown in Fig. 2. In Fig. 2, the symbols △ show the sites at which mRNA forms a hair pin structure (stem and roop structure). The pEPA was cleaved with different restriction enzymes to remove a part of the sequence of SEQ ID NO: 1, and the cleaved pEPA was religated, and used to transform E. coli. Next, the transformants were tested for the productivity of eicosapentaenoic acid. As a result, in the case where the parts shown by hatching in Fig. 2 were deleted, the production of eicosapentaenoic acid was not observed. Accordingly, it is considered that for the production of eicosapentaenoic acid in E. coli, at least one of ORFs 2 and 3, and at least one of ORFs 8 and 9 is essential.

By comparison of the amino acid sequences of various ORFs with known amino acid sequences, it was found that two regions in the ORF 4, one region in the ORF 5, and 2 regions in the ORF 7 have homology to some extent with amino acid sequences of enzymes participating in fatty acid synthesis. The results are shown in Table 3.

**Table 3**

| SEQ ID NO (ORF No.) | Position of amino acid sequence | Similar enzyme and position | References |
|---|---|---|---|
| 4 | 668(Leu)-930(Leu) | MalonylCoA-ACP transferase 56(Leu)-309(Leu) | (1) |
| 4 | 189(Phe)-424(His) | Fatty acid synthetase 120(Phe)-350(His) | (2) |
| 4 | 200(Ser)-483(Leu) | Fatty acid synthetase (3-ketoacyl-ACP synthetase domain) 137(Ala)-406(Asp) | (3) |
| 4 | 454(Ser)-585(Gln) | 3-Ketoacyl-ACP synthetase 137(Ala)-406(Asp) | (4) |
| 5 | 563(Phe)-694(Gly) | 2-Oxoacylreductase 1470(Leu)-1604(Gly) | (5) |
| 7 | 205(Ala)-442(Lys) | 3-Ketoacyl-ACP synthetase 187(Ala)-416(Asn) | (6) |
| 7 | 1373(Thr)-1547(Val) | 3-Hydroxydecanoyl-ACP dehydratase 29(Leu)-163(Val) | (7) |
| References (1) Magnuson K. et al., FEBS Lett. (1992) 299:262-266 (2) Kameda K. et al., J. Biol. Chem. (1991) 266:419-426 (3) Huang W. Y. et al., Arch. Biochem. Biophys. (1989) 270:92-98 (4) Kauppinen S. et al., Carlsberg Res. Commun. (1988) 53:357-370 (5) Beck J. et al., Eur. J. Biochem. (1990) 192:487-498 (6) Siggaard-Andersen M. et al., Proc. Natl. Acad.Sci. U.S.A. (1991) 88:4114-4118 (7) Cronan Jr. J. E. et al., J. Biol. Chem. (1988) 263:4641-4646 | | | |

Accordingly, the present invention provides genes coding for amino acid sequences shown in SEQ ID NOs 2 to 9, and further provides DNAs coding for amino acid sequence region having a homology with amino acid sequences of known enzymes or amino acid sequences containing the same. The present invention further includes nucleotide sequences which can hybridize with one of the nucleotide sequences shown in SEQ ID NOs: 1 to 9 or a part thereof and coding for a useful activity, and polypeptides encoded by one of these nucleotide sequences and having a useful activity.

### Example 2 Production of EPA by Transformant GA-1/pEPA

Transformant AG-1/pEPA was inoculated in 100 ml of LB medium containing 50 µl/ml ampicillin, and cultured at 25°C for 48 hours. The cells were obtained by centrifugation, washed once and suspended in 2 ml of pure water, and the suspension was extracted three times with 12 ml of a solvent composed of chloroform and methanol 2:1. The solvent layer was dried, and the residue was dissolved in 1.5 ml of methanol saturated with hydrogen chloride, and the solution was sealed and incubated at 80°C for an hour to methyl-esterify fatty acids. After allowing the solution to cool, it was extracted three times with 2 ml of hexane, and after the hexane layer was dried, the residue was dissolved in 20 µl of methanol. A part of the solution was analyzed by gas chromatography. As a result, a peak of EPA was observed, and a ratio of EPA relating to total fatty acid esters was calculated as about 1.36% from the area of peaks. The amount of EPA per culture volume was about 0.5 mg/l. The ester mixture thus obtained was spotted on a silver nitrate silica gel plate, which was then developed by a solvent composed of hexane and ether at a ratio of 3:1. The plate was colored with fluorescein and ultraviolet light, the spot of the ester of the polyunsaturated fatty acid was scraped off, 1.8 ml of methanol and 0.2 ml of 10% NaCl were added thereto, and the mixture was shaken at room temperature for 30 minutes. The mixture was extracted three times with 2 ml of hexane, the hexane layer was dried, the residue was dissolved in 40 µl of hexane, and GC-MS analysis was carried out. As a result, molecular weight of the substance of the desired peak on the chromatography was 316, and the peak of the fragment conformed to that of an authentic sample, and the substance was identified as EPA. Note that MS fragment peaks were as follow:
- Mass:: 316(M⁺), 287, 273, 262, 247, 234, 220, 201, 180, 161, 148, 133, 119, 108, 93, 79, 67, 55, 41, 28.

### Example 3 Production of EPA by Transformant JM109/pEPA

According to a conventional procedure the cosmid pEPA was used to transform E. coli K12/JM109. JM109/pEPA (FERM BP-4257) was obtained by selection using an LB agar medium containing 50 µl/ml ampicillin. According to the same procedure as described in Example 2, extraction of lipid from the cells, methyl-esterification and analysis by gas chromatography were carried out and a peak of EPA was detected. A ratio of EPA relating to a total of esters of fatty acids was calculated as about 1.43% on the basis of the area of peaks. An amount of EPA per culture volume was about 0.6 mg/l.

Reference to deposited microorganisms and the depository authority under the Rule 13-2
Depository authority: National Institute of Bioscience and Human-Technology Agency of Industrial Science and Technology
Deposition Number and Deposition Date:
May 14, 1992 FERM BP-4257

SEQUENCE LISTING

SEQ ID NO: 1
SEQUENCE LENGTH: 37913
SEQUENCE TYPE: Nucleic acid
STRANDNESS: Double strand
TOPOLOGY: Linear
MOLECULE TYPE: Genomic DNA
ORIGINAL SOURCE: Shewanella putrefaciens SCRC-2874
(FERM BP-1625)

SEQUENCE
GATCTCTTAC AAAGAAACTA TCTCAATGTG AATTTAACCT TAATTCCGTT TAATTACGGC 60
CTGATAGAGC ATCACCCAAT CAGCCATAAA ACTGTAAAGT GGGTACTCAA AGGTGGCTGG 120
GCGATTCTTC TCAAATACAA AGTGCCCAAC CCAAGCAAAT CCATATCCGA TAACAGGTAA 180
AAGTAGCAAT AAACCCCAGC GCTGAGTTAG TAATACATAA GCGAATAATA GGATCACTAA 240
ACTACTGCCG AAATAGTGTA ATATTCGACA GTTTCTATGC TGATGTTGAG ATAAATAAAA 300
AGGGTAAAAT TCAGCAAAAG AACGATAGCG CTTACTCATT ACTCACACCT CGGTAAAAAA 360
GCAACTCGCC ATTAACTTGG CCAATCGTCA GTTGTTCTAT CGTCTCAAAG TTATGCCGAC 420
TAAATAACTC TATATGTGCA TTATGATTAG CAAAAACTCC GATACCATCA AGATGAAGTT 480
GTTCATCACA CCAACTCAAA ACTGCGTCGA TAAGCTTACT GCCATAGCCC TTGCCTTGCT 540
CCACATTTGC GATAGCAATA AACTGTAAAA TGCCACATTG GCCACTTGGT AAGCTCTCTA 600
TAATCTGATT TTCTTTGTTA ATAAGTGCCT GAGTTGAATA CCAACCAGTA CTTAACAACA 660
TCTTTAAACG CCAATGCCAA AAACGCGCTT CACCTAAGGG AACCTGCTGA GTCACTATGC 720
AGGCTACGCC TATCAATCTA TCCCCAACGA ACATACCAAT AAGTGCTTGC TCCTGTTGCC 780
AGAGCTCATT GAGTTCTTCT CGAATAGCCC CGCGAAGCTT TTGCTCATAC TGCGCTTGAT 840
CACCACTAAA AAGTGTTTCG ATAAAAAAGG GATCATCATG ATAGGCGTTA TAGAGAATAG 900
AGGCTGCTAT GCGTAAATCT TCTGCCGTGA GATAAACTGC ACGACACTCT TCCATGGCTT 960
GATCTTCCAT TGTTATTGTC CTTGACCTTG ATCACACAAC ACCAATGTAA CAAGACTGTA 1020
TAGAAGTGCA ATTAATAATC AATTCGTGCA TTAAGCAGGT CAGCATTTCT TTGCTAAACA 1080
AGCTTTATTG GCTTTGACAA AACTTTGCCT AGACTTTAAC GATAGAAATC ATAATGAAAG 1140
AGAAAAGCTA CAACCTAGAG GGGAATAATC AAACAACTGC TAAGATCTAG ATAATGTAAT 1200
AAACACCGAG TTTATCGACC ATACTTAGAT AGAGTCATAG CAACGAGAAT AGTTATGGAT 1260
ACAACGCCGC AAGATCTATC ACACCTGTTT TTACAGCTAG GATTAGCAAA TGATCAACCC 1320
GCAATTGAAC AGTTTATCAA TGACCATCAA TTAGCGGACA ATATATTGCT ACATCAAGCA 1380
AGCTTTTGGA GCCCATCGCA AAAGCACTTC TTAATTGAGT CATTTAATGA AGATGCCCAG 1440
TGGACCGAAG TCATCGACCA CTTAGACACC TTATTAAGAA AAAACTAACC ATTACAACAG 1500

CAACTTTAAA TTTTGCCGTA AGCCATCTCC CCCCACCCCA CAACAGCGTT GTTGCTTATG 1560
ACCACTGGAG TACATTCGTC TTTAGTCGTT TTACCATCAC CATGGGTACG TTGAGTGCGA 1620
TAAAAAAGCA CATAAACTTC TTTATCGGCC TGAATATAGG CTTCGTTAAA ATCAGCTGTT 1680
CCCATTAAAG TAACCACTTG CTCTTTACTC ATGCCTAGAG ATATCTTTGT CAAATTGTCA 1740
CGGTTTTTAT CTTGAGTTTT CTCCCAAGCA CCGTGATTAT CCCAGTCAGA TTCCCCATCA 1800
CCAACATTGA CCACACAGCC CGTTAGCCCT AAGCTTGCAA TCCCAAAACA TGCTAAACCT 1860
AATAATTTAT TTTTCATTTT AACTTCCTGT TATGACATTA TTTTTGCTTA GAAGAAAAGC 1920
AACTTACATG CCAAAACACA AGCTGTTGTT TTAAATGACT TTATTTATTA TTAGCCTTTT 1980
AGGATATGCC TAGAGCAATA ATAATTACCA ATGTTTAAGG AATTTGACTA ACTATGAGTC 2040
CGATTGAGCA AGTGCTAACA GCTGCTAAAA AAATCAATGA ACAAGGTAGA GAACCAACAT 2100
TAGCATTGAT TAAAACCAAA CTTGGTAATA GCATCCCAAT GCGCGAGTTA ATCCAAGGTT 2160
TGCAACAGTT TAAGTCTATG AGTGCAGAAG AAAGACAAGC AATACCTAGC AGCTTAGCAA 2220
CAGCAAAAGA AACTCAATAT GGTCAATCAA GCTTATCTCA ATCTGAACAA GCTGATAGGA 2280
TCCTCCAGCT AGAAAACGCC CTCAATGAAT TAAGAAACGA ATTTAATGGG CTAAAAAGTC 2340
AATTTGATAA CTTACAACAA AACCTGATGA ATAAAGAGCC TGACACCAAA TGCATGTAAT 2400
TGAACTACGA TTTGAATGTT TTGATAACAC CACGATTACT GCAGCAGAAA AAGCCATTAA 2460
TGGTTTGCTT GAAGCTTATC GAGCCAATGG CCAGGTTCTA GGTCGTGAAT TTGCCGTTGC 2520
ATTTAACGAT GGTGAGTTTA AAGCACGCAT GTTAACCCCA GAAAAAAGCA GCTTATCTAA 2580
ACGCTTTAAT AGTCCTTGGG TAAATAGTGC ACTCGAAGAG CTAACCGAAG CCAAATTGCT 2640
TGCGCCACGT GAAAAGTATA TTGGCCAAGA TATTAATTCT GAAGCATCTA GCCAAGACAC 2700
ACCAAGTTGG CAGCTACTTT ACACAAGTTA TGTGCACATG TGCTCACCAC TAAGAAATGG 2760
CGACACCTTG CAGCCTATTC CACTGTATCA AATTCCAGCA ACTGCCAACG GCGATCATAA 2820
ACGAATGATC CGTTGGCAAA CAGAATGGCA AGCTTGTGAT GAATTGCAAA TGGCCGCAGC 2880
TACTAAAGCT GAATTTGCCG CACTTGAAGA GCTAACCAGT CATCAGAGTG ATCTATTTAG 2940
GCGTGGTTGG GACTTACGTG GCAGAGTCGA ATACTTGACG AAAATTCCGA CCTATTACTA 3000
TTTATACCGT GTTGGCGGTG AAAGCTTAGC AGTAGAAAAG CAGCGCTCTT GTCCTAAGTG 3060
TGGCAGTCAA GAATGGCTGC TCGATAAACC ATTATTGGAT ATGTTCCATT TTCGCTGTGA 3120
CACCTGCCGC ATCGTATCTA ATATCTCTTG GGACCATTTA TAACTCTTCC GAGTCTTATC 3180
ACACTAGAGT TTAGTCAGCA TAAAAATGGC GCTTATATTT CAATTAAAAG AAATATAAGC 3240
GCCATTTTCA TCGATACTAT ATATCAGCAG ACTATTTTCC GCGTAAATTA GCCCACATTA 3300
ATTTCATTCT TTGCCAGATC CCTGGATGAT CTAGTTGTGG CATCGACTCT TCAATAGGTT 3360
TAACCGCAGG TGTAACCCTT GGAGTCAATT CGTTTATAAA CTCGTTTAAA CTGTCACTTA 3420
ATTTAACGCT TTGTACTTCA CCTGGAATTT CAATCCATAC GCTGCCATCA CTATTATTAA 3480
CCGTCAACAT TTTATCTTCA TCATCAAGAA TACCAATAAA CCAAGTCGGC TCTTGCTTAA 3540
GCTTTCTCTT CATCATTAAA TGACCAATGA TGTTTTGTTG TAAGTATTCA AAATCAGTTT 3600
GATCCCACAC TTGGATTAGC TCACCTTGGC CCCATTGTGA GTCAAAAAAT AGCGGTGCAG 3660
AAAAATGACT GCCAAAAAAT GGATTAATTT CTGCAGATAA TGTCATTTCA AGTGCTGTTT 3720

CAACATTAGC AAATTCACCA GGTTGTTGAC GTACAACCGA TTGCCAAAAC ACTGCGCCAT 3780
CGGAGCCCGC TTCGGCGACA ACACACTCAG ACTTTTGTCC TTGCGCATAA TATCTTGGCT 3840
GTTCACCAAG CTTATCCATG TAGGCTTGTT GATATTTAGA TAAAAAAAGA TCTAAAGCAG 3900
GTAAAGAAGA CACTTAAGCC AGTTCCAAAA TCAGTTATAA TAGGGGTCTA TTTTGACATG 3960
GAAACCGTAT TGATGACACA ACATCATGAT CCCTACAGTA ACGCCCCCGA ACTTTCTGAA 4020
TTAACTTTAG GAAAGTCGAC CGGTTATCAA GAGCAGTATG ATGCATCTTT ACTACAAGCG 4080
TGCCGCGTAA ATTAAACCGT GATGCTATCG GTCTAACCAA TGAGCTACCT TTTCATGGCT 4140
GTGATATTTG GACTGGCTAC GAACTGTCTT GGCTAAATGC TAAAGGCAAG CCAATGATTG 4200
CTATTGCAGA CTTTAACCTA AGTTTTGATA GTAAAAATCT GATCGAGTCT AAGTCGTTTA 4260
AGCTGTATTT AAACAGCTAT AACCAAACAC GATTTGATAG CGTTCAAGCG GTTCAAGAAC 4320
GTTTAACTGA AGACTTAAGC GCCTGTGCCC AAGGCACAGT TACGGTAAAA GTGATTGAAC 4380
CTAAGCAATT TAACCACCTG AGAGTGGTTG ATATGCCAGG TACCTGCATT GACGATTTAG 4440
ATATTGAAGT TGATGACTAT AGCTTTAACT CTGACTATCT CACCGACAGT GTTGATGACA 4500
AAGTCATGGT TGCTGAAACG CTAACGTCAA ACTTATTGAA ATCAAACTGC CTAATCACTT 4560
CTCAGCCTGA CTGGGGTACA GTGATGATCC GTTATCAAGG GCCTAAGATA GACCGTGAAA 4620
AGCTACTTAG ATATCTGATT TCATTTAGAC AGCACAATGA ATTTCATGAG CAGTGTGTTG 4680
AGCGTATATT TGTTGATTTA AAGCACTATT GCCAATGTGC CAAACTTACT GTCTATGCAC 4740
GTTATACCCG CCGTGGTGGT TTAGATATCA ACCCATATCG TAGCGACTTT GAAAACCCTG 4800
CAGAAAATCA GCGCCTAGCG AGACAGTAAT TGATTGCAGT ACCTACAAAA AACAATGCCT 4860
ATAAGCCAAG CTTATGGGCA TTTTTATATT ATCAACTTGT CATCAAACCT CAGCCGCCAA 4920
GCCTTTTAGT TTTATCGCTA AATTAAGCCG CTCTCTCAGC CAAATATTTG CAGGATTTTG 4980
CTGTAATTTA TGGCTCCACA CCATGAAATA CTCTATCGGC TCTACCGCAA AAGGTAAGTC 5040
AAATACCTGT AAGCCAAACA GCTTGGCATA TTCGTCAGTG TGGGCTTTTG ACGCGATAGC 5100
TAACGCATCA CTTTTTGAGG CAACCGACAT CATACTTAAT ATTGATGATT GCTCGCTGTG 5160
CATTTGCCTT GCCGGTAACA CCTGTTTAGT CAGCAAGTCG GCAACACTTA AATTGTAGCG 5220
GCGCATCTTA AAAATAATAT GCTTTTCATT AAAGTATTGC TCTTGCGTCA ACCCACCTTG 5280
GATCCTTGGG TGAGCATTTC GTGCCACACA AACTAATTTA TCCTGCATTA CTTTTTGACT 5340
CTTAAATGCC GCAGATTCTG GCAGCCAAAT ATCTAAGGCT AAATCCACCT TTTCTAGTTG 5400
TAGGTCCATC TGCAACTCTT CTTCAATGAG CGGCGGCTCA CGAAATACAA TATTAATTGC 5460
AGTGCCCTGT AACACTTGCT CAATTTGATC TTGCAAGAGT TGTATTGCCG ACTCGCTGGC 5520
ATACACATAA AAAGTTCGCT CACTTGAAGT GGGGTCAAAT GCTTCAAAGC TAGTCGCAAC 5580
TTGCTCAATT GTTGACATAG CGCCCGCGAG CTGTTGATAA AGCGTCATCG CACTTGCGGT 5640
AGGTTTAACT CCCCTACCCA CTCGAGTAAA CAACTCTTCT CCAACAATAC TTTTTAGCCT 5700
CGAAATCGCA TTACTAACCG ACGACTGAGT CAAATCCAGC TCTTCTGCCG CCCGGCTAAA 5760
AGATGAGGTG CGATACACCG CAGTAAAAAC GCGAAATAAA TTAAGATCAA AAGCTTTTTG 5820
CTGCGACATA AATCAGCTAT CTCCTTATCC TTATCCTTAT CCTTATAAAA AGTTAGCTCC 5880
AGAGCACTCT AGCTCAAAAA CAACTCAGCG TATTAAGCCA ATATTTTGGG AACTCAATTA 5940

ATATTCATAA TAAAAGTATT CATAATATAA ATACCAAGTC ATAATTTAGC CCTAATTATT 6000
AATCAATTCA AGTTACCTAT ACTGGCCTCA ATTAAGCAAA TGTCTCATCA GTCTCCCTGC 6060
AACTAAATGC AATATTGAGA CATAAAGCTT TGAACTGATT CAATCTTACG AGGGTAACTT 6120
ATGAAACAGA CTCTAATGGC TATCTCAATC ATGTCGCTTT TTTCATTCAA TGCGCTAGCA 6180
GCGCAACATG AACATGACCA CATCACTGTT GATTACGAAG GGAAAGCCGC AACAGAACAC 6240
ACCATAGCTC ACAACCAAGC TGTAGCTAAA ACACTTAACT TTGCCGACAC GCGTGCATTT 6300
GAGCAATCGT CTAAAAATCT AGTCGCCAAG TTTGATAAAG CAACTGCCGA TATATTACGT 6360
GCCGAATTTG CTTTTATTAG CGATGAAATC CCTGACTCGG TTAACCCGTC TCTCTACCGT 6420
CAGGCTCAGC TTAATATGGT GCCTAATGGT CTGTATAAAG TGAGCGATGG CATTTACCAG 6480
GTCCGCGGTA CCGACTTATC TAACCTTACA CTTATCCGCA GTGATAACGG TTGGATAGCA 6540
TACGATGTTT TGTTAACCAA AGAAGCAGCA AAAGCCTCAC TACAATTTGC GTTAAAGAAT 6600
CTACCTAAAG ATGGCGATTT ACCCGTTGTT GCGATGATTT ACTCCCATAG CCATGCGGAC 6660
CACTTTGGCG GAGCTCGCGG TGTTCAAGAG ATGTTCCCTG ATGTCAAAGT CTACGGCTCA 6720
GATAACATCA CTAAAGAAAT TGTCGATGAG AACGTACTTG CCGGTAACGC CATGAGCCGC 6780
CGCGCAGCTT ATCAATACGG CGCAACACTG GGCAAACATG ACCACGGTAT TGTTGATGCT 6840
GCGCTAGGTA AAGGTCTATC AAAAGGTGAA ATCACTTACG TCGCCCCAGA CTACACCTTA 6900
AACAGTGAAG GCAAATGGGA AACGCTGACG ATTGATGGTC TAGAGATGGT GTTTATGGAT 6960
GCCTCGGGCA CCGAAGCTGA GTCAGAAATG ATCACTTATA TTCCCTCTAA AAAAGCGCTC 7020
TGGACGGCGG AGCTTACCTA TCAAGGTATG CACAACATTT ATACGCTGCG CGGCGCTAAA 7080
GTACGTGATG CGCTCAAGTG GTCAAAAGAT ATCAACGAAA TGATCAATGC CTTTGGTCAA 7140
GATGTCGAAG TGCTGTTTGC CTCGCACTCT GCGCCAGTGT GGGGTAACCA AGCGATCAAC 7200
GATTTCTTAC GCCTACAGCG TGATAACTAC GGCCTAGTGC ACAATCAAAC CTTGAGACTT 7260
GCCAACGATG GTGTCGGTAT ACAAGATATT GGCGATGCGA TTCAAGACAC GATTCCAGAG 7320
TCTATCTACA AGACGTGGCA TACCAATGGT TACCACGGCA CTTATAGCCA TAACGCTAAA 7380
GCGGTTTATA ACAAGTATCT AGGCTACTTC GATATGAACC CAGCCAACCT TAATCCGCTG 7440
CCAACCAAGC AAGAATCTGC CAAGTTTGTC GAATACATGG GCGGCGCAGA TGCCGCAATT 7500
AAGCGCGCTA AAGATGATTA CGCTCAAGGT GAATACCGCT TTGTTGCAAC GGCATTAAAT 7560
AAGGTGGTGA TGGCCGAGCC AGAAAATGAC TCCGCTCGTC AATTGCTAGC CGATACCTAT 7620
GAGCAACTTG GTTATCAAGC AGAAGGGGCT GGCTGGAGAA ACATTTACTT AACTGGCGCA 7680
CAAGAGCTAC GAGTAGGTAT TCAAGCTGGC GCGCCTAAAA CCGCATCGGC AGATGTCATC 7740
AGTGAAATGG ACATGCCGAC TCTATTTGAC TTCCTCGCGG TGAAGATTGA TAGTCAACAG 7800
GCGGCTAAGC ACGGCTTAGT TAAGATGAAT GTTATCACCC CTGATACTAA AGATATTCTC 7860
TATATTGAGC TAAGCAACGG TAACTTAAGC AACGCAGTGG TCGACAAAGA GCAAGCAGCT 7920
GACGCAAACC TTATGGTTAA TAAAGCTGAC GTTAACCGCA TCTTACTTGG CCAAGTAACC 7980
CTAAAAGCGT TATTAGCCAG CGGCGATGCC AAGCTCACTG GTGATAAAAC GGCATTTAGT 8040
AAAATAGCCG ATAGCATGGT CGAGTTTACA CCTGACTTCG AAATCGTACC AACGCCTGTT 8100
AAATGAGGCA TTAATCTCAA CAAGTGCAAG CTAGACATAA AAATGGGGCG ATTAGACGCC 8160

CCATTTTTTA TGCAATTTTG AACTAGCTAG TCTTAGCTGA AGCTCGAACA ACAGCTTTAA 8220
AATTCACTTC TTCTGCTGCA ATACTTATTT GCTGACACTG ACCAATACTC AGTGCAAAAC 8280
GATAACTATC ATCAAGATGG CCCAGTAAAC AATGCCAATT ATCAGCAGCG TTCATTTGCT 8340
GTTCTTTAGC CTCAATCAAA CCTAAACCAG ACTTTTGTGG CTCAGCGTTA GGCTTATTAG 8400
AACTCGACTC TAGTAAAGCA AGACCAATAT CTTGTTTTAA CAAAACCTGT CGCTGATTAA 8460
GTTGATGCTC AACCTTGTGA TCCGCAATAG CATCGGAAAT ATCAACACAA TGGCTCAAGC 8520
TTTTAGGTGC ATTAACTCCA AGAAAAGTTT CGCTCAGTGC AGAGAAGTCA AACGCAAAAG 8580
ATTTTAGCGA TAATGCCAGC CCAAGTCCTT TCGCTTTAAT GTAAGACTCC TTGAGCGCCC 8640
ACAAATCAAA AAAGCGGTCT CGCTGCAAGG CCTCTGGTAA CGCTAACAAG GCTCGCTTTT 8700
CTGATTCAGA GAAATAATGA CTAAGAATAG AGTGGATATT GGTGCTGTTA CGGCAACGCT 8760
CAATGTCGAC GCCAAACTCA ATACTAGCAG AGTCAGTTTC CTCCTTGCTT GCCTGACTGG 8820
CGCCTTTATT ATCAGCAGTG CAAATGCCTA CTAATAGCCA ATCTCCACTA TGACTCACAT 8880
TAAAGTGGAC CCCGGTTTGA GCAAATTGCG CATCACTCAA TCTAGGCTTA CCTTTGTCGC 8940
CATATTCAAA GCGCCATTCA TTGGGGCGTA TTTCACTATG TTGTGACAAT AAAGCGCGCA 9000
AATAGCCTCT TACCATTAAA CCTTGAGTTT TAGCTTCTTG TTTAATGTAG CGATTAACCT 9060
TAATTAACTC ATCTTCAGGC AGCCATGACT TAACCAACTC TGTAGTCTGG TTATCGCACT 9120
CTTGTATTGT TAACGGACAG AAGTATAAGG AAATCAATCG AGAAGTTAGC AATTTTTCAG 9180
GACACTCTTT AAAGCAACAA ACATAACCCC TATTTTTACC AATTTAAGAT CAAAACTAAA 9240
GCCAAAACTA ATTGAGAATA GTGTCAAACT AGCTTTAAAG GAAAAAAATA TAAAAAGAAC 9300
ATTATACTTG TATAAATTAT TTTACACACC AAAGCCATGA TCTTCACAAA ATTAGCTCCC 9360
TCTCCCTAAA ACAAGATTGA ATAAAAAAAT AAACCTTAAC TTTCATATAG ATAAAACAAA 9420
CCAATGGGAT AAAGTATATT GAATTCATTT TTAAGGAAAA ATTCAAATTG AATTCAAGCT 9480
CTTCAGTAAA AGCATATTTT GCCGTTAGTG TGAAAAAAAA CAAATTTAAA AACCAACATA 9540
GAACAAATAA GCAGACAATA AAACCAAGGC GCAACACAAA CAACGCGCTT ACAATTTTCA 9600
CAAAAAAGCA ACAAGAGTAA CGTTTAGTAT TTGGATATGG TTATTGTAAT TGAGAATTTT 9660
ATAACAATTA TATTAAGGGA ATGAGTATGT TTTTAAATTC AAAACTTTCG CGCTCAGTCA 9720
AACTTGCCAT ATCCGCAGGC TTAACAGCCT CGCTAGCTAT GCCTGTTTTT GCAGAAGAAA 9780
CTGCTGCTGA AGAACAAATA GAAAGAGTCG CAGTGACCGG ATCGCGAATC GCTAAAGCAG 9840
AGCTAACTCA ACCAGCTCCA GTCGTCAGCC TTTCAGCCGA AGAACTGACA AAATTTGGTA 9900
ATCAAGATTT AGGTAGCGTA CTAGCAGAAT TACCTGCTAT TGGTGCAACC AACACTATTA 9960
TTGGTAATAA CAATAGCAAC TCAAGCGCAG GTGTTAGCTC AGCAGACTTG CGTCGTCTAG 10020
GTGCTAACAG AACCTTAGTA TTAGTCAACG GTAAGCGCTA CGTTGCCGGC CAACCGGGCT 10080
CAGCTGAGGT AGATTTGTCA ACTATACCAA CTAGCATGAT CTCGCGAGTT GAGATTGTAA 10140
CCGGCGGTGC TTCAGCAATT TATGGTTCGG ACGCTGTATC AGGTGTTATC AACGTTATCC 10200
TTAAAGAAGA CTTTGAAGGC TTTGAGTTTA ACGCACGTAC TAGCGGTTCT ACTGAAAGTG 10260
TAGGCACTCA AGAGCACTCT TTTGACATTT TGGGTGGTGC AAACGTTGCA GATGGACGTG 10320
GTAATGTAAC CTTCTACGCA GGTTATGAAC GTACAAAAGA AGTCATGGCT ACCGACATTC 10380

GCCAATTCGA TGCTTGGGGA ACAATTAAAA ACGAAGCCGA TGGTGGTGAA GATGATGGTA 10440
TTCCAGACAG ACTACGTGTA CCACGAGTTT ATTCTGAAAT GATTAATGCT ACCGGTGTTA 10500
TCAATGCATT TGGTGGTGGA ATTGGTCGCT CAACCTTTGA CAGTAACGGC AATCCTATTG 10560
CACAACAAGA ACGTGATGGG ACTAACAGCT TTGCATTTGG TTCATTCCCT AATGGCTGTG 10620
ACACATGTTT CAACACTGAA GCATACGAAA ACTATATTCC AGGGGTAGAA AGAATAAACG 10680
TTGGCTCATC ATTCAACTTT GATTTTACCG ATAACATTCA ATTTTACACT GACTTCAGAT 10740
ATGTAAAGTC AGATATTCAG CAACAATTTC AGCCTTCATT CCGTTTTGGT AACATTAATA 10800
TCAATGTTGA AGATAACGCC TTTTTGAATG ACGACTTGCG TCAGCAAATG CTCGATGCGG 10860
GTCAAACCAA TGCTAGTTTT GCCAAGTTTT TTGATGAATT AGGAAATCGC TCAGCAGAAA 10920
ATAAACGCGA ACTTTTCCGT TACGTAGGTG GCTTTAAAGG TGGCTTTGAT ATTAGCGAAA 10980
CCATATTTGA TTACGACCTT TACTATGTTT ATGGCGAGAC TAATAACCGT CGTAAAACCC 11040
TTAATGACCT AATTCCTGAT AACTTTGTCG CAGCTGTCGA CTCTGTTATT GATCCTGATA 11100
CTGGCTTAGC AGCGTGTCGC TCACAAGTAG CAAGCGCTCA AGGCGATGAC TATACAGATC 11160
CCGCGTCTGT AAATGGTAGC GACTGTGTTG CTTATAACCC ATTTGGCATG GGTCAAGCTT 11220
CAGCAGAAGC CCGCGACTGG GTTTCTGCTG ATGTGACTCG TGAAGACAAA ATAACTCAAC 11280
AAGTGATTGG TGGTACTCTC GGTACCGATT CTGAAGAACT ATTTGAGCTT CAAGGTGGTG 11340
CAATCGCTAT GGTTGTTGGT TTTGAATACC GTGAAGAAAC GTCTGGTTCA ACAACCGATG 11400
AATTTACTAA AGCAGGTTTC TTGACAAGCG CTGCAACGCC AGATTCTTAT GGCGAATACG 11460
ACGTGACTGA GTATTTTGTT GAGGTGAACA TCCCAGTACT AAAAGAATTA CCTTTTGCAC 11520
ATGAGTTGAG CTTTGACGGT GCATACCGTA ATGCTGATTA CTCACATGCC GGTAAGACTG 11580
AAGCATGGAA AGCTGGTATG TTCTACTCAC CATTAGAGCA ACTTGCATTA CGTGGTACGG 11640
TAGGTGAAGC AGTACGAGCA CCAAACATTG CAGAAGCCTT TAGTCCACGC TCTCCTGGTT 11700
TTGGCCGCGT TTCAGATCCA TGTGATGCAG ATAACATTAA TGACGATCCG GATCGCGTGT 11760
CAAACTGTGC AGCATTGGGG ATCCCTCCAG GATTCCAAGC TAATGATAAC GTCAGTGTAG 11820
ATACCTTATC TGGTGGTAAC CCAGATCTAA AACCTGAAAC ATCAACATCC TTTACAGGTG 11880
GTCTTGTTTG GACACCAACG TTTGCTGACA ATCTATCATT CACTGTCGAT TATTATGATA 11940
TTCAAATTGA GGATGCTATT TTGTCAGTAG CCACCCAGAC TGTGGCTGAT AACTGTGTTG 12000
ACTCAACTGG CGGACCTGAC ACCGACTTCT GTAGTCAAGT TGATCGTAAT CCAACGACCT 12060
ATGATATTGA ACTTGTTCGC TCTGGTTATC TAAATGCCGC GGCATTGAAT ACCAAAGGTA 12120
TTGAATTTCA AGCTGCATAC TCATTAGATC TAGAGTCTTT CAACGCGCCT GGTGAACTAC 12180
GCTTCAACCT ATTGGGGAAC CAATTACTTG AACTAGAACG TCTTGAATTC CAAAATCGTC 12240
CTGATGAGAT TAATGATGAA AAAGGCGAAG TAGGTGATCC AGAGCTGCAG TTCCGCCTAG 12300
GCATCGATTA CCGTCTAGAT GATCTAAGTG TTAGCTGGAA CACGCGTTAT ATTGATAGCG 12360
TAGTAACTTA TGATGTCTCT GAAAATGGTG GCTCTCCTGA AGATTTATAT CCAGGCCACA 12420
TAGGCTCAAT GACAACTCAT GACTTGAGCG CTACATACTA CATCAATGAG AACTTCATGA 12480
TTAACGGTGG TGTACGTAAC CTATTTGACG CACTTCCACC TGGATACACT AACGATGCGC 12540
TATATGATCT AGTTGGTCGC CGTGCATTCC TAGGTATTAA GGTAATGATG TAATTAATTA 12600

TTACGCCTCT AACTAATAAA AATGCAATCT CTTCGTAGAG ATTGCATTTT TTTATGAAAT 12660
CCAATCTTAA ACTGGTTCTC CGAGCATCTT ACGCCTTAAA AACCCCGCCC CTCAATGTAA 12720
CGCCAAAGTT AATTGCTTAC ACGCACTTAC ACAAACGAAC AATTTCATTA ACACGAGACA 12780
CAGCTCACGC TTTTTATTTT ACCCTTGATT TTACTACATA AAATTGCGTT TTAGCGCACA 12840
AGTGTTCTCC CAAGCTGGTC GTATCTGTAA TTATTCAGTC CCAGGTGATT GTATTGACCC 12900
ATAAGCTCAG GTAGTCTGCT CTGCCATTAG CTAAACAATA TTGACAAAAT GGCGATAAAA 12960
TGTGGCTTAG CGCTAAGTTC ACCGTAAGTT TTATCGGCAT TAAGTCCCAA CAGATTATTA 13020
ACGGAAACCC GCTAAACTGA TGGCAAAAAT AAATAGTGAA CACTTGGATG AAGCTACTAT 13080
TACTTCGAAT AAGTGTACGC AAACAGAGAC TGAGGCTCGG CATAGAAATG CCACTACAAC 13140
ACCTGAGATG CGCCGATTCA TACAAGAGTC GGATCTCAGT GTTAGCCAAC TGTCTAAAAT 13200
ATTAAATATC AGTGAAGCTA CCGTACGTAA GTGGCGCAAG CGTGACTCTG TCGAAAACTG 13260
TCCTAATACC CCGCACCATC TCAATACCAC GCTAACCCCT TTGCAAGAAT ATGTGGTTGT 13320
GGGCCTGCGT TATCAATTGA AAATGCCATT AGACAGATTG CTCAAAGCAA CCCAAGAGTT 13380
TATCAATCCA AACGTGTCGC GCTCAGGTTT AGCAAGATGT TTGAAGCGTT ATGGCGTTTC 13440
ACGGGTGAGT GATATCCAAA GCCCACACGT ACCAATGCGC TACTTTAATC AAATTCCAGT 13500
CACTCAAGGC AGCGATGTGC AAACCTAACC CTGCACTATG AAACGCTGGC AAAAACCTTA 13560
GCCTTACCTA GTACCGATGG TGACAATGTG GTGCAAGTGG TGTCTCTCAC CATTCCACCA 13620
AAGTTAACCG AAGAAGCACC CAGTTCAATT TTGCTCGGCA TTGATCCTCA TAGCGACTGG 13680
ATCTATCTCG ACATATACCA AGATGGCAAT ACACAAGCCA CGAATAGATA TATGGCTTAT 13740
GTGCTAAAAC ACGGGCCATT CCATTTACGA AAGTTACTCG TGCGTAACTA TCACACCTTT 13800
TTACAGCGCT TTCCTGGAGC GACGCAAAAT CGCCGCCCCT CTAAAGATAT GCCTGAAACA 13860
ATCAACAAGA CGCCTGAAAC ACAGGCACCC AGTGGAGACT CATAATGAGC CAGACCTCTA 13920
AACCTACAAA CTCAGCAACT GAGCAAGCAC AAGACTCACA AGCTGACTCT CGTTTAAATA 13980
AACGACTAAA AGATATGCCA ATTGCTATTG TTGGCATGGC GAGTATTTTT GCAAACTCTC 14040
GCTATTTGAA TAAGTTTTGG GACTTAATCA GCGAAAAAAT TGATGCGATT ACTGAATTAC 14100
CATCAACTCA CTGGCAGCCT GAAGAATATT ACGACGCAGA TAAAACCGCA GCAGACAAAA 14160
GCTACTGTAA ACGTGGTGGC TTTTTGCCAG ATGTAGACTT CAACCCAATG GAGTTTGGCC 14220
TGCCGCCAAA CATTTTGGAA CTGACCGATT CATCGCAACT ATTATCACTC ATCGTTGCTA 14280
AAGAAGTGTT GGCTGATGCT AACTTACCTG AGAATTACGA CCGCGATAAA ATTGGTATCA 14340
CCTTAGGTGT CGGCGGTGGT CAAAAAATTA GCCACAGCCT AACAGCGCGT CTGCAATACC 14400
CAGTATTGAA GAAAGTATTC GCCAATAGCG GCATTAGTGA CACCGACAGC GAAATGCTTA 14460
TCAAGAAATT CCAAGACCAA TATGTACACT GGGAAGAAAA CTCGTTCCCA GGTTCACTTG 14520
GTAACGTTAT TGCGGGCCGT ATCGCCAACC GCTTCGATTT TGGCGGCATG AACTGTGTGG 14580
TTGATGCTGC CTGTGCTGGA TCACTTGCTG CTATGCGTAT GGCGCTAACA GAGCTAACTG 14640
AAGGTCGCTC TGAAATGATG ATCACCGGTG GTGTGTGTAC TGATAACTCA CCCTCTATGT 14700
ATATGAGCTT TTCAAAAACG CCCGCCTTTA CCACTAACGA AACCATTCAG CCATTTGATA 14760
TCGACTCAAA AGGCATGATG ATTGGTGAAG GTATTGGCAT GGTGGCGCTA AAGCGTCTTG 14820

AAGATGCAGA GCGCGATGGC GACCGCATTT ACTCTGTAAT TAAAGGTGTG GGTGCATCAT 14880
CTGACGGTAA GTTTAAATCA ATCTATGCCC CTCGCCCATC AGGCCAAGCT AAAGCACTTA 14940
ACCGTGCCTA TGATGACGCA GGTTTTGCGC CGCATACCTT AGGTCTAATT GAAGCTCACG 15000
GAACAGGTAC TGCAGCAGGT GACGCGGCAG AGTTTGCCGG CCTTTGCTCA GTATTTGCTG 15060
AAGGCAACGA TACCAAGCAA CACATTGCGC TAGGTTCAGT TAAATCACAA ATTGGTCATA 15120
CTAAATCAAC TGCAGGTACA GCAGGTTTAA TTAAAGCTGC TCTTGCTTTG CATCACAAGG 15180
TACTGCCGCC GACCATTAAC GTTAGTCAGC CAAGCCCTAA ACTTGATATC GAAAACTCAC 15240
CGTTTTATCT AAACACTGAG ACTCGTCCAT GGTTACCACG TGTTGATGGT ACGCCGCGCC 15300
GCGCGGGTAT TAGCTCATTT GGTTTTGGTG GCACTAACTT CCATTTTGTA CTAGAAGAGT 15360
ACAACCAAGA ACACAGCCGT ACTGATAGCG AAAAAGCTAA GTATCGTCAA CGCCAAGTGG 15420
CGCAAAGCTT CCTTGTTAGC GCAAGCGATA AAGCATCGCT AATTAACGAG TTAAACGTAC 15480
TAGCAGCATC TGCAAGCCAA GCTGAGTTTA TCCTCAAAGA TGCAGCAGCA AACTATGGCG 15540
TACGTGAGCT TGATAAAAAT GCACCACGGA TCGGTTTAGT TGCAAACACA GCTGAAGAGT 15600
TAGCAGGCCT AATTAAGCAA GCACTTGCCA AACTAGCAGC TAGCGATGAT AACGCATGGC 15660
AGCTACCTGG TGGCACTAGC TACCGCGCCG CTGCAGTAGA AGGTAAAGTT GCCGCACTGT 15720
TTGCTGGCCA AGGTTCACAA TATCTCAATA TGGGCCGTGA CCTTACTTGT TATTACCCAG 15780
AGATGCGTCA GCAATTTGTA ACTGCAGATA AAGTATTTGC CGCAAATGAT AAAACGCCGT 15840
TATCGCAAAC TCTGTATCCA AAGCCTGTAT TTAATAAAGA TGAATTAAAG GCTCAAGAAG 15900
CCATTTTGAC CAATACCGCC AATGCCCAAA GCGCAATTGG TGCGATTTCA ATGGGTCAAT 15960
ACGATTTGTT TACTGCGGCT GGCTTTAATG CCGACATGGT TGCAGGCCAT AGCTTTGGTG 16020
AGCTAAGTGC ACTGTGTGCT GCAGGTGTTA TTTCAGCTGA TGACTACTAC AAGCTGGCTT 16080
TTGCTCGTGG TGAGGCTATG GCAACAAAAG CACCGGCTAA AGACGGCGTT GAAGCAGATG 16140
CAGGAGCAAT GTTTGCAATC ATAACCAAGA GTGCTGCAGA CCTTGAAACC GTTGAAGCCA 16200
CCATCGCTAA ATTTGATGGG GTGAAAGTCG CTAACTATAA CGCGCCAACG CAATCAGTAA 16260
TTGCAGGCCC AACAGCAACT ACCGCTGATG CGGCTAAAGC GCTAACTGAG CTTGGTTACA 16320
AAGCGATTAA CCTGCCAGTA TCAGGTGCAT TCCACACTGA ACTTGTTGGT CACGCTCAAG 16380
CGCCATTTGC TAAAGCGATT GACGCAGCCA AATTTACTAA AACAAGCCGA GCACTTTACT 16440
CAAATGCAAC TGGCGGACTT TATGAAAGCA CTGCTGCAAA GATTAAAGCC TCGTTTAAGA 16500
AACATATGCT TCAATCAGTG CGCTTTACTA GCCAGCTAGA AGCCATGTAC AACGACGGCG 16560
CCCGTGTATT TGTTGAATTT GGTCCAAAGA ACATCTTACA AAAATTAGTT CAAGGCACGC 16620
TTGTCAACAC TGAAAATGAA GTTTGCACTA TCTCTATCAA CCCTAATCCT AAAGTTGATA 16680
GTGATCTGCA GCTTAAGCAA GCAGCAATGC AGCTAGCGGT TACTGGTGTG GTACTCAGTG 16740
AAATTGACCC ATACCAAGCC GATATTGCCG CACCAGCGAA AAAGTCGCCA ATGAGCATTT 16800
CGCTTAATGC TGCTAACCAT ATCAGCAAAG CAACTCGCGC TAAGATGGCC AAGTCTTTAG 16860
AGACAGGTAT CGTCACCTCG CAAATAGAAC ATGTTATTGA AGAAAAAATC GTTGAAGTTG 16920
AGAAACTGGT TGAAGTCGAA AAGATCGTCG AAAAAGTGGT TGAAGTAGAG AAAGTTGTTG 16980
AGGTTGAAGC TCCTGTTAAT TCAGTGCAAG CCAATGCAAT TCAAACCCGT TCAGTTGTCG 17040

CTCCAGTAAT AGAGAACCAA GTCGTGTCTA AAAACAGTAA GCCAGCAGTC CAGAGCATTA 17100
GTGGTGATGC ACTCAGCAAC TTTTTTGCTG CACAGCAGCA AACCGCACAG TTGCATCAGC 17160
AGTTCTTAGC TATTCCGCAG CAATATGGTG AGACGTTCAC TACGCTGATG ACCGAGCAAG 17220
CTAAACTGGC AAGTTCTGGT GTTGCAATTC CAGAGAGTCT GCAACGCTCA ATGGAGCAAT 17280
TCCACCAACT ACAAGCGCAA ACACTACAAA GCCACACCCA GTTCCTTGAG ATGCAAGCGG 17340
GTAGCAACAT TGCAGCGTTA AACCTACTCA ATAGCAGCCA AGCAACTTAC GCTCCAGCCA 17400
TTCACAATGA AGCGATTCAA AGCCAAGTGG TTCAAAGCCA AACTGCAGTC CAGCCAGTAA 17460
TTTCAACACA AGTTAACCAT GTGTCAGAGC AGCCAACTCA AGCTCCAGCT CCAAAAGCGC 17520
AGCCAGCACC TGTGACAACT GCAGTTCAAA CTGCTCCGGC ACAAGTTGTT CGTCAAGCCG 17580
CACCAGTTCA AGCCGCTATT GAACCGATTA ATACAAGTGT TGCGACTACA ACGCCTTCAG 17640
CCTTCAGCGC CGAAACAGCC CTGAGCGCAA CAAAAGTCCA AGCCACTATG CTTGAAGTGG 17700
TTGCTGAGAA AACCGGTTAC CCAACTGAAA TGCTAGAGCT TGAAATGGAT ATGGAAGCCG 17760
ATTTAGGCAT CGATTCTATC AAGCGTGTAG AAATTCTTGG CACAGTACAA GATGAGCTAC 17820
CGGGTCTACC TGAGCTTAGC CCTGAAGATC TAGCTGAGTG TCGAACGCTA GGCGAAATCG 17880
TTGACTATAT GGGCAGTAAA CTGCCGGCTG AAGGCTCTAT GAATTCTCAG CTGTCTACAG 17940
GTTCCGCAGC TGCGACTCCT GCAGCGAATG GTCTTTCTGC GGAGAAAGTT CAAGCGACTA 18000
TGATGTCTGT GGTTGCCGAA AAGACTGGCT ACCCAACTGA AATGCTAGAG CTTGAAATGG 18060
ATATGGAAGC CGATTTAGGC ATAGATTCTA TCAAGCGCGT TGAAATTCTT GGCACAGTAC 18120
AAGATGAGCT ACCGGGTCTA CCTGAGCTTA GCCCTGAAGA TCTAGCTGAG TGTCGTACTC 18180
TAGGCGAAAT CGTTGACTAT ATGAACTCTA AACTCGCTGA CGGCTCTAAG CTGCCGGCTG 18240
AAGGCTCTAT GAATTCTCAG CTGTCTACAA GTGCCGCAGC TGCGACTCCT GCAGCGAATG 18300
GTCTCTCTGC GGAGAAAGTT CAAGCGACTA TGATGTCTGT GGTTGCCGAA AAGACTGGCT 18360
ACCCAACTGA AATGCTAGAA CTTGAAATGG ATATGGAAGC TGACCTTGGC ATCGATTCAA 18420
TCAAGCGCGT TGAAATTCTT GGCACAGTAC AAGATGAGCT ACCGGGTTTA CCTGAGCTAA 18480
ATCCAGAAGA TTTGGCAGAG TGTCGTACTC TTGGCGAAAT CGTGACTTAT ATGAACTCTA 18540
AACTCGCTGA CGGCTCTAAG CTGCCAGCTG AAGGCTCTAT GCACTATCAG CTGTCTACAA 18600
GTACCGCTGC TGCGACTCCT GTAGCGAATG GTCTCTCTGC AGAAAAAGTT CAAGCGACCA 18660
TGATGTCTGT AGTTGCAGAT AAAACTGGCT ACCCAACTGA AATGCTTGAA CTTGAAATGG 18720
ATATGGAAGC CGATTTAGGT ATCGATTCTA TCAAGCGCGT TGAAATTCTT GGCACAGTAC 18780
AAGATGAGCT ACCGGGTTTA CCTGACTAAA TCCAGAAGAT CTAGCAGAGT GTCGCACCCT 18840
AGGCGAAATC GTTGACTATA TGGCAGTAAA CTGCCGGCTG AAGGCTCTGC TAATACAAGT 18900
GCCGCTGCGT CTCTTAATGT TAGTGCCGTT GCGGCGCCTC AAGCTGCTGC GACTCCTGTA 18960
TCGAACGGTC TCTCTGCAGA GAAAGTGCAA AGCACTATGA TGTCAGTAGT TGCAGAAAAG 19020
ACCGGCTACC CAACTGAAAT GCTAGAACTT GGCATGGATA TGGAAGCCGA TTTAGGTATC 19080
GACTCAATTA AACGCGTTGA GATTCTTGGC ACAGTACAAG ATGAGCTACC GGGTCTACCA 19140
GAGCTTAATC CTGAAGATTT AGCTGAGTGC CGTACGCTGG GCGAAATCGT TGACTATATG 19200
AACTCTAAGC TGGCTGACGG CTCTAAGCTT CCAGCTGAAG GCTCTGCTAA TACAAGTGCC 19260

ACTGCTGCGA CTCCTGCAGT GAATGGTCTT TCTGCTGACA AGGTACAGGC GACTATGATG 19320
TCTGTAGTTG CTGAAAAGAC CGGCTACCCA ACTGAAATGC TAGAACTTGG CATGGATATG 19380
GAAGCAGACC TTGGTATTGA TTCTATTAAG CGCGTTGAAA TTCTTGGCAC AGTACAAGAT 19440
GAGCTCCCAG GTTTACCTGA GCTTAATCCT GAAGATCTCG CTGAGTGCCG CACGCTTGGC 19500
GAAATCGTTA GCTATATGAA CTCTCAACTG GCTGATGGCT CTAAACTTTC TACAAGTGCG 19560
GCTGAAGGCT CTGCTGATAC AAGTGCTGCA AATGCTGCAA AGCCGGCAGC AATTTCGGCA 19620
GAACCAAGTG TTGAGCTTCC TCCTCATAGC GAGGTAGCGC TAAAAAAGCT TAATGCGGCG 19680
AACAAGCTAG AAAATTGTTT CGCCGCAGAC GCAAGTGTTG TGATTAACGA TGATGGTCAC 19740
AACGCAGGCG TTTTAGCTGA GAAACTTATT AAACAAGGCC TAAAAGTAGC CGTTGTGCGT 19800
TTACCGAAAG GTCAGCCTCA ATCGCCACTT TCAAGCGATG TTGCTAGCTT TGAGCTTGCC 19860
TCAAGCCAAG AATCTGAGCT TGAAGCCAGT ATCACTGCAG TTATCGCGCA GATTGAAACT 19920
CAGGTTGGCG CTATTGGTGG CTTTATTCAC TTGCAACCAG AAGCGAATAC AGAAGAGCAA 19980
ACGGCAGTAA ACCTAGATGC GCAAAGTTTT ACTCACGTTA GCAATGCGTT CTTGTGGGCC 20040
AAATTATTGC AACCAAAGCT CGTTGCTGGA GCAGATGCGC GTCGCTGTTT TGTAACAGTA 20100
AGCCGTATCG ACGGTGGCTT TGGTTACCTA AATACTGACG CCCTAAAAGA TGCTGAGCTA 20160
AACCAAGCAG CATTAGCTGG TTTAACTAAA ACCTTAAGCC ATGAATGGCC ACAAGTGTTC 20220
TGTCGCGCGC TAGATATTGC AACAGATGTT GATGCAACCC ATCTTGCTGA TGCAATCACC 20280
AGTGAACTAT TTGATAGCCA AGCTCAGCTA CCTGAAGTGG GCTTAAGCTT AATTGATGGC 20340
AAAGTTAACC GCGTAACTCT AGTTGCTGCT GAAGCTGCAG ATAAAACAGC AAAAGCAGAG 20400
CTTAACAGCA CAGATAAAAT CTTAGTGACT GGTGGGGCAA AAGGGGTGAC ATTTGAATGT 20460
GCACTGGCAT TAGCATCTCG CAGCCAGTCT CACTTTATCT TAGCTGGGCG CAGTGAATTA 20520
CAAGCTTTAC CAAGCTGGGC TGAGGGTAAG CAAACTAGCG AGCTAAAATC AGCTGCAATC 20580
GCACATATTA TTTCTACTGG TCAAAAGCCA ACGCCTAAGC AAGTTGAAGC CGCTGTGTGG 20640
CCAGTGCAAA GCAGCATTGA AATTAATGCC GCCCTAGCCG CCTTTAACAA AGTTGGCGCC 20700
TCAGCTGAAT ACGTCAGCAT GGATGTTACC GATAGCGCCG CAATCACAGC AGCACTTAAT 20760
GGTCGCTCAA ATGAGATCAC CGGTCTTATT CATGGCGCAG GTGTACTAGC CGACAAGCAT 20820
ATTCAAGACA AGACTCTTGC TGAACTTGCT AAAGTTTATG GCACTAAAGT CAACGGCCTA 20880
AAAGCGCTGC TCGCGGCACT TGAGCCAAGC AAAATTAAAT TACTTGCTAT GTTCTCATCT 20940
GCAGCAGGTT TTTACGGTAA TATCGGCCAA AGCGATTACG CGATGTCGAA CGATATTCTT 21000
AACAAGGCAG CGCTGCAGTT CACCGCTCGC AACCCACAAG CTAAAGTCAT GAGCTTTAAC 21060
TGGGGTCCTT GGGATGGCGG CATGGTTAAC CCAGCGCTTA AAAAGATGTT TACCGAGCGT 21120
GGTGTGTACG TTATTCCACT AAAAGCAGGT GCAGAGCTAT TTGCCACTCA GCTATTGGCT 21180
GAAACTGGCG TGCAGTTGCT CATTGGTACG TCAATGCAAG GTGGCAGCGA CACTAAAGCA 21240
ACTGAGACTG CTTCTGTAAA AAAGCTTAAT GCGGGTGAGG TGCTAAGTGC ATCGCATCCG 21300
CGTGCTGGTG CACAAAAAAC ACCACTACAA GCTGTCACTG CAACGCGTCT GTTAACCCCA 21360
AGTGCCATGG TCTTCATTGA AGATCACCGC ATTGGCGGTA ACAGTGTGTT GCCAACGGTA 21420
TGCGCCATCG ACTGGATGCG TGAAGCGGCA AGCGACATGC TTGGCGCTCA AGTTAAGGTA 21480

CTTGATTACA AGCTATTAAA AGGCATTGTA TTTGAGACTG ATGAGCCGCA AGAGTTAACA 21540
CTTGAGCTAA CGCCAGACGA TTCAGACGAA GCTACGCTAC AAGCATTAAT CAGCTGTAAT 21600
GGGCGTCCGC AATACAAGGC GACGCTTATC AGTGATAATG CCGATATTAA GCAACTTAAC 21660
AAGCAGTTTG ATTTAAGCGC TAAGGCGATT ACCACAGCAA AAGAGCTTTA TAGCAACGGC 21720
ACCTTGTTCC ACGGTCCGCG TCTACAAGGG ATCCAATCTG TAGTGCAGTT CGATGATCAA 21780
GGCTTAATTG CTAAAGTCGC TCTGCCTAAG GTTGAACTTA GCGATTGTGG TGAGTTCTTG 21840
CCGCAAACCC ACATGGGTGG CAGTCAACCT TTTGCTGAGG ACTTGCTATT ACAAGCTATG 21900
CTGGTTTGGG CTCGCCTTAA AACTGGCTCG GCAAGTTTGC CATCAAGCAT TGGTGAGTTT 21960
ACCTCATACC AACCAATGGC CTTTGGTGAA ACTGGTACCA TAGAGCTTGA AGTGATTAAG 22020
CACAACAAAC GCTCACTTGA AGCGAATGTT GCGCTATATC GTGACAACGG CGAGTTAAGT 22080
GCCATGTTTA AGTCAGCTAA AATCACCATT AGCAAAAGCT TAAATTCAGC ATTTTTACCT 22140
GCTGTCTTAG CAAACGACAG TGAGGCGAAT TAGTGGAACA AACGCCTAAA GCTAGTGCGA 22200
TGCCGCTGCG CATCGCACTT ATCTTACTGC CAACACCGCA GTTTGAAGTT AACTCTGTCG 22260
ACCAGTCAGT ATTAGCCAGC TATCAAACAC TGCAGCCTGA GCTAAATGCC CTGCTTAATA 22320
GTGCGCCGAC ACCTGAAATG CTCAGCATCA CTATCTCAGA TGATAGCGAT GCAAACAGCT 22380
TTGAGTCGCA GCTAAATGCT GCGACCAACG CAATTAACAA TGGCTATATC GTCAAGCTTG 22440
CTACGGCAAC TCACGCTTTG TTAATGCTGC CTGCATTAAA AGCGGCGCAA ATGCGGATCC 22500
ATCCTCATGC GCAGCTTGCC GCTATGCAGC AAGCTAAATC GACGCCAATG AGTCAAGTAT 22560
CTGGTGAGCT AAAGCTTGGC GCTAATGCGC TAAGCCTAGC TCAGACTAAT GCGCTGTCTC 22620
ATGCTTTAAG CCAAGCCAAG CGTAACTTAA CTGATGTCAG CGTGAATGAG TGTTTTGAGA 22680
ACCTCAAAAG TGAACAGCAG TTCACAGAGG TTTATTCGCT TATTCAGCAA CTTGCTAGCC 22740
GCACCCATGT GAGAAAAGAG GTTAATCAAG GTGTGGAACT TGGCCCTAAA CAAGCCAAAA 22800
GCCACTATTG GTTTAGCGAA TTTCACCAAA ACCGTGTTGC TGCCATCAAC TTTATTAATG 22860
GCCAACAAGC AACCAGCTAT GTGCTTACTC AAGGTTCAGG ATTGTTAGCT GCGAAATCAA 22920
TGCTAAACCA GCAAAGATTA ATGTTTATCT TGCCGGGTAA CAGTCAGCAA CAAATAACCG 22980
CATCAATAAC TCAGTTAATG CAGCAATTAG AGCGTTTGCA GGTAACTGAG GTTAATGAGC 23040
TTTCTCTAGA ATGCCAACTA GAGCTGCTCA GCATAATGTA TGACAACTTA GTCAACGCAG 23100
ACAAACTCAC TACTCGCGAT AGTAAGCCCG CTTATCAGGC TGTGATTCAA GCAAGCTCTG 23160
TTAGCGCTGC AAAGCAAGAG TTAAGCGCGC TTAACGATGC ACTCACAGCG CTGTTTGCTG 23220
AGCAAACAAA CGCCACATCA ACGAATAAAG GCTTAATCCA ATACAAAACA CCGGCGGGCA 23280
GTTACTTAAC CCTAACACCG CTTGGCAGCA ACAATGACAA CGCCCAAGCG GGTCTTGCTT 23340
TTGTCTATCC GGGTGTGGGA ACGGTTTACG CCGATATGCT TAATGAGCTG CATCAGTACT 23400
TCCCTGCGCT TTACGCCAAA CTTGAGCGTG AAGGCGATTT AAAGGCGATG CTACAAGCAG 23460
AAGATATCTA TCATCTTGAC CCTAAACATG CTGCCCAAAT GAGCTTAGGT GACTTAGCCA 23520
TTGCTGGCGT GGGGAGCAGC TACCTGTTAA CTCAGCTGCT CACCGATGAG TTTAATATTA 23580
AGCCTAATTT TGCATTAGGT TACTCAATGG GTGAAGCATC AATGTGGGCA AGCTTAGGCG 23640
TATGGCAAAA CCCGCATGCG CTGATCAGCA AAACCCAAAC CGACCCGCTA TTTACTTCTG 23700

CTATTTCCGG CAAATTGACC GCGGTTAGAC AAGCTTGGCA GCTTGATGAT ACCGCAGCGG 23760
AAATCCAGTG GAATAGCTTT GTGGTTAGAA GTGAAGCAGC GCCGATTGAA GCCTTGCTAA 23820
AAGATTACCC ACACGCTTAC CTCGCGATTA TTCAAGGGGA TACCTGCGTA ATCGCTGGCT 23880
GTGAAATCCA ATGTAAAGCG CTACTTGCAG CACTGGGTAA ACGCGGTATT GCAGCTAATC 23940
GTGTAACGGC GATGCATACG CAGCCTGCGA TGCAAGAGCA TCAAAATGTG ATGGATTTTT 24000
ATCTGCAACC GTTAAAAGCA GAGCTTCCTA GTGAAATAAG CTTTATCAGC GCCGCTGATT 24060
TAACTGCCAA GCAAACGGTG AGTGAGCAAG CACTTAGCAG CCAAGTCGTT GCTCAGTCTA 24120
TTGCCGACAC CTTCTGCCAA ACCTTGGACT TTACCGCGCT AGTACATCAC GCCCAACATC 24180
AAGGCGCTAA GCTGTTTGTT GAAATTGGCG CGGATAGACA AAACTGCACC TTGATAGACA 24240
AGATTGTTAA ACAAGATGGT GCCAGCAGTG TACAACATCA ACCTTGTTGC ACAGTGCCTA 24300
TGAACGCAAA AGGTAGCCAA GATATTACCA GCGTGATTAA AGCGCTTGGC CAATTAATTA 24360
GCCATCAGGT GCCATTATCG GTGCAACCAT TTATTGATGG ACTCAAGCGC GAGCTAACAC 24420
TTTGCCAATT GACCAGCCAA CAGCTGGCAG CACATGCAAA TGTTGACAGC AAGTTTGAGT 24480
CTAACCAAGA CCATTTACTT CAAGGGGAAG TCTAATGTCA TTACCAGACA ATGCTTCTAA 24540
CCACCTTTCT GCCAACCAGA AAGGCGCATC TCAGGCAAGT AAAACCAGTA AGCAAAGCAA 24600
AATCGCCATT GTCGGTTTAG CCACTCTGTA TCCAGACGCT AAAACCCCGC AAGAATTTTG 24660
GCAGAATTTG CTGGATAAAC GCGACTCTCG CAGCACCTTA ACTAACGAAA AACTCGGCGC 24720
TAACAGCCAA GATTATCAAG GTGTGCAAGG CCAATCTGAC CGTTTTTATT GTAATAAAGG 24780
CGGCTACATT GAGAACTTCA GCTTTAATGC TGCAGGCTAC AAATTGCCGG AGCAAAGCTT 24840
AAATGGCTTG GACGACAGCT TCCTTTGGGC GCTCGATACT AGCCGTAACG CACTAATTGA 24900
TGCTGGTATT GATATCAACG GCGCTGATTT AAGCCGCGCA GGTGTAGTCA TGGGCGCGCT 24960
GTCGTTCCCA ACTACCCGCT CAAACGATCT GTTTTTGCCA ATTTATCACA GCGCCGTTGA 25020
AAAAGCCCTG CAAGATAAAC TAGGCGTAAA GGCATTTAAG CTAAGCCCAA CTAATGCTCA 25080
TACCGCTCGC GCGGCAAATG AGAGCAGCCT AAATGCAGCC AATGGTGCCA TTGCCCATAA 25140
CAGCTCAAAA GTGGTGGCCG ATGCACTTGG CCTTGGCGGC GCACAACTAA GCCTAGATGC 25200
TGCCTGTGCT AGTTCGGTTT ACTCATTAAA GCTTGCCTGC GATTACCTAA GCACTGGCAA 25260
AGCCGATATC ATGCTAGCAG GCGCAGTATC TGGCGCGGAT CCTTTCTTTA TTAATATGGG 25320
ATTCTCAATC TTCCACGCCT ACCCAGACCA TGGTATCTCA GTACCGTTTG ATGCCAGCAG 25380
TAAAGGTTTG TTTGCTGGCG AAGGCGCTGG CGTATTAGTG CTTAAACGTC TTGAAGATGC 25440
CGAGCGCGAC AATGACAAAA TCTATGCGGT TGTTAGCGGC GTAGGTCTAT CAAACGACGG 25500
TAAAGGCCAG TTTGTATTAA GCCCTAATCC AAAAGGTCAG GTGAAGGCCT TTGAACGTGC 25560
TTATGCTGCC AGTGACATTG AGCCAAAAGA CATTGAAGTG ATTGAGTGCC ACGCAACAGG 25620
CACACCGCTT GGCGATAAAA TTGAGCTCAC TTCAATGGAA ACCTTCTTTG AAGACAAGCT 25680
GCAAGGCACC GATGCACCGT TAATTGGCTC AGCTAAGTCT AACTTAGGCC ACCTATTAAC 25740
TGCAGCGCAT GCGGGGATCA TGAAGATGAT CTTCGCCATG AAAGAAGGTT ACCTGCCGCC 25800
AAGTATCAAT ATTAGTGATG CTATCGCTTC GCCGAAAAAA CTCTTCGGTA AACCAACCCT 25860
GCCTAGCATG GTTCAAGGCT GGCCAGATAA GCCATCGAAT AATCATTTTG GTGTAAGAAC 25920

CCGTCACGCA GGCGTATCGG TATTTGGCTT TGGTGGCTGT AACGCCCATC TGTTGCTTGA 25980
GTCATACAAC GGCAAAGGAA CAGTAAAGGC AGAAGCCACT CAAGTACCGC GTCAAGCTGA 26040
GCCGCTAAAA GTGGTTGGCC TTGCCTCGCA CTTTGGGCCT CTTAGCAGCA TTAATGCACT 26100
CAACAATGCT GTGACCCAAG ATGGGAATGG CTTTATCGAA CTGCCGAAAA AGCGCTGGAA 26160
AGGCCTTGAA AAGCACAGTG AACTGTTAGC TGAATTTGGC TTAGCATCTG CGCCAAAAGG 26220
TGCTTATGTT GATAACTTCG AGCTGGACTT TTTACGCTTT AAACTGCCGC CAAACGAAGA 26280
TGACCGTTTG ATCTCACAGC AGCTAATGCT AATGCGAGTA ACAGACGAAG CCATTCGTGA 26340
TGCCAAGCTT GAGCCGGGGC AAAAAGTAGC TGTATTAGTG GCAATGGAAA CTGAGCTTGA 26400
ACTGCATCAG TTCCGCGGCC GGGTTAACTT GCATACTCAA TTAGCGCAAA GTCTTGCCGC 26460
CATGGGCGTG AGTTTATCAA CGGATGAATA CCAAGCGCTT GAAGCCATCG CCATGGACAG 26520
CGTGCTTGAT GCTGCCAAGC TCAATCAGTA CACCAGCTTT ATTGGTAATA TTATGGCGTC 26580
ACGCGTGGCG TCACTATGGG ACTTTAATGG CCCAGCCTTC ACTATTTCAG CAGCAGAGCA 26640
ATCTGTGAGC CGCTGTATCG ATGTGGCGCA AAACCTCATC ATGGAGGATA ACCTAGATGC 26700
GGTGGTGATT GCAGCGGTCG ATCTCTCTGG TAGCTTTGAG CAAGTCATTC TTAAAAATGC 26760
CATTGCACCT GTAGCCATTG AGCCAAACCT CGAAGCAAGC CTTAATCCAA CATCAGCAAG 26820
CTGGAATGTC GGTGAAGGTG CTGGCGCGGT CGTGCTTGTT AAAAATGAAG CTACATCGGG 26880
CTGCTCATAC GGCCAAATTG ATGCACTTGG CTTTGCTAAA ACTGCCGAAA CAGCGTTGGC 26940
TACCGACAAG CTACTGAGCC AAACTGCCAC AGACTTTAAT AAGGTTAAAG TGATTGAAAC 27000
TATGGCAGCG CCTGCTAGCC AAATTCAATT AGCGCCAATA GTTAGCTCTC AAGTGACTCA 27060
CACTGCTGCA GAGCAGCGTG TTGGTCACTG CTTTGCTGCA GCGGGTATGG CAAGCCTATT 27120
ACACGGCTTA CTTAACTTAA ATACTGTAGC CCAAACCAAT AAAGCCAATT GCGCGCTTAT 27180
CAACAATATC AGTGAAAACC AATTATCACA GCTGTTGATT AGCCAAACAG CGAGCGAACA 27240
ACAAGCATTA ACCGCGCGTT TAAGCAATGA GCTTAAATCC GATGCTAAAC ACCAACTGGT 27300
TAAGCAAGTC ACCTTAGGTG GCCGTGATAT CTACCAGCAT ATTGTTGATA CACCGCTTGC 27360
AAGCCTTGAA AGCATTACTC AGAAATTGGC GCAAGCGACA GCATCGACAG TGGTCAACCA 27420
AGTTAAACCT ATTAAGGCCG CTGGCTCAGT CGAAATGGCT AACTCATTCG AAACGGAAAG 27480
CTCAGCAGAG CCACAAATAA CAATTGCAGC ACAACAGACT GCAAACATTG GCGTCACCGC 27540
TCAGGCAACC AAACGTGAAT TAGGTACCCC ACCAATGACA ACAAATACCA TTGCTAATAC 27600
AGCAAATAAT TTAGACAAGA CTCTTGAGAC TGTTGCTGGC AATACTGTTG CTAGCAAGGT 27660
TGGCTCTGGC GACATAGTCA ATTTTCAACA GAACCAACAA TTGGCTCAAC AAGCTCACCT 27720
CGCCTTTCTT GAAAGCCGCA GTGCGGGTAT GAAGGTGGCT GATGCTTTAT TGAAGCAACA 27780
GCTAGCTCAA GTAACAGGCC AAACTATCGA TAATCAGGCC CTCGATACTC AAGCCGTCGA 27840
TACTCAAACA AGCGAGAATG TAGCGATTGC CGCAGAATCA CCAGTTCAAG TTACAACACC 27900
TGTTCAAGTT ACAACACCTG TTCAAATCAG TGTTGTGGAG TTAAAACCAG ATCACGCTAA 27960
TGTGCCACCA TACACGCCGC CAGTGCCTGC ATTAAAGCCG TGTATCTGGA ACTATGCCGA 28020
TTTAGTTGAG TACGCAGAAG GCGATATCGC CAAGGTATTT GGCAGTGATT ATGCCATTAT 28080
CGACAGCTAC TCGCGCCGCG TACGTCTACC GACCACTGAC TACCTGTTGG TATCGCGCGT 28140

GACCAAACTT GATGCGACCA TCAATCAATT TAAGCCATGC TCAATGACCA CTGAGTACGA 28200
CATCCCTGTT GATGCGCCGT ACTTAGTAGA CGGACAAATC CCTTGGGCGG TAGCAGTAGA 28260
ATCAGGCCAA TGTGACTTGA TGCTTATTAG CTATCTCGGT ATCGACTTTG AGAACAAAGG 28320
CGAGCGGGTT TATCGACTAC TCGATTGTAC CCTCACCTTC CTAGGCGACT TGCCACGTGG 28380
CGGAGATACC CTACGTTACG ACATTAAGAT CAATAACTAT GCTCGCAACG GCGACACCCT 28440
GCTGTTCTTC TTCTCGTATG AGTGTTTTGT TGGCGACAAG ATGATCCTCA AGATGGATGG 28500
CGGCTGCGCT GGCTTCTTCA CTGATGAAGA GCTTGCCGAC GGTAAAGGCG TGATTCGCAC 28560
AGAAGAAGAG ATTAAAGCTC GCAGCCTAGT GCAAAAGCAA CGCTTTAATC CGTTACTAGA 28620
TTGTCCTAAA ACCCAATTTA GTTATGGTGA TATTCATAAG CTATTAACTG CTGATATTGA 28680
GGGTTGTTTT GGCCCAAGCC ACAGTGGCGT CCACCAGCCG TCACTTTGTT TCGCATCTGA 28740
AAAATTCTTG ATGATTGAAC AAGTCAGCAA GGTTGATCGC ACTGGCGGTA CTTGGGGACT 28800
TGGCTTAATT GAGGGTCATA AGCAGCTTGA AGCAGACCAC TGGTACTTCC CATGTCATTT 28860
CAAGGGCGAC CAAGTGATGG CTGGCTCGCT AATGGCTGAA GGTTGTGGCC AGTTATTGCA 28920
GTTCTATATG CTGCACCTTG GTATGCATAC CCAAACTAAA AATGGTCGTT TCCAACCTCT 28980
TGAAAACGCC TCACAGCAAG TACGCTGTCG CGGTCAAGTG CTGCCACAAT CAGGCGTGCT 29040
AACTTACCGT ATGGAAGTGA CTGAAATCGG TTTCAGTCCA CGCCCATATG CTAAAGCTAA 29100
CATCGATATC TTGCTTAATG GCAAAGCGGT AGTGGATTTC CAAAACCTAG GGGTGATGAT 29160
AAAAGAGGAA GATGAGTGTA CTCGTTATCC ACTTTTGACT GAATCAACAA CGGCTAGCAC 29220
TGCACAAGTA AACGCTCAAA CAAGTGCGAA AAAGGTATAC AAGCCAGCAT CAGTCAATGC 29280
GCCATTAATG GCACAAATTC CTGATCTGAC TAAAGAGCCA AACAAGGGCG TTATTCCGAT 29340
TTCCCATGTT GAAGCACCAA TTACGCCAGA CTACCCGAAC CGTGTACCTG ATACAGTGCC 29400
ATTCACGCCG TATCACATGT TTGAGTTTGC TACAGGCAAT ATCGAAAACT GTTTCGGGCC 29460
AGAGTTCTCA ATCTATCGCG GCATGATCCC ACCACGTACA CCATGCGGTG ACTTACAAGT 29520
GACCACACGT GTGATTGAAG TTAACGGTAA GCGTGGCGAC TTTAAAAAGC CATCATCGTG 29580
TATCGCTGAA TATGAAGTGC CTGCAGATGC GTGGTATTTC GATAAAAACA GCCACGGCGC 29640
AGTGATGCCA TATTCAATTT TAATGGAGAT CTCACTGCAA CCTAACGGCT TTATCTCAGG 29700
TTACATGGGC ACAACCCTAG GCTTCCCTGG CCTTGAGCTG TTCTTCCGTA ACTTAGACGG 29760
TAGCGGTGAG TTACTACGTG AAGTAGATTT ACGTGGTAAA ACCATCCGTA ACGACTCACG 29820
TTTATTATCA ACAGTGATGG CCGGCACTAA CATCATCCAA AGCTTTAGCT TCGAGCTAAG 29880
CACTGACGGT GAGCCTTTCT ATCGCGGCAC TGCGGTATTT GGCTATTTTA AAGGTGACGC 29940
ACTTAAAGAT CAGCTAGGCC TAGATAACGG TAAAGTCACT CAGCCATGGC ATGTAGCTAA 30000
CGGCGTTGCT GCAAGCACTA AGGTGAACCT GCTTGATAAG AGCTGCCGTC ACTTTAATGC 30060
GCCAGCTAAC CAGCCACACT ATCGTCTAGC CGGTGGTCAG CTGAACTTTA TCGACAGTGT 30120
TGAAATTGTT GATAATGGCG GCACCGAAGT TTAAGGTTAC TTGTATGCCG AGCGCACCAT 30180
TGACCCAAGT GATTGGTTCT TCCAGTTCCA CTTCCACCAA GATCCGGTTA TGCCAGGCTC 30240
CTTAGGTGTT GAAGCAATTA TTGAAACCAT GCAAGCTTAC GCTATTAGTA AAGACTTGGG 30300
CGCAGATTTC AAAAATCCTA AGTTTGGTCA GATTTTATCG AACATCAAGT GGAAGTATCG 30360

CGGTCAAATC AATCCGCTGA ACAAGCAGAT GTCTATGGAT GTCAGCATTA CTTCAATCAA 30420
AGATGAAGAC GGTAAGAAAG TCATCACAGG TAATGCCAGC TTGAGTAAAG ATGGTCTGCG 30480
CATATACGAG GTCTTCGATA TAGCTATCAG CATCGAAGAA TCTGTATAAA TCGGAGTGAC 30540
TGTCTGGCTA TTTTACTCAA TTTCTGTGTC AAAAGTGCTC ACCTATATTC ATAGGCTGCG 30600
CGCTTTTTTC TGGAAATTGA GCAAAAGTAT CTGCGTCCTA ACTCGATTTA TAAGAATGGT 30660
TTAATTGAAA AGAACAACAG CTAAGAGCCG CAAGCTCAAT ATAAATAATT AAGGGTCTTA 30720
CAAATAATGA ATCCTACAGC AACTAACGAA ATGCTTTCTC CGTGGCCATG GGCTGTGACA 30780
GAGTCAAATA TCAGTTTTGA CGTGCAAGTG ATGGAACAAC AACTTAAAGA TTTTAGCCGG 30840
GCATGTTACG TGGTCAATCA TGCCGACCAC GGCTTTGGTA TTGCGCAAAC TGCCGATATC 30900
GTGACTGAAC AAGCGGCAAA CAGCACAGAT TTACCTGTTA GTGCTTTTAC TCCTGCATTA 30960
GGTACCGAAA GCCTAGGCGA CAATAATTTC CGCCGCGTTC ACGGCGTTAA ATACGCTTAT 31020
TACGCAGGCG CTATGGCAAA CGGTATTTCA TCTGAAGAGC TAGTGATTGC CCTAGGTCAA 31080
GCTGGCATTT TGTGTGGTTC GTTTGGAGCA GCCGGTCTTA TTCCAAGTCG CGTTGAAGCG 31140
GCAATTAACC GTATTCAAGC AGCGCTGCCA AATGGCCCTT ATATGTTTAA CCTTATCCAT 31200
AGTCCTAGCG AGCCAGCATT AGAGCGTGGC AGCGTAGAGC TATTTTTAAA GCATAAGGTA 31260
CGCACCGTTG AAGCATCAGC TTTCTTAGGT CTAACACCAC AAATCGTCTA TTACCGTGCA 31320
GCAGGATTGA GCCGAGACGC ACAAGGTAAA GTTGTGGTTG GTAACAAGGT TATCGCTAAA 31380
GTAAGTCGCA CCGAAGTGGC TGAAAAGTTT ATGATGCCAG CGCCCGCAAA AATGCTACAA 31440
AAACTAGTTG ATGACGGTTC AATTACCGCT GAGCAAATGG AGCTGGCGCA ACTTGTACCT 31500
ATGGCTGACG ACATCACTGC AGAGGCCGAT TCAGGTGGCC ATACTGATAA CCGTCCATTA 31560
GTAACATTGC TGCCAACCAT TTTAGCGCTG AAAGAAGAAA TTCAAGCTAA ATACCAATAC 31620
GACACTCCTA TTCGTGTCGG TTGTGGTGGC GGTGTGGGTA CGCCTGATGC AGCGCTGGCA 31680
ACGTTTAACA TGGGCGCGGC GTATATTGTT ACCGGCTCTA TCAACCAAGC TTGTGTTGAA 31740
GCGGGCGCAA GTGATCACAC TCGTAAATTA CTTGCCACCA CTGAAATGGC CGATGTGACT 31800
ATGGCACCAG CTGCAGATAT GTTCGAGATG GGCGTAAAAC TGCAGGTGGT TAAGCGCGGC 31860
ACGCTATTCC CAATGCGCGC TAACAAGCTA TATGAGATCT ACACCCGTTA CGATTCAATC 31920
GAAGCGATCC CATTAGACGA GCGTGAAAAG CTTGAGAAAC AAGTATTCCG CTCAAGCCTA 31980
GATGAAATAT GGGCAGGTAC AGTGGCGCAC TTTAACGAGC GCGACCCTAA GCAAATCGAA 32040
CGCGCAGAGG GTAACCCTAA GCGTAAAATG GCATTGATTT TCCGTTGGTA CTTAGGTCTT 32100
TCTAGTCGCT GGTCAAACTC AGGCGAAGTG GGTCGTGAAA TGGATTATCA AATTTGGGCT 32160
GGCCCTGCTC TCGGTGCATT TAACCAATGG GCAAAAGGCA GTTACTTAGA TAACTATCAA 32220
GACCGAAATG CCGTCGATTT GGCAAAGCAC TTAATGTACG GCGCGGCTTA CTTAAATCGT 32280
ATTAACTCGC TAACGGCTCA AGGCGTTAAA GTGCCAGCAC AGTTACTTCG CTGGAAGCCA 32340
AACCAAAGAA TGGCCTAATA CACTTACAAA GCACCAGTCT AAAAAGCCAC TAATCTTGAT 32400
TAGTGGCTTT TTTTATTGTG GTCAATATGA GGCTATTTAG CCTGTAAGCC TGAAAATATC 32460
AGCACTCTGA CTTTACAAGC AAATTATAAT TAAGGCAGGG CTCTACTCAT TTATACTGCT 32520
AGCAAACAAG CAAGTTGCCC AGTAAAACAA CAAGGTACCT GATTTATATC GTCATAAAAG 32580

TTGGCTAGAG ATTCGTTATT GATCTTTACT GATTAGAGTC GCTCTGTTTG GAAAAAGGTT 32640
TCTCGTTATC ATCAAAATAC ACTCTCAAAC CTTTAATCAA TTACAACTTA GGCTTTCTGC 32700
GGGCATTTTT ATCTTATTTG CCACAGCTGT ATTTGCCTTT AGGTTTTGGG TGCAACTACC 32760
ATTAATTGAG GCCTCATTAG TTAAATTATC TGAGCAAGAG CTCACCTCTT TAAATTACGC 32820
TTTTCAGCAA ATGAGAAAGC CACTACAAAC CATTAATTAC GACTATGCGG TGTGGGACAG 32880
AACCTACAGC TATATGAAAT CAAACTCAGC GAGCGCTAAA AGGTACTATG AAAAACATGA 32940
GTACCCAGAT GATACGTTCA AGAGTTTAAA AGTCGACGGA GTATTTATAT TCAACCGTAC 33000
AAATCAGCCA GTTTTTAGTA AAGGTTTTAA TCATAGAAAT GATATACCGC TGGTCTTTGA 33060
ATTAACTGAC TTTAAACAAC ATCCACAAAA CATCGCATTA TCTCCACAAA CCAAACAGGC 33120
ACACCCACCG GCAAGTAAGC CGTTAGACTC CCCTGATGAT GTGCCTTCTA CCCATGGGGT 33180
TATCGCCACA CGATACGGTC CAGCAATTTA TAGCTCTACC AGCATTTTAA AATCTGATCG 33240
TAGCGGCTCC CAACTTGGTT ATTTAGTCTT CATTAGGTTA ATTGATGAAT GGTTCATCGC 33300
TGAGCTATCG CAATACACTG CCGCAGGTGT TGAAATCGCT ATGGCTGATG CCGCAGACGC 33360
ACAATTAGCG AGATTAGGCG CAAACACTAA GCTTAATAAA GTAACCGCTA CATCCGAACG 33420
GTTAATAACT AATGTCGATG GTAAGCCTCT GTTGAAGTTA GTGCTTTACC ATACCAATAA 33480
CCAACCGCCG CCGATGCTAG ATTACAGTAT AATAATTCTA TTAGTTGAGA TGTCATTTTT 33540
ACTGATCCTC GCTTATTTCC TTTACTCCTA CTTCTTAGTC AGGCCAGTTA GAAAGCTGGC 33600
TTCAGATATT AAAAAAATGG ATAAAAGTCG TGAAATTAAA AAGCTAAGGT ATCACTACCC 33660
TATTACTGAG CTATTCTTAG TCAGGCCAGT TAGAAAGCTG GCTTCAGATA TTAAAAAAAT 33720
GGATAAAAGT CGTGAAATTA AAAAGCTAAG GTATCACTAC CCTATTACTG AGCTAGATAA 33780
ATTAACCAAT ATTCCCAATC GTCGCGCTTT TGAGCAGCGA CTTGAAACCT ATTGCCAACT 33840
GCTAGCCCGG CAACAAATTG GCTTTACTCT CATCATTGCC GATGTGGATC ATTTTAAAGA 33900
GTACAACGAT ACTCTTGGGC ACCTTGCTGG GGATGAAGCA TTAATAAAAG TGGCACAAAC 33960
ACTATCGCAA CAGTTTTACC GTGCAGAAGA TATTTGTGCC CGTTTTGGTG GTGAAGAATT 34020
TATTATGTTA TTTCGAGACA TACCTGATGA GCCCTTGCAG AGAAAGCTCG ATGCGATGCT 34080
GCACTCTTTT GCAGAGCTCA ACCTACCTCA TCCAAACTCA TCAACCGCTA ATTACGTTAC 34140
TGTGAGCCTT GGGGTTTGCA CAGTTGTTGC TGTTGATGAT TTTGAATTTA AAAGTGAGTC 34200
GCATATTATT GGCAGTCAGG CTGCATTAAT CGCAGATAAG GCGCTTTATC ATGCTAAAGC 34260
CTGTGGTCGT AACCAGTTGT CAAAAACTAC TATTACTGTT GATGAGATTG AGCAATTAGA 34320
AGCAAATAAA ATCGGTCATC AAGCCTAAAC TCGTTCGAGT ACTTTCCCCT AAGTCAGAGC 34380
TATTTGCCAC TTCAAGATGT GGCTACAAGG CTTACTCTTT CAAAACCTGC ATCAATAGAA 34440
CACAGCAAAA TACAATAATT TAAGTCAATT TAGCCTATTA AACAGAGTTA ATGACAGCTC 34500
ATGGTCGCAA CTTATTAGCT ATTTCTAGCA ATATAAAAAC TTATCCATTA GTAGTAACCA 34560
ATAAAAAAAC TAATATATAA AACTATTTAA TCATTATTTT ACAGATGATT AGCTACCACC 34620
CACCTTAAGC TGGCTATATT CGCACTAGTA AAAATAAACA TTAGATCGGG TTCAGATCAA 34680
TTTACGAGTC TCGTATAAAA TGTACAATAA TTCACTTAAT TTAATACTGC ATATTTTTAC 34740
AAGTAGAGAG CGGTGATGAA ACAAAATACG AAAGGCTTTA CATTAATTGA ATTAGTCATC 34800

GTGATTATTA TTCTCGGTAT ACTTGCTGCT GTGGCACTGC CGAAATTCAT CAATGTTCAA 34860
GATGACGCTA GGATCTCTGC GATGAGCGGT CAGTTTTCAT CATTTGAAAG TGCCGTAAAA 34920
CTATACCATA GCGGTTGGTT AGCCAAAGGC TACAACACTG CGGTTGAAAA GCTCTCAGGC 34980
TTTGGCCAAG GTAATGTTGC ATCAAGTGAC ACAGGTTTTC CGTACTCAAC ATCAGGCACG 35040
AGTACTGATG TGCATAAAGC TTGTGGTGAA CTATGGCATG GCATTACCGA TACAGACTTC 35100
ACAATTGGTG CGGTTAGTGA TGGCGATCTA ATGACTGCAG ATGTCGATAT TGCTTACACC 35160
TATCGTGGTG ATATGTGTAT CTATCGCGAT CTGTATTTTA TTCAGCGCTC ATTACCTACT 35220
AAGGTGATGA ACTACAAATT TAAAACTGGT GAAATAGAAA TTATTGATGC TTTCTACAAC 35280
CCTGACGGCT CAACTGGTCA ATTACCATAA ATTTGGCGCT TATCTAAGTT GTACTTGCTC 35340
TGACCGACAC AAATAATGTC GTTTCTCAGC ATATATCAAA ATACACAGCA AAAATTTGGG 35400
GTTAGCTATA TAGCTAACCC CAAATCATAT CTAACTTTAC ACTGCATCTA ATTCCAAACA 35460
GTATCCAGCC AAAAGCCTAA ACTATTGTTG ACTCAGCGCT AAAATATGCG ATGCAACAAA 35520
CAAGTCTTGG ATCGCAATAC CTGAGCTATC AAAAATGGTC ACCTCATCAG CACTTTGACG 35580
TCCTGTTGCG GACTCGTTTA TCACCTGACC AATCTCAATT ATCGGCGTAT TTCTGCTATG 35640
TTGAAACTCA CCAATAACAA TAGATTGAGA AGCAAAGTCG CAAAACAAGC GAGCATGACT 35700
ATATAGGTCA GTTGGCAACT CTTGCTTACC CACTTTATCA GCGCCCATTG CAGAAATATG 35760
CGTTCCTGCT TGTACCCACT GCGCTTCAAA TAAAGGCGCT TGAGCTGTGG TTGCTGTGAT 35820
AATAATATCT GCTTGTTCAC AAGCAGCTTG TGCATCACAA GCTTCGGCAT TAATGCCTTT 35880
TTCTAATAAA CGCTTAACCA AGTTTTCAGT TTTGCTAGCA CTACGGCCAA CTACCAATAC 35940
CTTAGTTAAT GAACGAACCT TGCTCACTGC TAGCACTTCA TATTCAGCCT GATGACCGGT 36000
ACCAAAAACA GTTAATACCG TAGCATCTTC TCTCGCGAGG TAACTCACTG CTACTGCATC 36060
GGCAGCACCA GTGCGGTAAG CATTAACGGT AGTGGCAGCA ATCACCGNCT GCAACATACC 36120
GGTTAATGGA TCGAGTAAAA ATACGTTAGT GCCGTGGCAT GGTAAACCAT GTTTATGGTT 36180
ATCAGGCCAA TAGCTGCCTG TTTTCCAGCC GACAAGGTTT GGCGTTGAAG CCGACTTTAA 36240
TGAGAACATT TCATTAAGGT TCGCGCCCTG TGCATTAACT ACCGGGAACA AGGTTGCTTT 36300
ATCATCTACG GCAGCGACAA ACGCTTCTTT AACAGCGATA TAAGCCAGCT CATGGGAGAT 36360
GAGCTTTGAT GTTTGCGCTT CAGTTAAATA GATCATATTA CCACCCCTGC ACTCGATTCC 36420
AGATCTCATA GCCACCATTA TCACCATCAG TATCAAATAC ATGGTACTGA GCGTGCATTG 36480
AAGCTGTTGC ACAGGCGTGG TTCGGCAAAA TATGTAGACG ACTACCTACC GGGAACTGCG 36540
CTAAATCAAT AACGCCGCCA TCAACTGCTT CAATAATGCC GTGCTCTTGA TTAACAGTTA 36600
TAACCTGTAG ACCTGATAAC ACGTGACCGC TGTCGTCACA CACTAAACCA TAACCACAAT 36660
CTTTTGGCTG CTCTGCAGTA CCTCTATCAC CCGAAAGAGC CATCCAACCC GCATCAATGA 36720
AAATCCAGTT TTTATCAGGA TTATGACCAA TAACACTGGT CACTACCGTT GCGGCAATAT 36780
CAGTTAACTG ACACACGTTT AGCCCTGCCA TGACTAAATC GAAGAAGGTG TACACACCCG 36840
CTCTAACCTC GGTGATCCCA TCAAGGTTTT GATAGCTTTG CGCTGTTGGT GTTGAACCAA 36900
TACTAACGAT GTCACATTGC ATACCCGCTG CGCGAATGCG TCAGCAGCTT GTACAGCCGC 36960
TGCAACTTCA TTTTGCGCCG CATCAATTAA TTGCTGTTTT TCAAAACATT GATATGACTC 37020

ACCAGCGTGA GTNAGTACGC CGTGAAAACT CGCTGCGCCA GACGTTAGTA TCTGAGCAAT 37080
TTCAATCAAC TTATCGGCTT CCGGTGGAAT ACCACCACGA TGGCCATCAC AATCAATTTC 37140
AATTAATGCT GGTATTTGGC AGTCATAAGA ACCACAGAAA TGATTTAGCT GATGCGCTTG 37200
CTCAACACTA TCAAGTAAAA CTCTTGCATT AATACCTTGG TCCAACATTT TAGCAATACG 37260
CGGCAACTTA CCATCGGCAA TACCTACTGC ATAAATAATG TCTGTGTAAC CTTTAGATGC 37320
TAAGGCCTCG GCCTCTTTTA CCGTTGATAC AGTGACTGGT GAGTTTTTAG TGGGTAATAA 37380
AAACTCGGCT GCTTCAAGTG ATCTTAACGT TTTAAAATGC GGTCTTAGGT TTGCACCTAA 37440
TCCTTCAATT TTTTGGCGTA GTTGACTGAG GTTATTAATA AATACTGGCT TATTTACATA 37500
TAAAAACGGT GTATCAATTG CTTGATACTG ACTTTGCTGA GTCGTGGAAA GTATTTGAGT 37560
AGATGGCATC TTTAATATCC TAGTTCATCA ATCAATCTAA CAAGTTTGAT GCCTAGCCAC 37620
AGTGGCTTGT ATTCATGATG CTTTGGAAAA TGCTTATATT CAAAGTATTT GAAAGACATC 37680
AAACTTCTTG TTTAATGCTC AGTATCCACC AGCACGCATT TATTTTATAT TAACTATTAT 37740
CAAGATATAG ATTAGGTTCA AACCAAATGA TTAGTACTGA AGATCTACGT TTTATCAGCG 37800
TAATCGCCAG TCATCGCACC TTAGCTGATG CCGCTAGAAC ACTAAATATC ACGCCACCAT 37860
CAGTGACATT AAGGTTGCAG CATATTGAAA AGAAACTATC GATTAGCCTG ATC 37913

SEQ ID NO: 2
SEQUENCE LENGTH: 1983
SEQUENCE TYPE: Nucleic acid
STRANDNESS: Double strand
TOPOLOGY: Linear
MOLECULE TYPE: Genomic DNA
ORIGINAL SOURCE: Shewanella putrefaciens SCRC-2874
(FERM BP-1625)

SEQUENCE
ATG AAA CAG ACT CTA ATG GCT ATC TCA ATC ATG TCG CTT TTT TCA TTC 48
Met Lys Gln Thr Leu Met Ala Ile Ser Ile Met Ser Leu Phe Ser Phe
1               5                   10                  15
AAT GCG CTA GCA GCG CAA CAT GAA CAT GAC CAC ATC ACT GTT GAT TAC 96
Asn Ala Leu Ala Ala Gln His Glu His Asp His Ile Thr Val Asp Tyr
            20                  25                  30
GAA GGG AAA GCC GCA ACA GAA CAC ACC ATA GCT CAC AAC CAA GCT GTA 144
Glu Gly Lys Ala Ala Thr Glu His Thr Ile Ala His Asn Gln Ala Val
        35                  40                  45

GCT AAA ACA CTT AAC TTT GCC GAC ACG CGT GCA TTT GAG CAA TCG TCT 192
Ala Lys Thr Leu Asn Phe Ala Asp Thr Arg Ala Phe Glu Gln Ser Ser
    50                  55                  60
AAA AAT CTA GTC GCC AAG TTT GAT AAA GCA ACT GCC GAT ATA TTA CGT 240
Lys Asn Leu Val Ala Lys Phe Asp Lys Ala Thr Ala Asp Ile Leu Arg
65                  70                  75                  80
GCC GAA TTT GCT TTT ATT AGC GAT GAA ATC CCT GAC TCG GTT AAC CCG 288
Ala Glu Phe Ala Phe Ile Ser Asp Glu Ile Pro Asp Ser Val Asn Pro
                85                  90                  95
TCT CTC TAC CGT CAG GCT CAG CTT AAT ATG GTG CCT AAT GGT CTG TAT 336
Ser Leu Tyr Arg Gln Ala Gln Leu Asn Met Val Pro Asn Gly Leu Tyr
            100                 105                 110
AAA GTG AGC GAT GGC ATT TAC CAG GTC CGC GGT ACC GAC TTA TCT AAC 384
Lys Val Ser Asp Gly Ile Tyr Gln Val Arg Gly Thr Asp Leu Ser Asn
        115                 120                 125
CTT ACA CTT ATC CGC AGT GAT AAC GGT TGG ATA GCA TAC GAT GTT TTG 432
Leu Thr Leu Ile Arg Ser Asp Asn Gly Trp Ile Ala Tyr Asp Val Leu
    130                 135                 140
TTA ACC AAA GAA GCA GCA AAA GCC TCA CTA CAA TTT GCG TTA AAG AAT 480
Leu Thr Lys Glu Ala Ala Lys Ala Ser Leu Gln Phe Ala Leu Lys Asn
145                 150                 155                 160
CTA CCT AAA GAT GGC GAT TTA CCC GTT GTT GCG ATG ATT TAC TCC CAT 528
Leu Pro Lys Asp Gly Asp Leu Pro Val Val Ala Met Ile Tyr Ser His
                165                 170                 175
AGC CAT GCG GAC CAC TTT GGC GGA GCT CGC GGT GTT CAA GAG ATG TTC 576
Ser His Ala Asp His Phe Gly Gly Ala Arg Gly Val Gln Glu Met Phe
            180                 185                 190
CCT GAT GTC AAA GTC TAC GGC TCA GAT AAC ATC ACT AAA GAA ATT GTC 624
Pro Asp Val Lys Val Tyr Gly Ser Asp Asn Ile Thr Lys Glu Ile Val
        195                 200                 205
GAT GAG AAC GTA CTT GCC GGT AAC GCC ATG AGC CGC CGC GCA GCT TAT 672
Asp Glu Asn Val Leu Ala Gly Asn Ala Met Ser Arg Arg Ala Ala Tyr
    210                 215                 220
CAA TAC GGC GCA ACA CTG GGC AAA CAT GAC CAC GGT ATT GTT GAT GCT 720
Gln Tyr Gly Ala Thr Leu Gly Lys His Asp His Gly Ile Val Asp Ala
225                 230                 235                 240

GCG CTA GGT AAA GGT CTA TCA AAA GGT GAA ATC ACT TAC GTC GCC CCA 768
Ala Leu Gly Lys Gly Leu Ser Lys Gly Glu Ile Thr Tyr Val Ala Pro
                245                 250                 255
GAC TAC ACC TTA AAC AGT GAA GGC AAA TGG GAA ACG CTG ACG ATT GAT 816
Asp Tyr Thr Leu Asn Ser Glu Gly Lys Trp Glu Thr Leu Thr Ile Asp
            260                 265                 270
GGT CTA GAG ATG GTG TTT ATG GAT GCC TCG GGC ACC GAA GCT GAG TCA 864
Gly Leu Glu Met Val Phe Met Asp Ala Ser Gly Thr Glu Ala Glu Ser
        275                 280                 285
GAA ATG ATC ACT TAT ATT CCC TCT AAA AAA GCG CTC TGG ACG GCG GAG 912
Glu Met Ile Thr Tyr Ile Pro Ser Lys Lys Ala Leu Trp Thr Ala Glu
    290                 295                 300
CTT ACC TAT CAA GGT ATG CAC AAC ATT TAT ACG CTG CGC GGC GCT AAA 960
Leu Thr Tyr Gln Gly Met His Asn Ile Tyr Thr Leu Arg Gly Ala Lys
305                 310                 315                 320
GTA CGT GAT GCG CTC AAG TGG TCA AAA GAT ATC AAC GAA ATG ATC AAT 1008
Val Arg Asp Ala Leu Lys Trp Ser Lys Asp Ile Asn Glu Met Ile Asn
                325                 330                 335
GCC TTT GGT CAA GAT GTC GAA GTG CTG TTT GCC TCG CAC TCT GCG CCA 1056
Ala Phe Gly Gln Asp Val Glu Val Leu Phe Ala Ser His Ser Ala Pro
            340                 345                 350
GTG TGG GGT AAC CAA GCG ATC AAC GAT TTC TTA CGC CTA CAG CGT GAT 1104
Val Trp Gly Asn Gln Ala Ile Asn Asp Phe Leu Arg Leu Gln Arg Asp
        355                 360                 365
AAC TAC GGC CTA GTG CAC AAT CAA ACC TTG AGA CTT GCC AAC GAT GGT 1152
Asn Tyr Gly Leu Val His Asn Gln Thr Leu Arg Leu Ala Asn Asp Gly
    370                 375                 380
GTC GGT ATA CAA GAT ATT GGC GAT GCG ATT CAA GAC ACG ATT CCA GAG 1200
Val Gly Ile Gln Asp Ile Gly Asp Ala Ile Gln Asp Thr Ile Pro Glu
385                 390                 395                 400
TCT ATC TAC AAG ACG TGG CAT ACC AAT GGT TAC CAC GGC ACT TAT AGC 1248
Ser Ile Tyr Lys Thr Trp His Thr Asn Gly Tyr His Gly Thr Tyr Ser
                405                 410                 415
CAT AAC GCT AAA GCG GTT TAT AAC AAG TAT CTA GGC TAC TTC GAT ATG 1296
His Asn Ala Lys Ala Val Tyr Asn Lys Tyr Leu Gly Tyr Phe Asp Met
            420                 425                 430

AAC CCA GCC AAC CTT AAT CCG CTG CCA ACC AAG CAA GAA TCT GCC AAG 1344
Asn Pro Ala Asn Leu Asn Pro Leu Pro Thr Lys Gln Glu Ser Ala Lys
        435                 440                 445
TTT GTC GAA TAC ATG GGC GGC GCA GAT GCC GCA ATT AAG CGC GCT AAA 1392
Phe Val Glu Tyr Met Gly Gly Ala Asp Ala Ala Ile Lys Arg Ala Lys
    450                 455                 460
GAT GAT TAC GCT CAA GGT GAA TAC CGC TTT GTT GCA ACG GCA TTA AAT 1440
Asp Asp Tyr Ala Gln Gly Glu Tyr Arg Phe Val Ala Thr Ala Leu Asn
465                 470                 475                 480
AAG GTG GTG ATG GCC GAG CCA GAA AAT GAC TCC GCT CGT CAA TTG CTA 1488
Lys Val Val Met Ala Glu Pro Glu Asn Asp Ser Ala Arg Gln Leu Leu
                485                 490                 495
GCC GAT ACC TAT GAG CAA CTT GGT TAT CAA GCA GAA GGG GCT GGC TGG 1536
Ala Asp Thr Tyr Glu Gln Leu Gly Tyr Gln Ala Glu Gly Ala Gly Trp
            500                 505                 510
AGA AAC ATT TAC TTA ACT GGC GCA CAA GAG CTA CGA GTA GGT ATT CAA 1584
Arg Asn Ile Tyr Leu Thr Gly Ala Gln Glu Leu Arg Val Gly Ile Gln
        515                 520                 525
GCT GGC GCG CCT AAA ACC GCA TCG GCA GAT GTC ATC AGT GAA ATG GAC 1632
Ala Gly Ala Pro Lys Thr Ala Ser Ala Asp Val Ile Ser Glu Met Asp
    530                 535                 540
ATG CCG ACT CTA TTT GAC TTC CTC GCG GTG AAG ATT GAT AGT CAA CAG 1680
Met Pro Thr Leu Phe Asp Phe Leu Ala Val Lys Ile Asp Ser Gln Gln
545                 550                 555                 560
GCG GCT AAG CAC GGC TTA GTT AAG ATG AAT GTT ATC ACC CCT GAT ACT 1728
Ala Ala Lys His Gly Leu Val Lys Met Asn Val Ile Thr Pro Asp Thr
                565                 570                 575
AAA GAT ATT CTC TAT ATT GAG CTA AGC AAC GGT AAC TTA AGC AAC GCA 1776
Lys Asp Ile Leu Tyr Ile Glu Leu Ser Asn Gly Asn Leu Ser Asn Ala
            580                 585                 590
GTG GTC GAC AAA GAG CAA GCA GCT GAC GCA AAC CTT ATG GTT AAT AAA 1824
Val Val Asp Lys Glu Gln Ala Ala Asp Ala Asn Leu Met Val Asn Lys
        595                 600                 605
GCT GAC GTT AAC CGC ATC TTA CTT GGC CAA GTA ACC CTA AAA GCG TTA 1872
Ala Asp Val Asn Arg Ile Leu Leu Gly Gln Val Thr Leu Lys Ala Leu
    610                 615                 620

TTA GCC AGC GGC GAT GCC AAG CTC ACT GGT GAT AAA ACG GCA TTT AGT 1920
Leu Ala Ser Gly Asp Ala Lys Leu Thr Gly Asp Lys Thr Ala Phe Ser
625                 630                 635                 640
AAA ATA GCC GAT AGC ATG GTC GAG TTT ACA CCT GAC TTC GAA ATC GTA 1968
Lys Ile Ala Asp Ser Met Val Glu Phe Thr Pro Asp Phe Glu Ile Val
                645                 650                 655
CCA ACG CCT GTT AAA 1983
Pro Thr Pro Val Lys
            660

SEQ ID NO: 3
SEQUENCE LENGTH: 2910
SEQUENCE TYPE: Nucleic acid
STRANDNESS: Double strand
TOPOLOGY: Linear
MOLECULE TYPE: Genomic DNA
ORIGINAL SOURCE: Shewanella putrefaciens SCRC-2874
(FERM BP-1625)

SEQUENCE
ATG AGT ATG TTT TTA AAT TCA AAA CTT TCG CGC TCA GTC AAA CTT GCC 48
Met Ser Met Phe Leu Asn Ser Lys Leu Ser Arg Ser Val Lys Leu Ala
1               5                   10                  15
ATA TCC GCA GGC TTA ACA GCC TCG CTA GCT ATG CCT GTT TTT GCA GAA 96
Ile Ser Ala Gly Leu Thr Ala Ser Leu Ala Met Pro Val Phe Ala Glu
            20                  25                  30
GAA ACT GCT GCT GAA GAA CAA ATA GAA AGA GTC GCA GTG ACC GGA TCG 144
Glu Thr Ala Ala Glu Glu Gln Ile Glu Arg Val Ala Val Thr Gly Ser
        35                  40                  45
CGA ATC GCT AAA GCA GAG CTA ACT CAA CCA GCT CCA GTC GTC AGC CTT 192
Arg Ile Ala Lys Ala Glu Leu Thr Gln Pro Ala Pro Val Val Ser Leu
    50                  55                  60
TCA GCC GAA GAA CTG ACA AAA TTT GGT AAT CAA GAT TTA GGT AGC GTA 240
Ser Ala Glu Glu Leu Thr Lys Phe Gly Asn Gln Asp Leu Gly Ser Val
65                  70                  75                  80

CTA GCA GAA TTA CCT GCT ATT GGT GCA ACC AAC ACT ATT ATT GGT AAT 288
Leu Ala Glu Leu Pro Ala Ile Gly Ala Thr Asn Thr Ile Ile Gly Asn
                85                  90                  95
AAC AAT AGC AAC TCA AGC GCA GGT GTT AGC TCA GCA GAC TTG CGT CGT 336
Asn Asn Ser Asn Ser Ser Ala Gly Val Ser Ser Ala Asp Leu Arg Arg
            100                 105                 110
CTA GGT GCT AAC AGA ACC TTA GTA TTA GTC AAC GGT AAG CGC TAC GTT 384
Leu Gly Ala Asn Arg Thr Leu Val Leu Val Asn Gly Lys Arg Tyr Val
        115                 120                 125
GCC GGC CAA CCG GGC TCA GCT GAG GTA GAT TTG TCA ACT ATA CCA ACT 432
Ala Gly Gln Pro Gly Ser Ala Glu Val Asp Leu Ser Thr Ile Pro Thr
    130                 135                 140
AGC ATG ATC TCG CGA GTT GAG ATT GTA ACC GGC GGT GCT TCA GCA ATT 480
Ser Met Ile Ser Arg Val Glu Ile Val Thr Gly Gly Ala Ser Ala Ile
145                 150                 155                 160
TAT GGT TCG GAC GCT GTA TCA GGT GTT ATC AAC GTT ATC CTT AAA GAA 528
Tyr Gly Ser Asp Ala Val Ser Gly Val Ile Asn Val Ile Leu Lys Glu
                165                 170                 175
GAC TTT GAA GGC TTT GAG TTT AAC GCA CGT ACT AGC GGT TCT ACT GAA 576
Asp Phe Glu Gly Phe Glu Phe Asn Ala Arg Thr Ser Gly Ser Thr Glu
            180                 185                 190
AGT GTA GGC ACT CAA GAG CAC TCT TTT GAC ATT TTG GGT GGT GCA AAC 624
Ser Val Gly Thr Gln Glu His Ser Phe Asp Ile Leu Gly Gly Ala Asn
        195                 200                 205
GTT GCA GAT GGA CGT GGT AAT GTA ACC TTC TAC GCA GGT TAT GAA CGT 672
Val Ala Asp Gly Arg Gly Asn Val Thr Phe Tyr Ala Gly Tyr Glu Arg
    210                 215                 220
ACA AAA GAA GTC ATG GCT ACC GAC ATT CGC CAA TTC GAT GCT TGG GGA 720
Thr Lys Glu Val Met Ala Thr Asp Ile Arg Gln Phe Asp Ala Trp Gly
225                 230                 235                 240
ACA ATT AAA AAC GAA GCC GAT GGT GGT GAA GAT GAT GGT ATT CCA GAC 768
Thr Ile Lys Asn Glu Ala Asp Gly Gly Glu Asp Asp Gly Ile Pro Asp
                245                 250                 255
AGA CTA CGT GTA CCA CGA GTT TAT TCT GAA ATG ATT AAT GCT ACC GGT 816
Arg Leu Arg Val Pro Arg Val Tyr Ser Glu Met Ile Asn Ala Thr Gly
            260                 265                 270

GTT ATC AAT GCA TTT GGT GGT GGA ATT GGT CGC TCA ACC TTT GAC AGT 864
Val Ile Asn Ala Phe Gly Gly Gly Ile Gly Arg Ser Thr Phe Asp Ser
        275                 280                 285
AAC GGC AAT CCT ATT GCA CAA CAA GAA CGT GAT GGG ACT AAC AGC TTT 912
Asn Gly Asn Pro Ile Ala Gln Gln Glu Arg Asp Gly Thr Asn Ser Phe
    290                 295                 300
GCA TTT GGT TCA TTC CCT AAT GGC TGT GAC ACA TGT TTC AAC ACT GAA 960
Ala Phe Gly Ser Phe Pro Asn Gly Cys Asp Thr Cys Phe Asn Thr Glu
305                 310                 315                 320
GCA TAC GAA AAC TAT ATT CCA GGG GTA GAA AGA ATA AAC GTT GGC TCA 1008
Ala Tyr Glu Asn Tyr Ile Pro Gly Val Glu Arg Ile Asn Val Gly Ser
                325                 330                 335
TCA TTC AAC TTT GAT TTT ACC GAT AAC ATT CAA TTT TAC ACT GAC TTC 1056
Ser Phe Asn Phe Asp Phe Thr Asp Asn Ile Gln Phe Tyr Thr Asp Phe
            340                 345                 350
AGA TAT GTA AAG TCA GAT ATT CAG CAA CAA TTT CAG CCT TCA TTC CGT 1104
Arg Tyr Val Lys Ser Asp Ile Gln Gln Gln Phe Gln Pro Ser Phe Arg
        355                 360                 365
TTT GGT AAC ATT AAT ATC AAT GTT GAA GAT AAC GCC TTT TTG AAT GAC 1152
Phe Gly Asn Ile Asn Ile Asn Val Glu Asp Asn Ala Phe Leu Asn Asp
    370                 375                 380
GAC TTG CGT CAG CAA ATG CTC GAT GCG GGT CAA ACC AAT GCT AGT TTT 1200
Asp Leu Arg Gln Gln Met Leu Asp Ala Gly Gln Thr Asn Ala Ser Phe
385                 390                 395                 400
GCC AAG TTT TTT GAT GAA TTA GGA AAT CGC TCA GCA GAA AAT AAA CGC 1248
Ala Lys Phe Phe Asp Glu Leu Gly Asn Arg Ser Ala Glu Asn Lys Arg
                405                 410                 415
GAA CTT TTC CGT TAC GTA GGT GGC TTT AAA GGT GGC TTT GAT ATT AGC 1296
Glu Leu Phe Arg Tyr Val Gly Gly Phe Lys Gly Gly Phe Asp Ile Ser
            420                 425                 430
GAA ACC ATA TTT GAT TAC GAC CTT TAC TAT GTT TAT GGC GAG ACT AAT 1344
Glu Thr Ile Phe Asp Tyr Asp Leu Tyr Tyr Val Tyr Gly Glu Thr Asn
        435                 440                 445
AAC CGT CGT AAA ACC CTT AAT GAC CTA ATT CCT GAT AAC TTT GTC GCA 1392
Asn Arg Arg Lys Thr Leu Asn Asp Leu Ile Pro Asp Asn Phe Val Ala
    450                 455                 460

GCT GTC GAC TCT GTT ATT GAT CCT GAT ACT GGC TTA GCA GCG TGT CGC 1440
Ala Val Asp Ser Val Ile Asp Pro Asp Thr Gly Leu Ala Ala Cys Arg
465                 470                 475                 480
TCA CAA GTA GCA AGC GCT CAA GGC GAT GAC TAT ACA GAT CCC GCG TCT 1488
Ser Gln Val Ala Ser Ala Gln Gly Asp Asp Tyr Thr Asp Pro Ala Ser
                485                 490                 495
GTA AAT GGT AGC GAC TGT GTT GCT TAT AAC CCA TTT GGC ATG GGT CAA 1536
Val Asn Gly Ser Asp Cys Val Ala Tyr Asn Pro Phe Gly Met Gly Gln
            500                 505                 510
GCT TCA GCA GAA GCC CGC GAC TGG GTT TCT GCT GAT GTG ACT CGT GAA 1584
Ala Ser Ala Glu Ala Arg Asp Trp Val Ser Ala Asp Val Thr Arg Glu
        515                 520                 525
GAC AAA ATA ACT CAA CAA GTG ATT GGT GGT ACT CTC GGT ACC GAT TCT 1632
Asp Lys Ile Thr Gln Gln Val Ile Gly Gly Thr Leu Gly Thr Asp Ser
    530                 535                 540
GAA GAA CTA TTT GAG CTT CAA GGT GGT GCA ATC GCT ATG GTT GTT GGT 1680
Glu Glu Leu Phe Glu Leu Gln Gly Gly Ala Ile Ala Met Val Val Gly
545                 550                 555                 560
TTT GAA TAC CGT GAA GAA ACG TCT GGT TCA ACA ACC GAT GAA TTT ACT 1728
Phe Glu Tyr Arg Glu Glu Thr Ser Gly Ser Thr Thr Asp Glu Phe Thr
                565                 570                 575
AAA GCA GGT TTC TTG ACA AGC GCT GCA ACG CCA GAT TCT TAT GGC GAA 1776
Lys Ala Gly Phe Leu Thr Ser Ala Ala Thr Pro Asp Ser Tyr Gly Glu
            580                 585                 590
TAC GAC GTG ACT GAG TAT TTT GTT GAG GTG AAC ATC CCA GTA CTA AAA 1824
Tyr Asp Val Thr Glu Tyr Phe Val Glu Val Asn Ile Pro Val Leu Lys
        595                 600                 605
GAA TTA CCT TTT GCA CAT GAG TTG AGC TTT GAC GGT GCA TAC CGT AAT 1872
Glu Leu Pro Phe Ala His Glu Leu Ser Phe Asp Gly Ala Tyr Arg Asn
    610                 615                 620
GCT GAT TAC TCA CAT GCC GGT AAG ACT GAA GCA TGG AAA GCT GGT ATG 1920
Ala Asp Tyr Ser His Ala Gly Lys Thr Glu Ala Trp Lys Ala Gly Met
625                 630                 635                 640
TTC TAC TCA CCA TTA GAG CAA CTT GCA TTA CGT GGT ACG GTA GGT GAA 1968
Phe Tyr Ser Pro Leu Glu Gln Leu Ala Leu Arg Gly Thr Val Gly Glu
                645                 650                 655

GCA GTA CGA GCA CCA AAC ATT GCA GAA GCC TTT AGT CCA CGC TCT CCT 2016
Ala Val Arg Ala Pro Asn Ile Ala Glu Ala Phe Ser Pro Arg Ser Pro
            660                 665                 670
GGT TTT GGC CGC GTT TCA GAT CCA TGT GAT GCA GAT AAC ATT AAT GAC 2064
Gly Phe Gly Arg Val Ser Asp Pro Cys Asp Ala Asp Asn Ile Asn Asp
        675                 680                 685
GAT CCG GAT CGC GTG TCA AAC TGT GCA GCA TTG GGG ATC CCT CCA GGA 2112
Asp Pro Asp Arg Val Ser Asn Cys Ala Ala Leu Gly Ile Pro Pro Gly
    690                 695                 700
TTC CAA GCT AAT GAT AAC GTC AGT GTA GAT ACC TTA TCT GGT GGT AAC 2160
Phe Gln Ala Asn Asp Asn Val Ser Val Asp Thr Leu Ser Gly Gly Asn
705                 710                 715                 720
CCA GAT CTA AAA CCT GAA ACA TCA ACA TCC TTT ACA GGT GGT CTT GTT 2208
Pro Asp Leu Lys Pro Glu Thr Ser Thr Ser Phe Thr Gly Gly Leu Val
                725                 730                 735
TGG ACA CCA ACG TTT GCT GAC AAT CTA TCA TTC ACT GTC GAT TAT TAT 2256
Trp Thr Pro Thr Phe Ala Asp Asn Leu Ser Phe Thr Val Asp Tyr Tyr
            740                 745                 750
GAT ATT CAA ATT GAG GAT GCT ATT TTG TCA GTA GCC ACC CAG ACT GTG 2304
Asp Ile Gln Ile Glu Asp Ala Ile Leu Ser Val Ala Thr Gln Thr Val
        755                 760                 765
GCT GAT AAC TGT GTT GAC TCA ACT GGC GGA CCT GAC ACC GAC TTC TGT 2352
Ala Asp Asn Cys Val Asp Ser Thr Gly Gly Pro Asp Thr Asp Phe Cys
    770                 775                 780
AGT CAA GTT GAT CGT AAT CCA ACG ACC TAT GAT ATT GAA CTT GTT CGC 2400
Ser Gln Val Asp Arg Asn Pro Thr Thr Tyr Asp Ile Glu Leu Val Arg
785                 790                 795                 800
TCT GGT TAT CTA AAT GCC GCG GCA TTG AAT ACC AAA GGT ATT GAA TTT 2448
Ser Gly Tyr Leu Asn Ala Ala Ala Leu Asn Thr Lys Gly Ile Glu Phe
                805                 810                 815
CAA GCT GCA TAC TCA TTA GAT CTA GAG TCT TTC AAC GCG CCT GGT GAA 2496
Gln Ala Ala Tyr Ser Leu Asp Leu Glu Ser Phe Asn Ala Pro Gly Glu
            820                 825                 830
CTA CGC TTC AAC CTA TTG GGG AAC CAA TTA CTT GAA CTA GAA CGT CTT 2544
Leu Arg Phe Asn Leu Leu Gly Asn Gln Leu Leu Glu Leu Glu Arg Leu
        835                 840                 845

GAA TTC CAA AAT CGT CCT GAT GAG ATT AAT GAT GAA AAA GGC GAA GTA 2592
Glu Phe Gln Asn Arg Pro Asp Glu Ile Asn Asp Glu Lys Gly Glu Val
    850                 855                 860
GGT GAT CCA GAG CTG CAG TTC CGC CTA GGC ATC GAT TAC CGT CTA GAT 2640
Gly Asp Pro Glu Leu Gln Phe Arg Leu Gly Ile Asp Tyr Arg Leu Asp
865                 870                 875                 880
GAT CTA AGT GTT AGC TGG AAC ACG CGT TAT ATT GAT AGC GTA GTA ACT 2688
Asp Leu Ser Val Ser Trp Asn Thr Arg Tyr Ile Asp Ser Val Val Thr
                885                 890                 895
TAT GAT GTC TCT GAA AAT GGT GGC TCT CCT GAA GAT TTA TAT CCA GGC 2736
Tyr Asp Val Ser Glu Asn Gly Gly Ser Pro Glu Asp Leu Tyr Pro Gly
            900                 905                 910
CAC ATA GGC TCA ATG ACA ACT CAT GAC TTG AGC GCT ACA TAC TAC ATC 2784
His Ile Gly Ser Met Thr Thr His Asp Leu Ser Ala Thr Tyr Tyr Ile
        915                 920                 925
AAT GAG AAC TTC ATG ATT AAC GGT GGT GTA CGT AAC CTA TTT GAC GCA 2832
Asn Glu Asn Phe Met Ile Asn Gly Gly Val Arg Asn Leu Phe Asp Ala
    930                 935                 940
CTT CCA CCT GGA TAC ACT AAC GAT GCG CTA TAT GAT CTA GTT GGT CGC 2880
Leu Pro Pro Gly Tyr Thr Asn Asp Ala Leu Tyr Asp Leu Val Gly Arg
945                 950                 955                 960
CGT GCA TTC CTA GGT ATT AAG GTA ATG ATG 2910
Arg Ala Phe Leu Gly Ile Lys Val Met Met
                965                 970

SEQ ID NO: 4
SEQUENCE LENGTH: 4902
SEQUENCE TYPE: Nucleic acid
STRANDNESS: Double strand
TOPOLOGY: Linear
MOLECULE TYPE: Genomic DNA
ORIGINAL SOURCE: Shewanella putrefaciens SCRC-2874
(FERM BP-1625)

SEQUENCE

ATG AGC CAG ACC TCT AAA CCT ACA AAC TCA GCA ACT GAG CAA GCA CAA 48
Met Ser Gln Thr Ser Lys Pro Thr Asn Ser Ala Thr Glu Gln Ala Gln
1               5                   10                  15
GAC TCA CAA GCT GAC TCT CGT TTA AAT AAA CGA CTA AAA GAT ATG CCA 96
Asp Ser Gln Ala Asp Ser Arg Leu Asn Lys Arg Leu Lys Asp Met Pro
            20                  25                  30
ATT GCT ATT GTT GGC ATG GCG AGT ATT TTT GCA AAC TCT CGC TAT TTG 144
Ile Ala Ile Val Gly Met Ala Ser Ile Phe Ala Asn Ser Arg Tyr Leu
        35                  40                  45
AAT AAG TTT TGG GAC TTA ATC AGC GAA AAA ATT GAT GCG ATT ACT GAA 192
Asn Lys Phe Trp Asp Leu Ile Ser Glu Lys Ile Asp Ala Ile Thr Glu
    50                  55                  60
TTA CCA TCA ACT CAC TGG CAG CCT GAA GAA TAT TAC GAC GCA GAT AAA 240
Leu Pro Ser Thr His Trp Gln Pro Glu Glu Tyr Tyr Asp Ala Asp Lys
65                  70                  75                  80
ACC GCA GCA GAC AAA AGC TAC TGT AAA CGT GGT GGC TTT TTG CCA GAT 288
Thr Ala Ala Asp Lys Ser Tyr Cys Lys Arg Gly Gly Phe Leu Pro Asp
                85                  90                  95
GTA GAC TTC AAC CCA ATG GAG TTT GGC CTG CCG CCA AAC ATT TTG GAA 336
Val Asp Phe Asn Pro Met Glu Phe Gly Leu Pro Pro Asn Ile Leu Glu
            100                 105                 110
CTG ACC GAT TCA TCG CAA CTA TTA TCA CTC ATC GTT GCT AAA GAA GTG 384
Leu Thr Asp Ser Ser Gln Leu Leu Ser Leu Ile Val Ala Lys Glu Val
        115                 120                 125
TTG GCT GAT GCT AAC TTA CCT GAG AAT TAC GAC CGC GAT AAA ATT GGT 432
Leu Ala Asp Ala Asn Leu Pro Glu Asn Tyr Asp Arg Asp Lys Ile Gly
    130                 135                 140
ATC ACC TTA GGT GTC GGC GGT GGT CAA AAA ATT AGC CAC AGC CTA ACA 480
Ile Thr Leu Gly Val Gly Gly Gly Gln Lys Ile Ser His Ser Leu Thr
145                 150                 155                 160
GCG CGT CTG CAA TAC CCA GTA TTG AAG AAA GTA TTC GCC AAT AGC GGC 528
Ala Arg Leu Gln Tyr Pro Val Leu Lys Lys Val Phe Ala Asn Ser Gly
                165                 170                 175
ATT AGT GAC ACC GAC AGC GAA ATG CTT ATC AAG AAA TTC CAA GAC CAA 576
Ile Ser Asp Thr Asp Ser Glu Met Leu Ile Lys Lys Phe Gln Asp Gln
            180                 185                 190

TAT GTA CAC TGG GAA GAA AAC TCG TTC CCA GGT TCA CTT GGT AAC GTT 624
Tyr Val His Trp Glu Glu Asn Ser Phe Pro Gly Ser Leu Gly Asn Val
        195                 200                 205
ATT GCG GGC CGT ATC GCC AAC CGC TTC GAT TTT GGC GGC ATG AAC TGT 672
Ile Ala Gly Arg Ile Ala Asn Arg Phe Asp Phe Gly Gly Met Asn Cys
    210                 215                 220
GTG GTT GAT GCT GCC TGT GCT GGA TCA CTT GCT GCT ATG CGT ATG GCG 720
Val Val Asp Ala Ala Cys Ala Gly Ser Leu Ala Ala Met Arg Met Ala
225                 230                 235                 240
CTA ACA GAG CTA ACT GAA GGT CGC TCT GAA ATG ATG ATC ACC GGT GGT 768
Leu Thr Glu Leu Thr Glu Gly Arg Ser Glu Met Met Ile Thr Gly Gly
                245                 250                 255
GTG TGT ACT GAT AAC TCA CCC TCT ATG TAT ATG AGC TTT TCA AAA ACG 816
Val Cys Thr Asp Asn Ser Pro Ser Met Tyr Met Ser Phe Ser Lys Thr
            260                 265                 270
CCC GCC TTT ACC ACT AAC GAA ACC ATT CAG CCA TTT GAT ATC GAC TCA 864
Pro Ala Phe Thr Thr Asn Glu Thr Ile Gln Pro Phe Asp Ile Asp Ser
        275                 280                 285
AAA GGC ATG ATG ATT GGT GAA GGT ATT GGC ATG GTG GCG CTA AAG CGT 912
Lys Gly Met Met Ile Gly Glu Gly Ile Gly Met Val Ala Leu Lys Arg
    290                 295                 300
CTT GAA GAT GCA GAG CGC GAT GGC GAC CGC ATT TAC TCT GTA ATT AAA 960
Leu Glu Asp Ala Glu Arg Asp Gly Asp Arg Ile Tyr Ser Val Ile Lys
305                 310                 315                 320
GGT GTG GGT GCA TCA TCT GAC GGT AAG TTT AAA TCA ATC TAT GCC CCT 1008
Gly Val Gly Ala Ser Ser Asp Gly Lys Phe Lys Ser Ile Tyr Ala Pro
                325                 330                 335
CGC CCA TCA GGC CAA GCT AAA GCA CTT AAC CGT GCC TAT GAT GAC GCA 1056
Arg Pro Ser Gly Gln Ala Lys Ala Leu Asn Arg Ala Tyr Asp Asp Ala
            340                 345                 350
GGT TTT GCG CCG CAT ACC TTA GGT CTA ATT GAA GCT CAC GGA ACA GGT 1104
Gly Phe Ala Pro His Thr Leu Gly Leu Ile Glu Ala His Gly Thr Gly
        355                 360                 365
ACT GCA GCA GGT GAC GCG GCA GAG TTT GCC GGC CTT TGC TCA GTA TTT 1152
Thr Ala Ala Gly Asp Ala Ala Glu Phe Ala Gly Leu Cys Ser Val Phe
    370                 375                 380

GCT GAA GGC AAC GAT ACC AAG CAA CAC ATT GCG CTA GGT TCA GTT AAA 1200
Ala Glu Gly Asn Asp Thr Lys Gln His Ile Ala Leu Gly Ser Val Lys
385                 390                 395                 400
TCA CAA ATT GGT CAT ACT AAA TCA ACT GCA GGT ACA GCA GGT TTA ATT 1248
Ser Gln Ile Gly His Thr Lys Ser Thr Ala Gly Thr Ala Gly Leu Ile
                405                 410                 415
AAA GCT GCT CTT GCT TTG CAT CAC AAG GTA CTG CCG CCG ACC ATT AAC 1296
Lys Ala Ala Leu Ala Leu His His Lys Val Leu Pro Pro Thr Ile Asn
            420                 425                 430
GTT AGT CAG CCA AGC CCT AAA CTT GAT ATC GAA AAC TCA CCG TTT TAT 1344
Val Ser Gln Pro Ser Pro Lys Leu Asp Ile Glu Asn Ser Pro Phe Tyr
        435                 440                 445
CTA AAC ACT GAG ACT CGT CCA TGG TTA CCA CGT GTT GAT GGT ACG CCG 1392
Leu Asn Thr Glu Thr Arg Pro Trp Leu Pro Arg Val Asp Gly Thr Pro
    450                 455                 460
CGC CGC GCG GGT ATT AGC TCA TTT GGT TTT GGT GGC ACT AAC TTC CAT 1440
Arg Arg Ala Gly Ile Ser Ser Phe Gly Phe Gly Gly Thr Asn Phe His
465                 470                 475                 480
TTT GTA CTA GAA GAG TAC AAC CAA GAA CAC AGC CGT ACT GAT AGC GAA 1488
Phe Val Leu Glu Glu Tyr Asn Gln Glu His Ser Arg Thr Asp Ser Glu
                485                 490                 495
AAA GCT AAG TAT CGT CAA CGC CAA GTG GCG CAA AGC TTC CTT GTT AGC 1536
Lys Ala Lys Tyr Arg Gln Arg Gln Val Ala Gln Ser Phe Leu Val Ser
            500                 505                 510
GCA AGC GAT AAA GCA TCG CTA ATT AAC GAG TTA AAC GTA CTA GCA GCA 1584
Ala Ser Asp Lys Ala Ser Leu Ile Asn Glu Leu Asn Val Leu Ala Ala
        515                 520                 525
TCT GCA AGC CAA GCT GAG TTT ATC CTC AAA GAT GCA GCA GCA AAC TAT 1632
Ser Ala Ser Gln Ala Glu Phe Ile Leu Lys Asp Ala Ala Ala Asn Tyr
    530                 535                 540
GGC GTA CGT GAG CTT GAT AAA AAT GCA CCA CGG ATC GGT TTA GTT GCA 1680
Gly Val Arg Glu Leu Asp Lys Asn Ala Pro Arg Ile Gly Leu Val Ala
545                 550                 555                 560
AAC ACA GCT GAA GAG TTA GCA GGC CTA ATT AAG CAA GCA CTT GCC AAA 1728
Asn Thr Ala Glu Glu Leu Ala Gly Leu Ile Lys Gln Ala Leu Ala Lys
                565                 570                 575

CTA GCA GCT AGC GAT GAT AAC GCA TGG CAG CTA CCT GGT GGC ACT AGC 1776
Leu Ala Ala Ser Asp Asp Asn Ala Trp Gln Leu Pro Gly Gly Thr Ser
            580                 585                 590
TAC CGC GCC GCT GCA GTA GAA GGT AAA GTT GCC GCA CTG TTT GCT GGC 1824
Tyr Arg Ala Ala Ala Val Glu Gly Lys Val Ala Ala Leu Phe Ala Gly
        595                 600                 605
CAA GGT TCA CAA TAT CTC AAT ATG GGC CGT GAC CTT ACT TGT TAT TAC 1872
Gln Gly Ser Gln Tyr Leu Asn Met Gly Arg Asp Leu Thr Cys Tyr Tyr
    610                 615                 620
CCA GAG ATG CGT CAG CAA TTT GTA ACT GCA GAT AAA GTA TTT GCC GCA 1920
Pro Glu Met Arg Gln Gln Phe Val Thr Ala Asp Lys Val Phe Ala Ala
625                 630                 635                 640
AAT GAT AAA ACG CCG TTA TCG CAA ACT CTG TAT CCA AAG CCT GTA TTT 1968
Asn Asp Lys Thr Pro Leu Ser Gln Thr Leu Tyr Pro Lys Pro Val Phe
                645                 650                 655
AAT AAA GAT GAA TTA AAG GCT CAA GAA GCC ATT TTG ACC AAT ACC GCC 2016
Asn Lys Asp Glu Leu Lys Ala Gln Glu Ala Ile Leu Thr Asn Thr Ala
            660                 665                 670
AAT GCC CAA AGC GCA ATT GGT GCG ATT TCA ATG GGT CAA TAC GAT TTG 2064
Asn Ala Gln Ser Ala Ile Gly Ala Ile Ser Met Gly Gln Tyr Asp Leu
        675                 680                 685
TTT ACT GCG GCT GGC TTT AAT GCC GAC ATG GTT GCA GGC CAT AGC TTT 2112
Phe Thr Ala Ala Gly Phe Asn Ala Asp Met Val Ala Gly His Ser Phe
    690                 695                 700
GGT GAG CTA AGT GCA CTG TGT GCT GCA GGT GTT ATT TCA GCT GAT GAC 2160
Gly Glu Leu Ser Ala Leu Cys Ala Ala Gly Val Ile Ser Ala Asp Asp
705                 710                 715                 720
TAC TAC AAG CTG GCT TTT GCT CGT GGT GAG GCT ATG GCA ACA AAA GCA 2208
Tyr Tyr Lys Leu Ala Phe Ala Arg Gly Glu Ala Met Ala Thr Lys Ala
                725                 730                 735
CCG GCT AAA GAC GGC GTT GAA GCA GAT GCA GGA GCA ATG TTT GCA ATC 2256
Pro Ala Lys Asp Gly Val Glu Ala Asp Ala Gly Ala Met Phe Ala Ile
            740                 745                 750
ATA ACC AAG AGT GCT GCA GAC CTT GAA ACC GTT GAA GCC ACC ATC GCT 2304
Ile Thr Lys Ser Ala Ala Asp Leu Glu Thr Val Glu Ala Thr Ile Ala
        755                 760                 765

AAA TTT GAT GGG GTG AAA GTC GCT AAC TAT AAC GCG CCA ACG CAA TCA 2352
Lys Phe Asp Gly Val Lys Val Ala Asn Tyr Asn Ala Pro Thr Gln Ser
    770                 775                 780
GTA ATT GCA GGC CCA ACA GCA ACT ACC GCT GAT GCG GCT AAA GCG CTA 2400
Val Ile Ala Gly Pro Thr Ala Thr Thr Ala Asp Ala Ala Lys Ala Leu
785                 790                 795                 800
ACT GAG CTT GGT TAC AAA GCG ATT AAC CTG CCA GTA TCA GGT GCA TTC 2448
Thr Glu Leu Gly Tyr Lys Ala Ile Asn Leu Pro Val Ser Gly Ala Phe
                805                 810                 815
CAC ACT GAA CTT GTT GGT CAC GCT CAA GCG CCA TTT GCT AAA GCG ATT 2496
His Thr Glu Leu Val Gly His Ala Gln Ala Pro Phe Ala Lys Ala Ile
            820                 825                 830
GAC GCA GCC AAA TTT ACT AAA ACA AGC CGA GCA CTT TAC TCA AAT GCA 2544
Asp Ala Ala Lys Phe Thr Lys Thr Ser Arg Ala Leu Tyr Ser Asn Ala
        835                 840                 845
ACT GGC GGA CTT TAT GAA AGC ACT GCT GCA AAG ATT AAA GCC TCG TTT 2592
Thr Gly Gly Leu Tyr Glu Ser Thr Ala Ala Lys Ile Lys Ala Ser Phe
    850                 855                 860
AAG AAA CAT ATG CTT CAA TCA GTG CGC TTT ACT AGC CAG CTA GAA GCC 2640
Lys Lys His Met Leu Gln Ser Val Arg Phe Thr Ser Gln Leu Glu Ala
865                 870                 875                 880
ATG TAC AAC GAC GGC GCC CGT GTA TTT GTT GAA TTT GGT CCA AAG AAC 2688
Met Tyr Asn Asp Gly Ala Arg Val Phe Val Glu Phe Gly Pro Lys Asn
                885                 890                 895
ATC TTA CAA AAA TTA GTT CAA GGC ACG CTT GTC AAC ACT GAA AAT GAA 2736
Ile Leu Gln Lys Leu Val Gln Gly Thr Leu Val Asn Thr Glu Asn Glu
            900                 905                 910
GTT TGC ACT ATC TCT ATC AAC CCT AAT CCT AAA GTT GAT AGT GAT CTG 2784
Val Cys Thr Ile Ser Ile Asn Pro Asn Pro Lys Val Asp Ser Asp Leu
        915                 920                 925
CAG CTT AAG CAA GCA GCA ATG CAG CTA GCG GTT ACT GGT GTG GTA CTC 2832
Gln Leu Lys Gln Ala Ala Met Gln Leu Ala Val Thr Gly Val Val Leu
    930                 935                 940
AGT GAA ATT GAC CCA TAC CAA GCC GAT ATT GCC GCA CCA GCG AAA AAG 2880
Ser Glu Ile Asp Pro Tyr Gln Ala Asp Ile Ala Ala Pro Ala Lys Lys
945                 950                 955                 960

TCG CCA ATG AGC ATT TCG CTT AAT GCT GCT AAC CAT ATC AGC AAA GCA 2928
Ser Pro Met Ser Ile Ser Leu Asn Ala Ala Asn His Ile Ser Lys Ala
                965                 970                 975
ACT CGC GCT AAG ATG GCC AAG TCT TTA GAG ACA GGT ATC GTC ACC TCG 2976
Thr Arg Ala Lys Met Ala Lys Ser Leu Glu Thr Gly Ile Val Thr Ser
            980                 985                 990
CAA ATA GAA CAT GTT ATT GAA GAA AAA ATC GTT GAA GTT GAG AAA CTG 3024
Gln Ile Glu His Val Ile Glu Glu Lys Ile Val Glu Val Glu Lys Leu
        995                 1000                1005
GTT GAA GTC GAA AAG ATC GTC GAA AAA GTG GTT GAA GTA GAG AAA GTT 3072
Val Glu Val Glu Lys Ile Val Glu Lys Val Val Glu Val Glu Lys Val
    1010                1015                1020
GTT GAG GTT GAA GCT CCT GTT AAT TCA GTG CAA GCC AAT GCA ATT CAA 3120
Val Glu Val Glu Ala Pro Val Asn Ser Val Gln Ala Asn Ala Ile Gln
1025                1030                1035                1040
ACC CGT TCA GTT GTC GCT CCA GTA ATA GAG AAC CAA GTC GTG TCT AAA 3168
Thr Arg Ser Val Val Ala Pro Val Ile Glu Asn Gln Val Val Ser Lys
                1045                1050                1055
AAC AGT AAG CCA GCA GTC CAG AGC ATT AGT GGT GAT GCA CTC AGC AAC 3216
Asn Ser Lys Pro Ala Val Gln Ser Ile Ser Gly Asp Ala Leu Ser Asn
            1060                1065                1070
TTT TTT GCT GCA CAG CAG CAA ACC GCA CAG TTG CAT CAG CAG TTC TTA 3264
Phe Phe Ala Ala Gln Gln Gln Thr Ala Gln Leu His Gln Gln Phe Leu
        1075                1080                1085
GCT ATT CCG CAG CAA TAT GGT GAG ACG TTC ACT ACG CTG ATG ACC GAG 3312
Ala Ile Pro Gln Gln Tyr Gly Glu Thr Phe Thr Thr Leu Met Thr Glu
    1090                1095                1100
CAA GCT AAA CTG GCA AGT TCT GGT GTT GCA ATT CCA GAG AGT CTG CAA 3360
Gln Ala Lys Leu Ala Ser Ser Gly Val Ala Ile Pro Glu Ser Leu Gln
1105                1110                1115                1120
CGC TCA ATG GAG CAA TTC CAC CAA CTA CAA GCG CAA ACA CTA CAA AGC 3408
Arg Ser Met Glu Gln Phe His Gln Leu Gln Ala Gln Thr Leu Gln Ser
                1125                1130                1135
CAC ACC CAG TTC CTT GAG ATG CAA GCG GGT AGC AAC ATT GCA GCG TTA 3456
His Thr Gln Phe Leu Glu Met Gln Ala Gly Ser Asn Ile Ala Ala Leu
            1140                1145                1150

AAC CTA CTC AAT AGC AGC CAA GCA ACT TAC GCT CCA GCC ATT CAC AAT 3504
Asn Leu Leu Asn Ser Ser Gln Ala Thr Tyr Ala Pro Ala Ile His Asn
        1155                1160                1165
GAA GCG ATT CAA AGC CAA GTG GTT CAA AGC CAA ACT GCA GTC CAG CCA 3552
Glu Ala Ile Gln Ser Gln Val Val Gln Ser Gln Thr Ala Val Gln Pro
    1170                1175                1180
GTA ATT TCA ACA CAA GTT AAC CAT GTG TCA GAG CAG CCA ACT CAA GCT 3600
Val Ile Ser Thr Gln Val Asn His Val Ser Glu Gln Pro Thr Gln Ala
1185                1190                1195                1200
CCA GCT CCA AAA GCG CAG CCA GCA CCT GTG ACA ACT GCA GTT CAA ACT 3648
Pro Ala Pro Lys Ala Gln Pro Ala Pro Val Thr Thr Ala Val Gln Thr
                1205                1210                1215
GCT CCG GCA CAA GTT GTT CGT CAA GCC GCA CCA GTT CAA GCC GCT ATT 3696
Ala Pro Ala Gln Val Val Arg Gln Ala Ala Pro Val Gln Ala Ala Ile
            1220                1225                1230
GAA CCG ATT AAT ACA AGT GTT GCG ACT ACA ACG CCT TCA GCC TTC AGC 3744
Glu Pro Ile Asn Thr Ser Val Ala Thr Thr Thr Pro Ser Ala Phe Ser
        1235                1240                1245
GCC GAA ACA GCC CTG AGC GCA ACA AAA GTC CAA GCC ACT ATG CTT GAA 3792
Ala Glu Thr Ala Leu Ser Ala Thr Lys Val Gln Ala Thr Met Leu Glu
    1250                1255                1260
GTG GTT GCT GAG AAA ACC GGT TAC CCA ACT GAA ATG CTA GAG CTT GAA 3840
Val Val Ala Glu Lys Thr Gly Tyr Pro Thr Glu Met Leu Glu Leu Glu
1265                1270                1275                1280
ATG GAT ATG GAA GCC GAT TTA GGC ATC GAT TCT ATC AAG CGT GTA GAA 3888
Met Asp Met Glu Ala Asp Leu Gly Ile Asp Ser Ile Lys Arg Val Glu
                1285                1290                1295
ATT CTT GGC ACA GTA CAA GAT GAG CTA CCG GGT CTA CCT GAG CTT AGC 3936
Ile Leu Gly Thr Val Gln Asp Glu Leu Pro Gly Leu Pro Glu Leu Ser
            1300                1305                1310
CCT GAA GAT CTA GCT GAG TGT CGA ACG CTA GGC GAA ATC GTT GAC TAT 3984
Pro Glu Asp Leu Ala Glu Cys Arg Thr Leu Gly Glu Ile Val Asp Tyr
        1315                1320                1325
ATG GGC AGT AAA CTG CCG GCT GAA GGC TCT ATG AAT TCT CAG CTG TCT 4032
Met Gly Ser Lys Leu Pro Ala Glu Gly Ser Met Asn Ser Gln Leu Ser
    1330                1335                1340

ACA GGT TCC GCA GCT GCG ACT CCT GCA GCG AAT GGT CTT TCT GCG GAG 4080
Thr Gly Ser Ala Ala Ala Thr Pro Ala Ala Asn Gly Leu Ser Ala Glu
1345                1350                1355                1360
AAA GTT CAA GCG ACT ATG ATG TCT GTG GTT GCC GAA AAG ACT GGC TAC 4128
Lys Val Gln Ala Thr Met Met Ser Val Val Ala Glu Lys Thr Gly Tyr
                1365                1370                1375
CCA ACT GAA ATG CTA GAG CTT GAA ATG GAT ATG GAA GCC GAT TTA GGC 4176
Pro Thr Glu Met Leu Glu Leu Glu Met Asp Met Glu Ala Asp Leu Gly
            1380                1385                1390
ATA GAT TCT ATC AAG CGC GTT GAA ATT CTT GGC ACA GTA CAA GAT GAG 4224
Ile Asp Ser Ile Lys Arg Val Glu Ile Leu Gly Thr Val Gln Asp Glu
        1395                1400                1405
CTA CCG GGT CTA CCT GAG CTT AGC CCT GAA GAT CTA GCT GAG TGT CGT 4272
Leu Pro Gly Leu Pro Glu Leu Ser Pro Glu Asp Leu Ala Glu Cys Arg
    1410                1415                1420
ACT CTA GGC GAA ATC GTT GAC TAT ATG AAC TCT AAA CTC GCT GAC GGC 4320
Thr Leu Gly Glu Ile Val Asp Tyr Met Asn Ser Lys Leu Ala Asp Gly
1425                1430                1435                1440
TCT AAG CTG CCG GCT GAA GGC TCT ATG AAT TCT CAG CTG TCT ACA AGT 4368
Ser Lys Leu Pro Ala Glu Gly Ser Met Asn Ser Gln Leu Ser Thr Ser
                1445                1450                1455
GCC GCA GCT GCG ACT CCT GCA GCG AAT GGT CTC TCT GCG GAG AAA GTT 4416
Ala Ala Ala Ala Thr Pro Ala Ala Asn Gly Leu Ser Ala Glu Lys Val
            1460                1465                1470
CAA GCG ACT ATG ATG TCT GTG GTT GCC GAA AAG ACT GGC TAC CCA ACT 4464
Gln Ala Thr Met Met Ser Val Val Ala Glu Lys Thr Gly Tyr Pro Thr
        1475                1480                1485
GAA ATG CTA GAA CTT GAA ATG GAT ATG GAA GCT GAC CTT GGC ATC GAT 4512
Glu Met Leu Glu Leu Glu Met Asp Met Glu Ala Asp Leu Gly Ile Asp
    1490                1495                1500
TCA ATC AAG CGC GTT GAA ATT CTT GGC ACA GTA CAA GAT GAG CTA CCG 4560
Ser Ile Lys Arg Val Glu Ile Leu Gly Thr Val Gln Asp Glu Leu Pro
1505                1510                1515                1520
GGT TTA CCT GAG CTA AAT CCA GAA GAT TTG GCA GAG TGT CGT ACT CTT 4608
Gly Leu Pro Glu Leu Asn Pro Glu Asp Leu Ala Glu Cys Arg Thr Leu
                1525                1530                1535

GGC GAA ATC GTG ACT TAT ATG AAC TCT AAA CTC GCT GAC GGC TCT AAG 4656
Gly Glu Ile Val Thr Tyr Met Asn Ser Lys Leu Ala Asp Gly Ser Lys
            1540                1545                1550
CTG CCA GCT GAA GGC TCT ATG CAC TAT CAG CTG TCT ACA AGT ACC GCT 4704
Leu Pro Ala Glu Gly Ser Met His Tyr Gln Leu Ser Thr Ser Thr Ala
        1555                1560                1565
GCT GCG ACT CCT GTA GCG AAT GGT CTC TCT GCA GAA AAA GTT CAA GCG 4752
Ala Ala Thr Pro Val Ala Asn Gly Leu Ser Ala Glu Lys Val Gln Ala
    1570                1575                1580
ACC ATG ATG TCT GTA GTT GCA GAT AAA ACT GGC TAC CCA ACT GAA ATG 4800
Thr Met Met Ser Val Val Ala Asp Lys Thr Gly Tyr Pro Thr Glu Met
1585                1590                1595                1600
CTT GAA CTT GAA ATG GAT ATG GAA GCC GAT TTA GGT ATC GAT TCT ATC 4848
Leu Glu Leu Glu Met Asp Met Glu Ala Asp Leu Gly Ile Asp Ser Ile
                1605                1610                1615
AAG CGC GTT GAA ATT CTT GGC ACA GTA CAA GAT GAG CTA CCG GGT TTA 4896
Lys Arg Val Glu Ile Leu Gly Thr Val Gln Asp Glu Leu Pro Gly Leu
            1620                1625                1630
CCT GAC 4902
Pro Asp

SEQ ID NO: 5
SEQUENCE LENGTH: 3174
SEQUENCE TYPE: Nucleic acid
STRANDNESS: Double strand
TOPOLOGY: Linear
MOLECULE TYPE: Genomic DNA
ORIGINAL SOURCE: Shewanella putrefaciens SCRC-2874
(FERM BP-1625)

SEQUENCE
ATG ATG TCA GTA GTT GCA GAA AAG ACC GGC TAC CCA ACT GAA ATG CTA 48
Met Met Ser Val Val Ala Glu Lys Thr Gly Tyr Pro Thr Glu Met Leu
1               5                   10                  15
GAA CTT GGC ATG GAT ATG GAA GCC GAT TTA GGT ATC GAC TCA ATT AAA 96
Glu Leu Gly Met Asp Met Glu Ala Asp Leu Gly Ile Asp Ser Ile Lys
            20                  25                  30

CGC GTT GAG ATT CTT GGC ACA GTA CAA GAT GAG CTA CCG GGT CTA CCA 144
Arg Val Glu Ile Leu Gly Thr Val Gln Asp Glu Leu Pro Gly Leu Pro
        35                  40                  45
GAG CTT AAT CCT GAA GAT TTA GCT GAG TGC CGT ACG CTG GGC GAA ATC 192
Glu Leu Asn Pro Glu Asp Leu Ala Glu Cys Arg Thr Leu Gly Glu Ile
    50                  55                  60
GTT GAC TAT ATG AAC TCT AAG CTG GCT GAC GGC TCT AAG CTT CCA GCT 240
Val Asp Tyr Met Asn Ser Lys Leu Ala Asp Gly Ser Lys Leu Pro Ala
65                  70                  75                  80
GAA GGC TCT GCT AAT ACA AGT GCC ACT GCT GCG ACT CCT GCA GTG AAT 288
Glu Gly Ser Ala Asn Thr Ser Ala Thr Ala Ala Thr Pro Ala Val Asn
                85                  90                  95
GGT CTT TCT GCT GAC AAG GTA CAG GCG ACT ATG ATG TCT GTA GTT GCT 336
Gly Leu Ser Ala Asp Lys Val Gln Ala Thr Met Met Ser Val Val Ala
            100                 105                 110
GAA AAG ACC GGC TAC CCA ACT GAA ATG CTA GAA CTT GGC ATG GAT ATG 384
Glu Lys Thr Gly Tyr Pro Thr Glu Met Leu Glu Leu Gly Met Asp Met
        115                 120                 125
GAA GCA GAC CTT GGT ATT GAT TCT ATT AAG CGC GTT GAA ATT CTT GGC 432
Glu Ala Asp Leu Gly Ile Asp Ser Ile Lys Arg Val Glu Ile Leu Gly
    130                 135                 140
ACA GTA CAA GAT GAG CTC CCA GGT TTA CCT GAG CTT AAT CCT GAA GAT 480
Thr Val Gln Asp Glu Leu Pro Gly Leu Pro Glu Leu Asn Pro Glu Asp
145                 150                 155                 160
CTC GCT GAG TGC CGC ACG CTT GGC GAA ATC GTT AGC TAT ATG AAC TCT 528
Leu Ala Glu Cys Arg Thr Leu Gly Glu Ile Val Ser Tyr Met Asn Ser
                165                 170                 175
CAA CTG GCT GAT GGC TCT AAA CTT TCT ACA AGT GCG GCT GAA GGC TCT 576
Gln Leu Ala Asp Gly Ser Lys Leu Ser Thr Ser Ala Ala Glu Gly Ser
            180                 185                 190
GCT GAT ACA AGT GCT GCA AAT GCT GCA AAG CCG GCA GCA ATT TCG GCA 624
Ala Asp Thr Ser Ala Ala Asn Ala Ala Lys Pro Ala Ala Ile Ser Ala
        195                 200                 205
GAA CCA AGT GTT GAG CTT CCT CCT CAT AGC GAG GTA GCG CTA AAA AAG 672
Glu Pro Ser Val Glu Leu Pro Pro His Ser Glu Val Ala Leu Lys Lys
    210                 215                 220

CTT AAT GCG GCG AAC AAG CTA GAA AAT TGT TTC GCC GCA GAC GCA AGT 720
Leu Asn Ala Ala Asn Lys Leu Glu Asn Cys Phe Ala Ala Asp Ala Ser
225                 230                 235                 240
GTT GTG ATT AAC GAT GAT GGT CAC AAC GCA GGC GTT TTA GCT GAG AAA 768
Val Val Ile Asn Asp Asp Gly His Asn Ala Gly Val Leu Ala Glu Lys
                245                 250                 255
CTT ATT AAA CAA GGC CTA AAA GTA GCC GTT GTG CGT TTA CCG AAA GGT 816
Leu Ile Lys Gln Gly Leu Lys Val Ala Val Val Arg Leu Pro Lys Gly
            260                 265                 270
CAG CCT CAA TCG CCA CTT TCA AGC GAT GTT GCT AGC TTT GAG CTT GCC 864
Gln Pro Gln Ser Pro Leu Ser Ser Asp Val Ala Ser Phe Glu Leu Ala
        275                 280                 285
TCA AGC CAA GAA TCT GAG CTT GAA GCC AGT ATC ACT GCA GTT ATC GCG 912
Ser Ser Gln Glu Ser Glu Leu Glu Ala Ser Ile Thr Ala Val Ile Ala
    290                 295                 300
CAG ATT GAA ACT CAG GTT GGC GCT ATT GGT GGC TTT ATT CAC TTG CAA 960
Gln Ile Glu Thr Gln Val Gly Ala Ile Gly Gly Phe Ile His Leu Gln
305                 310                 315                 320
CCA GAA GCG AAT ACA GAA GAG CAA ACG GCA GTA AAC CTA GAT GCG CAA 1008
Pro Glu Ala Asn Thr Glu Glu Gln Thr Ala Val Asn Leu Asp Ala Gln
                325                 330                 335
AGT TTT ACT CAC GTT AGC AAT GCG TTC TTG TGG GCC AAA TTA TTG CAA 1056
Ser Phe Thr His Val Ser Asn Ala Phe Leu Trp Ala Lys Leu Leu Gln
            340                 345                 350
CCA AAG CTC GTT GCT GGA GCA GAT GCG CGT CGC TGT TTT GTA ACA GTA 1104
Pro Lys Leu Val Ala Gly Ala Asp Ala Arg Arg Cys Phe Val Thr Val
        355                 360                 365
AGC CGT ATC GAC GGT GGC TTT GGT TAC CTA AAT ACT GAC GCC CTA AAA 1152
Ser Arg Ile Asp Gly Gly Phe Gly Tyr Leu Asn Thr Asp Ala Leu Lys
    370                 375                 380
GAT GCT GAG CTA AAC CAA GCA GCA TTA GCT GGT TTA ACT AAA ACC TTA 1200
Asp Ala Glu Leu Asn Gln Ala Ala Leu Ala Gly Leu Thr Lys Thr Leu
385                 390                 395                 400
AGC CAT GAA TGG CCA CAA GTG TTC TGT CGC GCG CTA GAT ATT GCA ACA 1248
Ser His Glu Trp Pro Gln Val Phe Cys Arg Ala Leu Asp Ile Ala Thr
                405                 410                 415

GAT GTT GAT GCA ACC CAT CTT GCT GAT GCA ATC ACC AGT GAA CTA TTT 1296
Asp Val Asp Ala Thr His Leu Ala Asp Ala Ile Thr Ser Glu Leu Phe
            420                 425                 430
GAT AGC CAA GCT CAG CTA CCT GAA GTG GGC TTA AGC TTA ATT GAT GGC 1344
Asp Ser Gln Ala Gln Leu Pro Glu Val Gly Leu Ser Leu Ile Asp Gly
        435                 440                 445
AAA GTT AAC CGC GTA ACT CTA GTT GCT GCT GAA GCT GCA GAT AAA ACA 1392
Lys Val Asn Arg Val Thr Leu Val Ala Ala Glu Ala Ala Asp Lys Thr
    450                 455                 460
GCA AAA GCA GAG CTT AAC AGC ACA GAT AAA ATC TTA GTG ACT GGT GGG 1440
Ala Lys Ala Glu Leu Asn Ser Thr Asp Lys Ile Leu Val Thr Gly Gly
465                 470                 475                 480
GCA AAA GGG GTG ACA TTT GAA TGT GCA CTG GCA TTA GCA TCT CGC AGC 1488
Ala Lys Gly Val Thr Phe Glu Cys Ala Leu Ala Leu Ala Ser Arg Ser
                485                 490                 495
CAG TCT CAC TTT ATC TTA GCT GGG CGC AGT GAA TTA CAA GCT TTA CCA 1536
Gln Ser His Phe Ile Leu Ala Gly Arg Ser Glu Leu Gln Ala Leu Pro
            500                 505                 510
AGC TGG GCT GAG GGT AAG CAA ACT AGC GAG CTA AAA TCA GCT GCA ATC 1584
Ser Trp Ala Glu Gly Lys Gln Thr Ser Glu Leu Lys Ser Ala Ala Ile
        515                 520                 525
GCA CAT ATT ATT TCT ACT GGT CAA AAG CCA ACG CCT AAG CAA GTT GAA 1632
Ala His Ile Ile Ser Thr Gly Gln Lys Pro Thr Pro Lys Gln Val Glu
    530                 535                 540
GCC GCT GTG TGG CCA GTG CAA AGC AGC ATT GAA ATT AAT GCC GCC CTA 1680
Ala Ala Val Trp Pro Val Gln Ser Ser Ile Glu Ile Asn Ala Ala Leu
545                 550                 555                 560
GCC GCC TTT AAC AAA GTT GGC GCC TCA GCT GAA TAC GTC AGC ATG GAT 1728
Ala Ala Phe Asn Lys Val Gly Ala Ser Ala Glu Tyr Val Ser Met Asp
                565                 570                 575
GTT ACC GAT AGC GCC GCA ATC ACA GCA GCA CTT AAT GGT CGC TCA AAT 1776
Val Thr Asp Ser Ala Ala Ile Thr Ala Ala Leu Asn Gly Arg Ser Asn
            580                 585                 590
GAG ATC ACC GGT CTT ATT CAT GGC GCA GGT GTA CTA GCC GAC AAG CAT 1824
Glu Ile Thr Gly Leu Ile His Gly Ala Gly Val Leu Ala Asp Lys His
        595                 600                 605

ATT CAA GAC AAG ACT CTT GCT GAA CTT GCT AAA GTT TAT GGC ACT AAA 1872
Ile Gln Asp Lys Thr Leu Ala Glu Leu Ala Lys Val Tyr Gly Thr Lys
    610                 615                 620
GTC AAC GGC CTA AAA GCG CTG CTC GCG GCA CTT GAG CCA AGC AAA ATT 1920
Val Asn Gly Leu Lys Ala Leu Leu Ala Ala Leu Glu Pro Ser Lys Ile
625                 630                 635                 640
AAA TTA CTT GCT ATG TTC TCA TCT GCA GCA GGT TTT TAC GGT AAT ATC 1968
Lys Leu Leu Ala Met Phe Ser Ser Ala Ala Gly Phe Tyr Gly Asn Ile
                645                 650                 655
GGC CAA AGC GAT TAC GCG ATG TCG AAC GAT ATT CTT AAC AAG GCA GCG 2016
Gly Gln Ser Asp Tyr Ala Met Ser Asn Asp Ile Leu Asn Lys Ala Ala
            660                 665                 670
CTG CAG TTC ACC GCT CGC AAC CCA CAA GCT AAA GTC ATG AGC TTT AAC 2064
Leu Gln Phe Thr Ala Arg Asn Pro Gln Ala Lys Val Met Ser Phe Asn
        675                 680                 685
TGG GGT CCT TGG GAT GGC GGC ATG GTT AAC CCA GCG CTT AAA AAG ATG 2112
Trp Gly Pro Trp Asp Gly Gly Met Val Asn Pro Ala Leu Lys Lys Met
    690                 695                 700
TTT ACC GAG CGT GGT GTG TAC GTT ATT CCA CTA AAA GCA GGT GCA GAG 2160
Phe Thr Glu Arg Gly Val Tyr Val Ile Pro Leu Lys Ala Gly Ala Glu
705                 710                 715                 720
CTA TTT GCC ACT CAG CTA TTG GCT GAA ACT GGC GTG CAG TTG CTC ATT 2208
Leu Phe Ala Thr Gln Leu Leu Ala Glu Thr Gly Val Gln Leu Leu Ile
                725                 730                 735
GGT ACG TCA ATG CAA GGT GGC AGC GAC ACT AAA GCA ACT GAG ACT GCT 2256
Gly Thr Ser Met Gln Gly Gly Ser Asp Thr Lys Ala Thr Glu Thr Ala
            740                 745                 750
TCT GTA AAA AAG CTT AAT GCG GGT GAG GTG CTA AGT GCA TCG CAT CCG 2304
Ser Val Lys Lys Leu Asn Ala Gly Glu Val Leu Ser Ala Ser His Pro
        755                 760                 765
CGT GCT GGT GCA CAA AAA ACA CCA CTA CAA GCT GTC ACT GCA ACG CGT 2352
Arg Ala Gly Ala Gln Lys Thr Pro Leu Gln Ala Val Thr Ala Thr Arg
    770                 775                 780
CTG TTA ACC CCA AGT GCC ATG GTC TTC ATT GAA GAT CAC CGC ATT GGC 2400
Leu Leu Thr Pro Ser Ala Met Val Phe Ile Glu Asp His Arg Ile Gly
785                 790                 795                 800

GGT AAC AGT GTG TTG CCA ACG GTA TGC GCC ATC GAC TGG ATG CGT GAA 2448
Gly Asn Ser Val Leu Pro Thr Val Cys Ala Ile Asp Trp Met Arg Glu
                805                 810                 815
GCG GCA AGC GAC ATG CTT GGC GCT CAA GTT AAG GTA CTT GAT TAC AAG 2496
Ala Ala Ser Asp Met Leu Gly Ala Gln Val Lys Val Leu Asp Tyr Lys
            820                 825                 830
CTA TTA AAA GGC ATT GTA TTT GAG ACT GAT GAG CCG CAA GAG TTA ACA 2544
Leu Leu Lys Gly Ile Val Phe Glu Thr Asp Glu Pro Gln Glu Leu Thr
        835                 840                 845
CTT GAG CTA ACG CCA GAC GAT TCA GAC GAA GCT ACG CTA CAA GCA TTA 2592
Leu Glu Leu Thr Pro Asp Asp Ser Asp Glu Ala Thr Leu Gln Ala Leu
    850                 855                 860
ATC AGC TGT AAT GGG CGT CCG CAA TAC AAG GCG ACG CTT ATC AGT GAT 2640
Ile Ser Cys Asn Gly Arg Pro Gln Tyr Lys Ala Thr Leu Ile Ser Asp
865                 870                 875                 880
AAT GCC GAT ATT AAG CAA CTT AAC AAG CAG TTT GAT TTA AGC GCT AAG 2688
Asn Ala Asp Ile Lys Gln Leu Asn Lys Gln Phe Asp Leu Ser Ala Lys
                885                 890                 895
GCG ATT ACC ACA GCA AAA GAG CTT TAT AGC AAC GGC ACC TTG TTC CAC 2736
Ala Ile Thr Thr Ala Lys Glu Leu Tyr Ser Asn Gly Thr Leu Phe His
            900                 905                 910
GGT CCG CGT CTA CAA GGG ATC CAA TCT GTA GTG CAG TTC GAT GAT CAA 2784
Gly Pro Arg Leu Gln Gly Ile Gln Ser Val Val Gln Phe Asp Asp Gln
        915                 920                 925
GGC TTA ATT GCT AAA GTC GCT CTG CCT AAG GTT GAA CTT AGC GAT TGT 2832
Gly Leu Ile Ala Lys Val Ala Leu Pro Lys Val Glu Leu Ser Asp Cys
    930                 935                 940
GGT GAG TTC TTG CCG CAA ACC CAC ATG GGT GGC AGT CAA CCT TTT GCT 2880
Gly Glu Phe Leu Pro Gln Thr His Met Gly Gly Ser Gln Pro Phe Ala
945                 950                 955                 960
GAG GAC TTG CTA TTA CAA GCT ATG CTG GTT TGG GCT CGC CTT AAA ACT 2928
Glu Asp Leu Leu Leu Gln Ala Met Leu Val Trp Ala Arg Leu Lys Thr
                965                 970                 975
GGC TCG GCA AGT TTG CCA TCA AGC ATT GGT GAG TTT ACC TCA TAC CAA 2976
Gly Ser Ala Ser Leu Pro Ser Ser Ile Gly Glu Phe Thr Ser Tyr Gln
            980                 985                 990

CCA ATG GCC TTT GGT GAA ACT GGT ACC ATA GAG CTT GAA GTG ATT AAG 3024
Pro Met Ala Phe Gly Glu Thr Gly Thr Ile Glu Leu Glu Val Ile Lys
        995                 1000                1005
CAC AAC AAA CGC TCA CTT GAA GCG AAT GTT GCG CTA TAT CGT GAC AAC 3072
His Asn Lys Arg Ser Leu Glu Ala Asn Val Ala Leu Tyr Arg Asp Asn
    1010                1015                1020
GGC GAG TTA AGT GCC ATG TTT AAG TCA GCT AAA ATC ACC ATT AGC AAA 3120
Gly Glu Leu Ser Ala Met Phe Lys Ser Ala Lys Ile Thr Ile Ser Lys
1025                1030                1035                1040
AGC TTA AAT TCA GCA TTT TTA CCT GCT GTC TTA GCA AAC GAC AGT GAG 3168
Ser Leu Asn Ser Ala Phe Leu Pro Ala Val Leu Ala Asn Asp Ser Glu
                1045                1050                1055
GCG AAT 3174
Ala Asn

SEQ ID NO: 6
SEQUENCE LENGTH: 2340
SEQUENCE TYPE: Nucleic acid
STRANDNESS: Double strand
TOPOLOGY: Linear
MOLECULE TYPE: Genomic DNA
ORIGINAL SOURCE: Shewanella putrefaciens SCRC-2874
(FERM BP-1625)

SEQUENCE
GTG GAA CAA ACG CCT AAA GCT AGT GCG ATG CCG CTG CGC ATC GCA CTT 48
Val Glu Gln Thr Pro Lys Ala Ser Ala Met Pro Leu Arg Ile Ala Leu
1               5                   10                  15
ATC TTA CTG CCA ACA CCG CAG TTT GAA GTT AAC TCT GTC GAC CAG TCA 96
Ile Leu Leu Pro Thr Pro Gln Phe Glu Val Asn Ser Val Asp Gln Ser
            20                  25                  30
GTA TTA GCC AGC TAT CAA ACA CTG CAG CCT GAG CTA AAT GCC CTG CTT 144
Val Leu Ala Ser Tyr Gln Thr Leu Gln Pro Glu Leu Asn Ala Leu Leu
        35                  40                  45
AAT AGT GCG CCG ACA CCT GAA ATG CTC AGC ATC ACT ATC TCA GAT GAT 192
Asn Ser Ala Pro Thr Pro Glu Met Leu Ser Ile Thr Ile Ser Asp Asp
    50                  55                  60

AGC GAT GCA AAC AGC TTT GAG TCG CAG CTA AAT GCT GCG ACC AAC GCA 240
Ser Asp Ala Asn Ser Phe Glu Ser Gln Leu Asn Ala Ala Thr Asn Ala
65                  70                  75                  80
ATT AAC AAT GGC TAT ATC GTC AAG CTT GCT ACG GCA ACT CAC GCT TTG 288
Ile Asn Asn Gly Tyr Ile Val Lys Leu Ala Thr Ala Thr His Ala Leu
                85                  90                  95
TTA ATG CTG CCT GCA TTA AAA GCG GCG CAA ATG CGG ATC CAT CCT CAT 336
Leu Met Leu Pro Ala Leu Lys Ala Ala Gln Met Arg Ile His Pro His
            100                 105                 110
GCG CAG CTT GCC GCT ATG CAG CAA GCT AAA TCG ACG CCA ATG AGT CAA 384
Ala Gln Leu Ala Ala Met Gln Gln Ala Lys Ser Thr Pro Met Ser Gln
        115                 120                 125
GTA TCT GGT GAG CTA AAG CTT GGC GCT AAT GCG CTA AGC CTA GCT CAG 432
Val Ser Gly Glu Leu Lys Leu Gly Ala Asn Ala Leu Ser Leu Ala Gln
    130                 135                 140
ACT AAT GCG CTG TCT CAT GCT TTA AGC CAA GCC AAG CGT AAC TTA ACT 480
Thr Asn Ala Leu Ser His Ala Leu Ser Gln Ala Lys Arg Asn Leu Thr
145                 150                 155                 160
GAT GTC AGC GTG AAT GAG TGT TTT GAG AAC CTC AAA AGT GAA CAG CAG 528
Asp Val Ser Val Asn Glu Cys Phe Glu Asn Leu Lys Ser Glu Gln Gln
                165                 170                 175
TTC ACA GAG GTT TAT TCG CTT ATT CAG CAA CTT GCT AGC CGC ACC CAT 576
Phe Thr Glu Val Tyr Ser Leu Ile Gln Gln Leu Ala Ser Arg Thr His
            180                 185                 190
GTG AGA AAA GAG GTT AAT CAA GGT GTG GAA CTT GGC CCT AAA CAA GCC 624
Val Arg Lys Glu Val Asn Gln Gly Val Glu Leu Gly Pro Lys Gln Ala
        195                 200                 205
AAA AGC CAC TAT TGG TTT AGC GAA TTT CAC CAA AAC CGT GTT GCT GCC 672
Lys Ser His Tyr Trp Phe Ser Glu Phe His Gln Asn Arg Val Ala Ala
    210                 215                 220
ATC AAC TTT ATT AAT GGC CAA CAA GCA ACC AGC TAT GTG CTT ACT CAA 720
Ile Asn Phe Ile Asn Gly Gln Gln Ala Thr Ser Tyr Val Leu Thr Gln
225                 230                 235                 240
GGT TCA GGA TTG TTA GCT GCG AAA TCA ATG CTA AAC CAG CAA AGA TTA 768
Gly Ser Gly Leu Leu Ala Ala Lys Ser Met Leu Asn Gln Gln Arg Leu
                245                 250                 255

ATG TTT ATC TTG CCG GGT AAC AGT CAG CAA CAA ATA ACC GCA TCA ATA 816
Met Phe Ile Leu Pro Gly Asn Ser Gln Gln Gln Ile Thr Ala Ser Ile
            260                 265                 270
ACT CAG TTA ATG CAG CAA TTA GAG CGT TTG CAG GTA ACT GAG GTT AAT 864
Thr Gln Leu Met Gln Gln Leu Glu Arg Leu Gln Val Thr Glu Val Asn
        275                 280                 285
GAG CTT TCT CTA GAA TGC CAA CTA GAG CTG CTC AGC ATA ATG TAT GAC 912
Glu Leu Ser Leu Glu Cys Gln Leu Glu Leu Leu Ser Ile Met Tyr Asp
    290                 295                 300
AAC TTA GTC AAC GCA GAC AAA CTC ACT ACT CGC GAT AGT AAG CCC GCT 960
Asn Leu Val Asn Ala Asp Lys Leu Thr Thr Arg Asp Ser Lys Pro Ala
305                 310                 315                 320
TAT CAG GCT GTG ATT CAA GCA AGC TCT GTT AGC GCT GCA AAG CAA GAG 1008
Tyr Gln Ala Val Ile Gln Ala Ser Ser Val Ser Ala Ala Lys Gln Glu
                325                 330                 335
TTA AGC GCG CTT AAC GAT GCA CTC ACA GCG CTG TTT GCT GAG CAA ACA 1056
Leu Ser Ala Leu Asn Asp Ala Leu Thr Ala Leu Phe Ala Glu Gln Thr
            340                 345                 350
AAC GCC ACA TCA ACG AAT AAA GGC TTA ATC CAA TAC AAA ACA CCG GCG 1104
Asn Ala Thr Ser Thr Asn Lys Gly Leu Ile Gln Tyr Lys Thr Pro Ala
        355                 360                 365
GGC AGT TAC TTA ACC CTA ACA CCG CTT GGC AGC AAC AAT GAC AAC GCC 1152
Gly Ser Tyr Leu Thr Leu Thr Pro Leu Gly Ser Asn Asn Asp Asn Ala
    370                 375                 380
CAA GCG GGT CTT GCT TTT GTC TAT CCG GGT GTG GGA ACG GTT TAC GCC 1200
Gln Ala Gly Leu Ala Phe Val Tyr Pro Gly Val Gly Thr Val Tyr Ala
385                 390                 395                 400
GAT ATG CTT AAT GAG CTG CAT CAG TAC TTC CCT GCG CTT TAC GCC AAA 1248
Asp Met Leu Asn Glu Leu His Gln Tyr Phe Pro Ala Leu Tyr Ala Lys
                405                 410                 415
CTT GAG CGT GAA GGC GAT TTA AAG GCG ATG CTA CAA GCA GAA GAT ATC 1296
Leu Glu Arg Glu Gly Asp Leu Lys Ala Met Leu Gln Ala Glu Asp Ile
            420                 425                 430
TAT CAT CTT GAC CCT AAA CAT GCT GCC CAA ATG AGC TTA GGT GAC TTA 1344
Tyr His Leu Asp Pro Lys His Ala Ala Gln Met Ser Leu Gly Asp Leu
        435                 440                 445

GCC ATT GCT GGC GTG GGG AGC AGC TAC CTG TTA ACT CAG CTG CTC ACC 1392
Ala Ile Ala Gly Val Gly Ser Ser Tyr Leu Leu Thr Gln Leu Leu Thr
    450                 455                 460
GAT GAG TTT AAT ATT AAG CCT AAT TTT GCA TTA GGT TAC TCA ATG GGT 1440
Asp Glu Phe Asn Ile Lys Pro Asn Phe Ala Leu Gly Tyr Ser Met Gly
465                 470                 475                 480
GAA GCA TCA ATG TGG GCA AGC TTA GGC GTA TGG CAA AAC CCG CAT GCG 1488
Glu Ala Ser Met Trp Ala Ser Leu Gly Val Trp Gln Asn Pro His Ala
                485                 490                 495
CTG ATC AGC AAA ACC CAA ACC GAC CCG CTA TTT ACT TCT GCT ATT TCC 1536
Leu Ile Ser Lys Thr Gln Thr Asp Pro Leu Phe Thr Ser Ala Ile Ser
            500                 505                 510
GGC AAA TTG ACC GCG GTT AGA CAA GCT TGG CAG CTT GAT GAT ACC GCA 1584
Gly Lys Leu Thr Ala Val Arg Gln Ala Trp Gln Leu Asp Asp Thr Ala
        515                 520                 525
GCG GAA ATC CAG TGG AAT AGC TTT GTG GTT AGA AGT GAA GCA GCG CCG 1632
Ala Glu Ile Gln Trp Asn Ser Phe Val Val Arg Ser Glu Ala Ala Pro
    530                 535                 540
ATT GAA GCC TTG CTA AAA GAT TAC CCA CAC GCT TAC CTC GCG ATT ATT 1680
Ile Glu Ala Leu Leu Lys Asp Tyr Pro His Ala Tyr Leu Ala Ile Ile
545                 550                 555                 560
CAA GGG GAT ACC TGC GTA ATC GCT GGC TGT GAA ATC CAA TGT AAA GCG 1728
Gln Gly Asp Thr Cys Val Ile Ala Gly Cys Glu Ile Gln Cys Lys Ala
                565                 570                 575
CTA CTT GCA GCA CTG GGT AAA CGC GGT ATT GCA GCT AAT CGT GTA ACG 1776
Leu Leu Ala Ala Leu Gly Lys Arg Gly Ile Ala Ala Asn Arg Val Thr
            580                 585                 590
GCG ATG CAT ACG CAG CCT GCG ATG CAA GAG CAT CAA AAT GTG ATG GAT 1824
Ala Met His Thr Gln Pro Ala Met Gln Glu His Gln Asn Val Met Asp
        595                 600                 605
TTT TAT CTG CAA CCG TTA AAA GCA GAG CTT CCT AGT GAA ATA AGC TTT 1872
Phe Tyr Leu Gln Pro Leu Lys Ala Glu Leu Pro Ser Glu Ile Ser Phe
    610                 615                 620
ATC AGC GCC GCT GAT TTA ACT GCC AAG CAA ACG GTG AGT GAG CAA GCA 1920
Ile Ser Ala Ala Asp Leu Thr Ala Lys Gln Thr Val Ser Glu Gln Ala
625                 630                 635                 640

CTT AGC AGC CAA GTC GTT GCT CAG TCT ATT GCC GAC ACC TTC TGC CAA 1968
Leu Ser Ser Gln Val Val Ala Gln Ser Ile Ala Asp Thr Phe Cys Gln
                645                 650                 655
ACC TTG GAC TTT ACC GCG CTA GTA CAT CAC GCC CAA CAT CAA GGC GCT 2016
Thr Leu Asp Phe Thr Ala Leu Val His His Ala Gln His Gln Gly Ala
            660                 665                 670
AAG CTG TTT GTT GAA ATT GGC GCG GAT AGA CAA AAC TGC ACC TTG ATA 2064
Lys Leu Phe Val Glu Ile Gly Ala Asp Arg Gln Asn Cys Thr Leu Ile
        675                 680                 685
GAC AAG ATT GTT AAA CAA GAT GGT GCC AGC AGT GTA CAA CAT CAA CCT 2112
Asp Lys Ile Val Lys Gln Asp Gly Ala Ser Ser Val Gln His Gln Pro
    690                 695                 700
TGT TGC ACA GTG CCT ATG AAC GCA AAA GGT AGC CAA GAT ATT ACC AGC 2160
Cys Cys Thr Val Pro Met Asn Ala Lys Gly Ser Gln Asp Ile Thr Ser
705                 710                 715                 720
GTG ATT AAA GCG CTT GGC CAA TTA ATT AGC CAT CAG GTG CCA TTA TCG 2208
Val Ile Lys Ala Leu Gly Gln Leu Ile Ser His Gln Val Pro Leu Ser
                725                 730                 735
GTG CAA CCA TTT ATT GAT GGA CTC AAG CGC GAG CTA ACA CTT TGC CAA 2256
Val Gln Pro Phe Ile Asp Gly Leu Lys Arg Glu Leu Thr Leu Cys Gln
            740                 745                 750
TTG ACC AGC CAA CAG CTG GCA GCA CAT GCA AAT GTT GAC AGC AAG TTT 2304
Leu Thr Ser Gln Gln Leu Ala Ala His Ala Asn Val Asp Ser Lys Phe
        755                 760                 765
GAG TCT AAC CAA GAC CAT TTA CTT CAA GGG GAA GTC 2340
Glu Ser Asn Gln Asp His Leu Leu Gln Gly Glu Val
    770                 775                 780

SEQ ID NO: 7
SEQUENCE LENGTH: 5637
SEQUENCE TYPE: Nucleic acid
STRANDNESS: Double strand
TOPOLOGY: Linear
MOLECULE TYPE: Genomic DNA
ORIGINAL SOURCE: Shewanella putrefaciens SCRC-2874
(FERM BP-1625)

SEQUENCE
ATG TCA TTA CCA GAC AAT GCT TCT AAC CAC CTT TCT GCC AAC CAG AAA 48
Met Ser Leu Pro Asp Asn Ala Ser Asn His Leu Ser Ala Asn Gln Lys
1               5                   10                  15
GGC GCA TCT CAG GCA AGT AAA ACC AGT AAG CAA AGC AAA ATC GCC ATT 96
Gly Ala Ser Gln Ala Ser Lys Thr Ser Lys Gln Ser Lys Ile Ala Ile
            20                  25                  30
GTC GGT TTA GCC ACT CTG TAT CCA GAC GCT AAA ACC CCG CAA GAA TTT 144
Val Gly Leu Ala Thr Leu Tyr Pro Asp Ala Lys Thr Pro Gln Glu Phe
        35                  40                  45
TGG CAG AAT TTG CTG GAT AAA CGC GAC TCT CGC AGC ACC TTA ACT AAC 192
Trp Gln Asn Leu Leu Asp Lys Arg Asp Ser Arg Ser Thr Leu Thr Asn
    50                  55                  60
GAA AAA CTC GGC GCT AAC AGC CAA GAT TAT CAA GGT GTG CAA GGC CAA 240
Glu Lys Leu Gly Ala Asn Ser Gln Asp Tyr Gln Gly Val Gln Gly Gln
65                  70                  75                  80
TCT GAC CGT TTT TAT TGT AAT AAA GGC GGC TAC ATT GAG AAC TTC AGC 288
Ser Asp Arg Phe Tyr Cys Asn Lys Gly Gly Tyr Ile Glu Asn Phe Ser
                85                  90                  95
TTT AAT GCT GCA GGC TAC AAA TTG CCG GAG CAA AGC TTA AAT GGC TTG 336
Phe Asn Ala Ala Gly Tyr Lys Leu Pro Glu Gln Ser Leu Asn Gly Leu
            100                 105                 110
GAC GAC AGC TTC CTT TGG GCG CTC GAT ACT AGC CGT AAC GCA CTA ATT 384
Asp Asp Ser Phe Leu Trp Ala Leu Asp Thr Ser Arg Asn Ala Leu Ile
        115                 120                 125
GAT GCT GGT ATT GAT ATC AAC GGC GCT GAT TTA AGC CGC GCA GGT GTA 432
Asp Ala Gly Ile Asp Ile Asn Gly Ala Asp Leu Ser Arg Ala Gly Val
    130                 135                 140
GTC ATG GGC GCG CTG TCG TTC CCA ACT ACC CGC TCA AAC GAT CTG TTT 480
Val Met Gly Ala Leu Ser Phe Pro Thr Thr Arg Ser Asn Asp Leu Phe
145                 150                 155                 160
TTG CCA ATT TAT CAC AGC GCC GTT GAA AAA GCC CTG CAA GAT AAA CTA 528
Leu Pro Ile Tyr His Ser Ala Val Glu Lys Ala Leu Gln Asp Lys Leu
                165                 170                 175
GGC GTA AAG GCA TTT AAG CTA AGC CCA ACT AAT GCT CAT ACC GCT CGC 576
Gly Val Lys Ala Phe Lys Leu Ser Pro Thr Asn Ala His Thr Ala Arg
            180                 185                 190

GCG GCA AAT GAG AGC AGC CTA AAT GCA GCC AAT GGT GCC ATT GCC CAT 624
Ala Ala Asn Glu Ser Ser Leu Asn Ala Ala Asn Gly Ala Ile Ala His
        195                 200                 205
AAC AGC TCA AAA GTG GTG GCC GAT GCA CTT GGC CTT GGC GGC GCA CAA 672
Asn Ser Ser Lys Val Val Ala Asp Ala Leu Gly Leu Gly Gly Ala Gln
    210                 215                 220
CTA AGC CTA GAT GCT GCC TGT GCT AGT TCG GTT TAC TCA TTA AAG CTT 720
Leu Ser Leu Asp Ala Ala Cys Ala Ser Ser Val Tyr Ser Leu Lys Leu
225                 230                 235                 240
GCC TGC GAT TAC CTA AGC ACT GGC AAA GCC GAT ATC ATG CTA GCA GGC 768
Ala Cys Asp Tyr Leu Ser Thr Gly Lys Ala Asp Ile Met Leu Ala Gly
                245                 250                 255
GCA GTA TCT GGC GCG GAT CCT TTC TTT ATT AAT ATG GGA TTC TCA ATC 816
Ala Val Ser Gly Ala Asp Pro Phe Phe Ile Asn Met Gly Phe Ser Ile
            260                 265                 270
TTC CAC GCC TAC CCA GAC CAT GGT ATC TCA GTA CCG TTT GAT GCC AGC 864
Phe His Ala Tyr Pro Asp His Gly Ile Ser Val Pro Phe Asp Ala Ser
        275                 280                 285
AGT AAA GGT TTG TTT GCT GGC GAA GGC GCT GGC GTA TTA GTG CTT AAA 912
Ser Lys Gly Leu Phe Ala Gly Glu Gly Ala Gly Val Leu Val Leu Lys
    290                 295                 300
CGT CTT GAA GAT GCC GAG CGC GAC AAT GAC AAA ATC TAT GCG GTT GTT 960
Arg Leu Glu Asp Ala Glu Arg Asp Asn Asp Lys Ile Tyr Ala Val Val
305                 310                 315                 320
AGC GGC GTA GGT CTA TCA AAC GAC GGT AAA GGC CAG TTT GTA TTA AGC 1008
Ser Gly Val Gly Leu Ser Asn Asp Gly Lys Gly Gln Phe Val Leu Ser
                325                 330                 335
CCT AAT CCA AAA GGT CAG GTG AAG GCC TTT GAA CGT GCT TAT GCT GCC 1056
Pro Asn Pro Lys Gly Gln Val Lys Ala Phe Glu Arg Ala Tyr Ala Ala
            340                 345                 350
AGT GAC ATT GAG CCA AAA GAC ATT GAA GTG ATT GAG TGC CAC GCA ACA 1104
Ser Asp Ile Glu Pro Lys Asp Ile Glu Val Ile Glu Cys His Ala Thr
        355                 360                 365
GGC ACA CCG CTT GGC GAT AAA ATT GAG CTC ACT TCA ATG GAA ACC TTC 1152
Gly Thr Pro Leu Gly Asp Lys Ile Glu Leu Thr Ser Met Glu Thr Phe
    370                 375                 380

TTT GAA GAC AAG CTG CAA GGC ACC GAT GCA CCG TTA ATT GGC TCA GCT 1200
Phe Glu Asp Lys Leu Gln Gly Thr Asp Ala Pro Leu Ile Gly Ser Ala
385                 390                 395                 400
AAG TCT AAC TTA GGC CAC CTA TTA ACT GCA GCG CAT GCG GGG ATC ATG 1248
Lys Ser Asn Leu Gly His Leu Leu Thr Ala Ala His Ala Gly Ile Met
                405                 410                 415
AAG ATG ATC TTC GCC ATG AAA GAA GGT TAC CTG CCG CCA AGT ATC AAT 1296
Lys Met Ile Phe Ala Met Lys Glu Gly Tyr Leu Pro Pro Ser Ile Asn
            420                 425                 430
ATT AGT GAT GCT ATC GCT TCG CCG AAA AAA CTC TTC GGT AAA CCA ACC 1344
Ile Ser Asp Ala Ile Ala Ser Pro Lys Lys Leu Phe Gly Lys Pro Thr
        435                 440                 445
CTG CCT AGC ATG GTT CAA GGC TGG CCA GAT AAG CCA TCG AAT AAT CAT 1392
Leu Pro Ser Met Val Gln Gly Trp Pro Asp Lys Pro Ser Asn Asn His
    450                 455                 460
TTT GGT GTA AGA ACC CGT CAC GCA GGC GTA TCG GTA TTT GGC TTT GGT 1440
Phe Gly Val Arg Thr Arg His Ala Gly Val Ser Val Phe Gly Phe Gly
465                 470                 475                 480
GGC TGT AAC GCC CAT CTG TTG CTT GAG TCA TAC AAC GGC AAA GGA ACA 1488
Gly Cys Asn Ala His Leu Leu Leu Glu Ser Tyr Asn Gly Lys Gly Thr
                485                 490                 495
GTA AAG GCA GAA GCC ACT CAA GTA CCG CGT CAA GCT GAG CCG CTA AAA 1536
Val Lys Ala Glu Ala Thr Gln Val Pro Arg Gln Ala Glu Pro Leu Lys
            500                 505                 510
GTG GTT GGC CTT GCC TCG CAC TTT GGG CCT CTT AGC AGC ATT AAT GCA 1584
Val Val Gly Leu Ala Ser His Phe Gly Pro Leu Ser Ser Ile Asn Ala
        515                 520                 525
CTC AAC AAT GCT GTG ACC CAA GAT GGG AAT GGC TTT ATC GAA CTG CCG 1632
Leu Asn Asn Ala Val Thr Gln Asp Gly Asn Gly Phe Ile Glu Leu Pro
    530                 535                 540
AAA AAG CGC TGG AAA GGC CTT GAA AAG CAC AGT GAA CTG TTA GCT GAA 1680
Lys Lys Arg Trp Lys Gly Leu Glu Lys His Ser Glu Leu Leu Ala Glu
545                 550                 555                 560
TTT GGC TTA GCA TCT GCG CCA AAA GGT GCT TAT GTT GAT AAC TTC GAG 1728
Phe Gly Leu Ala Ser Ala Pro Lys Gly Ala Tyr Val Asp Asn Phe Glu
                565                 570                 575

CTG GAC TTT TTA CGC TTT AAA CTG CCG CCA AAC GAA GAT GAC CGT TTG 1776
Leu Asp Phe Leu Arg Phe Lys Leu Pro Pro Asn Glu Asp Asp Arg Leu
            580                 585                 590
ATC TCA CAG CAG CTA ATG CTA ATG CGA GTA ACA GAC GAA GCC ATT CGT 1824
Ile Ser Gln Gln Leu Met Leu Met Arg Val Thr Asp Glu Ala Ile Arg
        595                 600                 605
GAT GCC AAG CTT GAG CCG GGG CAA AAA GTA GCT GTA TTA GTG GCA ATG 1872
Asp Ala Lys Leu Glu Pro Gly Gln Lys Val Ala Val Leu Val Ala Met
    610                 615                 620
GAA ACT GAG CTT GAA CTG CAT CAG TTC CGC GGC CGG GTT AAC TTG CAT 1920
Glu Thr Glu Leu Glu Leu His Gln Phe Arg Gly Arg Val Asn Leu His
625                 630                 635                 640
ACT CAA TTA GCG CAA AGT CTT GCC GCC ATG GGC GTG AGT TTA TCA ACG 1968
Thr Gln Leu Ala Gln Ser Leu Ala Ala Met Gly Val Ser Leu Ser Thr
                645                 650                 655
GAT GAA TAC CAA GCG CTT GAA GCC ATC GCC ATG GAC AGC GTG CTT GAT 2016
Asp Glu Tyr Gln Ala Leu Glu Ala Ile Ala Met Asp Ser Val Leu Asp
            660                 665                 670
GCT GCC AAG CTC AAT CAG TAC ACC AGC TTT ATT GGT AAT ATT ATG GCG 2064
Ala Ala Lys Leu Asn Gln Tyr Thr Ser Phe Ile Gly Asn Ile Met Ala
        675                 680                 685
TCA CGC GTG GCG TCA CTA TGG GAC TTT AAT GGC CCA GCC TTC ACT ATT 2112
Ser Arg Val Ala Ser Leu Trp Asp Phe Asn Gly Pro Ala Phe Thr Ile
    690                 695                 700
TCA GCA GCA GAG CAA TCT GTG AGC CGC TGT ATC GAT GTG GCG CAA AAC 2160
Ser Ala Ala Glu Gln Ser Val Ser Arg Cys Ile Asp Val Ala Gln Asn
705                 710                 715                 720
CTC ATC ATG GAG GAT AAC CTA GAT GCG GTG GTG ATT GCA GCG GTC GAT 2208
Leu Ile Met Glu Asp Asn Leu Asp Ala Val Val Ile Ala Ala Val Asp
                725                 730                 735
CTC TCT GGT AGC TTT GAG CAA GTC ATT CTT AAA AAT GCC ATT GCA CCT 2256
Leu Ser Gly Ser Phe Glu Gln Val Ile Leu Lys Asn Ala Ile Ala Pro
            740                 745                 750
GTA GCC ATT GAG CCA AAC CTC GAA GCA AGC CTT AAT CCA ACA TCA GCA 2304
Val Ala Ile Glu Pro Asn Leu Glu Ala Ser Leu Asn Pro Thr Ser Ala
        755                 760                 765

AGC TGG AAT GTC GGT GAA GGT GCT GGC GCG GTC GTG CTT GTT AAA AAT 2352
Ser Trp Asn Val Gly Glu Gly Ala Gly Ala Val Val Leu Val Lys Asn
    770                 775                 780
GAA GCT ACA TCG GGC TGC TCA TAC GGC CAA ATT GAT GCA CTT GGC TTT 2400
Glu Ala Thr Ser Gly Cys Ser Tyr Gly Gln Ile Asp Ala Leu Gly Phe
785                 790                 795                 800
GCT AAA ACT GCC GAA ACA GCG TTG GCT ACC GAC AAG CTA CTG AGC CAA 2448
Ala Lys Thr Ala Glu Thr Ala Leu Ala Thr Asp Lys Leu Leu Ser Gln
                805                 810                 815
ACT GCC ACA GAC TTT AAT AAG GTT AAA GTG ATT GAA ACT ATG GCA GCG 2496
Thr Ala Thr Asp Phe Asn Lys Val Lys Val Ile Glu Thr Met Ala Ala
            820                 825                 830
CCT GCT AGC CAA ATT CAA TTA GCG CCA ATA GTT AGC TCT CAA GTG ACT 2544
Pro Ala Ser Gln Ile Gln Leu Ala Pro Ile Val Ser Ser Gln Val Thr
        835                 840                 845
CAC ACT GCT GCA GAG CAG CGT GTT GGT CAC TGC TTT GCT GCA GCG GGT 2592
His Thr Ala Ala Glu Gln Arg Val Gly His Cys Phe Ala Ala Ala Gly
    850                 855                 860
ATG GCA AGC CTA TTA CAC GGC TTA CTT AAC TTA AAT ACT GTA GCC CAA 2640
Met Ala Ser Leu Leu His Gly Leu Leu Asn Leu Asn Thr Val Ala Gln
865                 870                 875                 880
ACC AAT AAA GCC AAT TGC GCG CTT ATC AAC AAT ATC AGT GAA AAC CAA 2688
Thr Asn Lys Ala Asn Cys Ala Leu Ile Asn Asn Ile Ser Glu Asn Gln
                885                 890                 895
TTA TCA CAG CTG TTG ATT AGC CAA ACA GCG AGC GAA CAA CAA GCA TTA 2736
Leu Ser Gln Leu Leu Ile Ser Gln Thr Ala Ser Glu Gln Gln Ala Leu
            900                 905                 910
ACC GCG CGT TTA AGC AAT GAG CTT AAA TCC GAT GCT AAA CAC CAA CTG 2784
Thr Ala Arg Leu Ser Asn Glu Leu Lys Ser Asp Ala Lys His Gln Leu
        915                 920                 925
GTT AAG CAA GTC ACC TTA GGT GGC CGT GAT ATC TAC CAG CAT ATT GTT 2832
Val Lys Gln Val Thr Leu Gly Gly Arg Asp Ile Tyr Gln His Ile Val
    930                 935                 940
GAT ACA CCG CTT GCA AGC CTT GAA AGC ATT ACT CAG AAA TTG GCG CAA 2880
Asp Thr Pro Leu Ala Ser Leu Glu Ser Ile Thr Gln Lys Leu Ala Gln
945                 950                 955                 960

GCG ACA GCA TCG ACA GTG GTC AAC CAA GTT AAA CCT ATT AAG GCC GCT 2928
Ala Thr Ala Ser Thr Val Val Asn Gln Val Lys Pro Ile Lys Ala Ala
                965                 970                 975
GGC TCA GTC GAA ATG GCT AAC TCA TTC GAA ACG GAA AGC TCA GCA GAG 2976
Gly Ser Val Glu Met Ala Asn Ser Phe Glu Thr Glu Ser Ser Ala Glu
            980                 985                 990
CCA CAA ATA ACA ATT GCA GCA CAA CAG ACT GCA AAC ATT GGC GTC ACC 3024
Pro Gln Ile Thr Ile Ala Ala Gln Gln Thr Ala Asn Ile Gly Val Thr
        995                 1000                1005
GCT CAG GCA ACC AAA CGT GAA TTA GGT ACC CCA CCA ATG ACA ACA AAT 3072
Ala Gln Ala Thr Lys Arg Glu Leu Gly Thr Pro Pro Met Thr Thr Asn
    1010                1015                1020
ACC ATT GCT AAT ACA GCA AAT AAT TTA GAC AAG ACT CTT GAG ACT GTT 3120
Thr Ile Ala Asn Thr Ala Asn Asn Leu Asp Lys Thr Leu Glu Thr Val
1025                1030                1035                1040
GCT GGC AAT ACT GTT GCT AGC AAG GTT GGC TCT GGC GAC ATA GTC AAT 3168
Ala Gly Asn Thr Val Ala Ser Lys Val Gly Ser Gly Asp Ile Val Asn
                1045                1050                1055
TTT CAA CAG AAC CAA CAA TTG GCT CAA CAA GCT CAC CTC GCC TTT CTT 3216
Phe Gln Gln Asn Gln Gln Leu Ala Gln Gln Ala His Leu Ala Phe Leu
            1060                1065                1070
GAA AGC CGC AGT GCG GGT ATG AAG GTG GCT GAT GCT TTA TTG AAG CAA 3264
Glu Ser Arg Ser Ala Gly Met Lys Val Ala Asp Ala Leu Leu Lys Gln
        1075                1080                1085
CAG CTA GCT CAA GTA ACA GGC CAA ACT ATC GAT AAT CAG GCC CTC GAT 3312
Gln Leu Ala Gln Val Thr Gly Gln Thr Ile Asp Asn Gln Ala Leu Asp
    1090                1095                1100
ACT CAA GCC GTC GAT ACT CAA ACA AGC GAG AAT GTA GCG ATT GCC GCA 3360
Thr Gln Ala Val Asp Thr Gln Thr Ser Glu Asn Val Ala Ile Ala Ala
1105                1110                1115                1120
GAA TCA CCA GTT CAA GTT ACA ACA CCT GTT CAA GTT ACA ACA CCT GTT 3408
Glu Ser Pro Val Gln Val Thr Thr Pro Val Gln Val Thr Thr Pro Val
                1125                1130                1135
CAA ATC AGT GTT GTG GAG TTA AAA CCA GAT CAC GCT AAT GTG CCA CCA 3456
Gln Ile Ser Val Val Glu Leu Lys Pro Asp His Ala Asn Val Pro Pro
            1140                1145                1150

TAC ACG CCG CCA GTG CCT GCA TTA AAG CCG TGT ATC TGG AAC TAT GCC 3504
Tyr Thr Pro Pro Val Pro Ala Leu Lys Pro Cys Ile Trp Asn Tyr Ala
        1155                1160                1165
GAT TTA GTT GAG TAC GCA GAA GGC GAT ATC GCC AAG GTA TTT GGC AGT 3552
Asp Leu Val Glu Tyr Ala Glu Gly Asp Ile Ala Lys Val Phe Gly Ser
    1170                1175                1180
GAT TAT GCC ATT ATC GAC AGC TAC TCG CGC CGC GTA CGT CTA CCG ACC 3600
Asp Tyr Ala Ile Ile Asp Ser Tyr Ser Arg Arg Val Arg Leu Pro Thr
1185                1190                1195                1200
ACT GAC TAC CTG TTG GTA TCG CGC GTG ACC AAA CTT GAT GCG ACC ATC 3648
Thr Asp Tyr Leu Leu Val Ser Arg Val Thr Lys Leu Asp Ala Thr Ile
                1205                1210                1215
AAT CAA TTT AAG CCA TGC TCA ATG ACC ACT GAG TAC GAC ATC CCT GTT 3696
Asn Gln Phe Lys Pro Cys Ser Met Thr Thr Glu Tyr Asp Ile Pro Val
            1220                1225                1230
GAT GCG CCG TAC TTA GTA GAC GGA CAA ATC CCT TGG GCG GTA GCA GTA 3744
Asp Ala Pro Tyr Leu Val Asp Gly Gln Ile Pro Trp Ala Val Ala Val
        1235                1240                1245
GAA TCA GGC CAA TGT GAC TTG ATG CTT ATT AGC TAT CTC GGT ATC GAC 3792
Glu Ser Gly Gln Cys Asp Leu Met Leu Ile Ser Tyr Leu Gly Ile Asp
    1250                1255                1260
TTT GAG AAC AAA GGC GAG CGG GTT TAT CGA CTA CTC GAT TGT ACC CTC 3840
Phe Glu Asn Lys Gly Glu Arg Val Tyr Arg Leu Leu Asp Cys Thr Leu
1265                1270                1275                1280
ACC TTC CTA GGC GAC TTG CCA CGT GGC GGA GAT ACC CTA CGT TAC GAC 3888
Thr Phe Leu Gly Asp Leu Pro Arg Gly Gly Asp Thr Leu Arg Tyr Asp
                1285                1290                1295
ATT AAG ATC AAT AAC TAT GCT CGC AAC GGC GAC ACC CTG CTG TTC TTC 3936
Ile Lys Ile Asn Asn Tyr Ala Arg Asn Gly Asp Thr Leu Leu Phe Phe
            1300                1305                1310
TTC TCG TAT GAG TGT TTT GTT GGC GAC AAG ATG ATC CTC AAG ATG GAT 3984
Phe Ser Tyr Glu Cys Phe Val Gly Asp Lys Met Ile Leu Lys Met Asp
        1315                1320                1325
GGC GGC TGC GCT GGC TTC TTC ACT GAT GAA GAG CTT GCC GAC GGT AAA 4032
Gly Gly Cys Ala Gly Phe Phe Thr Asp Glu Glu Leu Ala Asp Gly Lys
    1330                1335                1340

GGC GTG ATT CGC ACA GAA GAA GAG ATT AAA GCT CGC AGC CTA GTG CAA 4080
Gly Val Ile Arg Thr Glu Glu Glu Ile Lys Ala Arg Ser Leu Val Gln
1345                1350                1355                1360
AAG CAA CGC TTT AAT CCG TTA CTA GAT TGT CCT AAA ACC CAA TTT AGT 4128
Lys Gln Arg Phe Asn Pro Leu Leu Asp Cys Pro Lys Thr Gln Phe Ser
                1365                1370                1375
TAT GGT GAT ATT CAT AAG CTA TTA ACT GCT GAT ATT GAG GGT TGT TTT 4176
Tyr Gly Asp Ile His Lys Leu Leu Thr Ala Asp Ile Glu Gly Cys Phe
            1380                1385                1390
GGC CCA AGC CAC AGT GGC GTC CAC CAG CCG TCA CTT TGT TTC GCA TCT 4224
Gly Pro Ser His Ser Gly Val His Gln Pro Ser Leu Cys Phe Ala Ser
        1395                1400                1405
GAA AAA TTC TTG ATG ATT GAA CAA GTC AGC AAG GTT GAT CGC ACT GGC 4272
Glu Lys Phe Leu Met Ile Glu Gln Val Ser Lys Val Asp Arg Thr Gly
    1410                1415                1420
GGT ACT TGG GGA CTT GGC TTA ATT GAG GGT CAT AAG CAG CTT GAA GCA 4320
Gly Thr Trp Gly Leu Gly Leu Ile Glu Gly His Lys Gln Leu Glu Ala
1425                1430                1435                1440
GAC CAC TGG TAC TTC CCA TGT CAT TTC AAG GGC GAC CAA GTG ATG GCT 4368
Asp His Trp Tyr Phe Pro Cys His Phe Lys Gly Asp Gln Val Met Ala
                1445                1450                1455
GGC TCG CTA ATG GCT GAA GGT TGT GGC CAG TTA TTG CAG TTC TAT ATG 4416
Gly Ser Leu Met Ala Glu Gly Cys Gly Gln Leu Leu Gln Phe Tyr Met
            1460                1465                1470
CTG CAC CTT GGT ATG CAT ACC CAA ACT AAA AAT GGT CGT TTC CAA CCT 4464
Leu His Leu Gly Met His Thr Gln Thr Lys Asn Gly Arg Phe Gln Pro
        1475                1480                1485
CTT GAA AAC GCC TCA CAG CAA GTA CGC TGT CGC GGT CAA GTG CTG CCA 4512
Leu Glu Asn Ala Ser Gln Gln Val Arg Cys Arg Gly Gln Val Leu Pro
    1490                1495                1500
CAA TCA GGC GTG CTA ACT TAC CGT ATG GAA GTG ACT GAA ATC GGT TTC 4560
Gln Ser Gly Val Leu Thr Tyr Arg Met Glu Val Thr Glu Ile Gly Phe
1505                1510                1515                1520
AGT CCA CGC CCA TAT GCT AAA GCT AAC ATC GAT ATC TTG CTT AAT GGC 4608
Ser Pro Arg Pro Tyr Ala Lys Ala Asn Ile Asp Ile Leu Leu Asn Gly
                1525                1530                1535

AAA GCG GTA GTG GAT TTC CAA AAC CTA GGG GTG ATG ATA AAA GAG GAA 4656
Lys Ala Val Val Asp Phe Gln Asn Leu Gly Val Met Ile Lys Glu Glu
            1540                1545                1550
GAT GAG TGT ACT CGT TAT CCA CTT TTG ACT GAA TCA ACA ACG GCT AGC 4704
Asp Glu Cys Thr Arg Tyr Pro Leu Leu Thr Glu Ser Thr Thr Ala Ser
        1555                1560                1565
ACT GCA CAA GTA AAC GCT CAA ACA AGT GCG AAA AAG GTA TAC AAG CCA 4752
Thr Ala Gln Val Asn Ala Gln Thr Ser Ala Lys Lys Val Tyr Lys Pro
    1570                1575                1580
GCA TCA GTC AAT GCG CCA TTA ATG GCA CAA ATT CCT GAT CTG ACT AAA 4800
Ala Ser Val Asn Ala Pro Leu Met Ala Gln Ile Pro Asp Leu Thr Lys
1585                1590                1595                1600
GAG CCA AAC AAG GGC GTT ATT CCG ATT TCC CAT GTT GAA GCA CCA ATT 4848
Glu Pro Asn Lys Gly Val Ile Pro Ile Ser His Val Glu Ala Pro Ile
                1605                1610                1615
ACG CCA GAC TAC CCG AAC CGT GTA CCT GAT ACA GTG CCA TTC ACG CCG 4896
Thr Pro Asp Tyr Pro Asn Arg Val Pro Asp Thr Val Pro Phe Thr Pro
            1620                1625                1630
TAT CAC ATG TTT GAG TTT GCT ACA GGC AAT ATC GAA AAC TGT TTC GGG 4944
Tyr His Met Phe Glu Phe Ala Thr Gly Asn Ile Glu Asn Cys Phe Gly
        1635                1640                1645
CCA GAG TTC TCA ATC TAT CGC GGC ATG ATC CCA CCA CGT ACA CCA TGC 4992
Pro Glu Phe Ser Ile Tyr Arg Gly Met Ile Pro Pro Arg Thr Pro Cys
    1650                1655                1660
GGT GAC TTA CAA GTG ACC ACA CGT GTG ATT GAA GTT AAC GGT AAG CGT 5040
Gly Asp Leu Gln Val Thr Thr Arg Val Ile Glu Val Asn Gly Lys Arg
1665                1670                1675                1680
GGC GAC TTT AAA AAG CCA TCA TCG TGT ATC GCT GAA TAT GAA GTG CCT 5088
Gly Asp Phe Lys Lys Pro Ser Ser Cys Ile Ala Glu Tyr Glu Val Pro
                1685                1690                1695
GCA GAT GCG TGG TAT TTC GAT AAA AAC AGC CAC GGC GCA GTG ATG CCA 5136
Ala Asp Ala Trp Tyr Phe Asp Lys Asn Ser His Gly Ala Val Met Pro
            1700                1705                1710
TAT TCA ATT TTA ATG GAG ATC TCA CTG CAA CCT AAC GGC TTT ATC TCA 5184
Tyr Ser Ile Leu Met Glu Ile Ser Leu Gln Pro Asn Gly Phe Ile Ser
        1715                1720                1725

GGT TAC ATG GGC ACA ACC CTA GGC TTC CCT GGC CTT GAG CTG TTC TTC 5232
Gly Tyr Met Gly Thr Thr Leu Gly Phe Pro Gly Leu Glu Leu Phe Phe
    1730                1735                1740
CGT AAC TTA GAC GGT AGC GGT GAG TTA CTA CGT GAA GTA GAT TTA CGT 5280
Arg Asn Leu Asp Gly Ser Gly Glu Leu Leu Arg Glu Val Asp Leu Arg
1745                1750                1755                1760
GGT AAA ACC ATC CGT AAC GAC TCA CGT TTA TTA TCA ACA GTG ATG GCC 5328
Gly Lys Thr Ile Arg Asn Asp Ser Arg Leu Leu Ser Thr Val Met Ala
                1765                1770                1775
GGC ACT AAC ATC ATC CAA AGC TTT AGC TTC GAG CTA AGC ACT GAC GGT 5376
Gly Thr Asn Ile Ile Gln Ser Phe Ser Phe Glu Leu Ser Thr Asp Gly
            1780                1785                1790
GAG CCT TTC TAT CGC GGC ACT GCG GTA TTT GGC TAT TTT AAA GGT GAC 5424
Glu Pro Phe Tyr Arg Gly Thr Ala Val Phe Gly Tyr Phe Lys Gly Asp
        1795                1800                1805
GCA CTT AAA GAT CAG CTA GGC CTA GAT AAC GGT AAA GTC ACT CAG CCA 5472
Ala Leu Lys Asp Gln Leu Gly Leu Asp Asn Gly Lys Val Thr Gln Pro
    1810                1815                1820
TGG CAT GTA GCT AAC GGC GTT GCT GCA AGC ACT AAG GTG AAC CTG CTT 5520
Trp His Val Ala Asn Gly Val Ala Ala Ser Thr Lys Val Asn Leu Leu
1825                1830                1835                1840
GAT AAG AGC TGC CGT CAC TTT AAT GCG CCA GCT AAC CAG CCA CAC TAT 5568
Asp Lys Ser Cys Arg His Phe Asn Ala Pro Ala Asn Gln Pro His Tyr
                1845                1850                1855
CGT CTA GCC GGT GGT CAG CTG AAC TTT ATC GAC AGT GTT GAA ATT GTT 5616
Arg Leu Ala Gly Gly Gln Leu Asn Phe Ile Asp Ser Val Glu Ile Val
            1860                1865                1870
GAT AAT GGC GGC ACC GAA GTT 5637
Asp Asn Gly Gly Thr Glu Val
        1875

SEQ ID NO: 8
SEQUENCE LENGTH: 1629
SEQUENCE TYPE: Nucleic acid
STRANDNESS: Double strand
TOPOLOGY: Linear
MOLECULE TYPE: Genomic DNA

ORIGINAL SOURCE: Shewanella putrefaciens SCRC-2874
(FERM BP-1625)

SEQUENCE
ATG AAT CCT ACA GCA ACT AAC GAA ATG CTT TCT CCG TGG CCA TGG GCT 48
Met Asn Pro Thr Ala Thr Asn Glu Met Leu Ser Pro Trp Pro Trp Ala
1               5                   10                  15
GTG ACA GAG TCA AAT ATC AGT TTT GAC GTG CAA GTG ATG GAA CAA CAA 96
Val Thr Glu Ser Asn Ile Ser Phe Asp Val Gln Val Met Glu Gln Gln
            20                  25                  30
CTT AAA GAT TTT AGC CGG GCA TGT TAC GTG GTC AAT CAT GCC GAC CAC 144
Leu Lys Asp Phe Ser Arg Ala Cys Tyr Val Val Asn His Ala Asp His
        35                  40                  45
GGC TTT GGT ATT GCG CAA ACT GCC GAT ATC GTG ACT GAA CAA GCG GCA 192
Gly Phe Gly Ile Ala Gln Thr Ala Asp Ile Val Thr Glu Gln Ala Ala
    50                  55                  60
AAC AGC ACA GAT TTA CCT GTT AGT GCT TTT ACT CCT GCA TTA GGT ACC 240
Asn Ser Thr Asp Leu Pro Val Ser Ala Phe Thr Pro Ala Leu Gly Thr
65                  70                  75                  80
GAA AGC CTA GGC GAC AAT AAT TTC CGC CGC GTT CAC GGC GTT AAA TAC 288
Glu Ser Leu Gly Asp Asn Asn Phe Arg Arg Val His Gly Val Lys Tyr
                85                  90                  95
GCT TAT TAC GCA GGC GCT ATG GCA AAC GGT ATT TCA TCT GAA GAG CTA 336
Ala Tyr Tyr Ala Gly Ala Met Ala Asn Gly Ile Ser Ser Glu Glu Leu
            100                 105                 110
GTG ATT GCC CTA GGT CAA GCT GGC ATT TTG TGT GGT TCG TTT GGA GCA 384
Val Ile Ala Leu Gly Gln Ala Gly Ile Leu Cys Gly Ser Phe Gly Ala
        115                 120                 125
GCC GGT CTT ATT CCA AGT CGC GTT GAA GCG GCA ATT AAC CGT ATT CAA 432
Ala Gly Leu Ile Pro Ser Arg Val Glu Ala Ala Ile Asn Arg Ile Gln
    130                 135                 140
GCA GCG CTG CCA AAT GGC CCT TAT ATG TTT AAC CTT ATC CAT AGT CCT 480
Ala Ala Leu Pro Asn Gly Pro Tyr Met Phe Asn Leu Ile His Ser Pro
145                 150                 155                 160
AGC GAG CCA GCA TTA GAG CGT GGC AGC GTA GAG CTA TTT TTA AAG CAT 528
Ser Glu Pro Ala Leu Glu Arg Gly Ser Val Glu Leu Phe Leu Lys His
                165                 170                 175

AAG GTA CGC ACC GTT GAA GCA TCA GCT TTC TTA GGT CTA ACA CCA CAA 576
Lys Val Arg Thr Val Glu Ala Ser Ala Phe Leu Gly Leu Thr Pro Gln
            180                 185                 190
ATC GTC TAT TAC CGT GCA GCA GGA TTG AGC CGA GAC GCA CAA GGT AAA 624
Ile Val Tyr Tyr Arg Ala Ala Gly Leu Ser Arg Asp Ala Gln Gly Lys
        195                 200                 205
GTT GTG GTT GGT AAC AAG GTT ATC GCT AAA GTA AGT CGC ACC GAA GTG 672
Val Val Val Gly Asn Lys Val Ile Ala Lys Val Ser Arg Thr Glu Val
    210                 215                 220
GCT GAA AAG TTT ATG ATG CCA GCG CCC GCA AAA ATG CTA CAA AAA CTA 720
Ala Glu Lys Phe Met Met Pro Ala Pro Ala Lys Met Leu Gln Lys Leu
225                 230                 235                 240
GTT GAT GAC GGT TCA ATT ACC GCT GAG CAA ATG GAG CTG GCG CAA CTT 768
Val Asp Asp Gly Ser Ile Thr Ala Glu Gln Met Glu Leu Ala Gln Leu
                245                 250                 255
GTA CCT ATG GCT GAC GAC ATC ACT GCA GAG GCC GAT TCA GGT GGC CAT 816
Val Pro Met Ala Asp Asp Ile Thr Ala Glu Ala Asp Ser Gly Gly His
            260                 265                 270
ACT GAT AAC CGT CCA TTA GTA ACA TTG CTG CCA ACC ATT TTA GCG CTG 864
Thr Asp Asn Arg Pro Leu Val Thr Leu Leu Pro Thr Ile Leu Ala Leu
        275                 280                 285
AAA GAA GAA ATT CAA GCT AAA TAC CAA TAC GAC ACT CCT ATT CGT GTC 912
Lys Glu Glu Ile Gln Ala Lys Tyr Gln Tyr Asp Thr Pro Ile Arg Val
    290                 295                 300
GGT TGT GGT GGC GGT GTG GGT ACG CCT GAT GCA GCG CTG GCA ACG TTT 960
Gly Cys Gly Gly Gly Val Gly Thr Pro Asp Ala Ala Leu Ala Thr Phe
305                 310                 315                 320
AAC ATG GGC GCG GCG TAT ATT GTT ACC GGC TCT ATC AAC CAA GCT TGT 1008
Asn Met Gly Ala Ala Tyr Ile Val Thr Gly Ser Ile Asn Gln Ala Cys
                325                 330                 335
GTT GAA GCG GGC GCA AGT GAT CAC ACT CGT AAA TTA CTT GCC ACC ACT 1056
Val Glu Ala Gly Ala Ser Asp His Thr Arg Lys Leu Leu Ala Thr Thr
            340                 345                 350
GAA ATG GCC GAT GTG ACT ATG GCA CCA GCT GCA GAT ATG TTC GAG ATG 1104
Glu Met Ala Asp Val Thr Met Ala Pro Ala Ala Asp Met Phe Glu Met
        355                 360                 365

GGC GTA AAA CTG CAG GTG GTT AAG CGC GGC ACG CTA TTC CCA ATG CGC 1152
Gly Val Lys Leu Gln Val Val Lys Arg Gly Thr Leu Phe Pro Met Arg
    370                 375                 380
GCT AAC AAG CTA TAT GAG ATC TAC ACC CGT TAC GAT TCA ATC GAA GCG 1200
Ala Asn Lys Leu Tyr Glu Ile Tyr Thr Arg Tyr Asp Ser Ile Glu Ala
385                 390                 395                 400
ATC CCA TTA GAC GAG CGT GAA AAG CTT GAG AAA CAA GTA TTC CGC TCA 1248
Ile Pro Leu Asp Glu Arg Glu Lys Leu Glu Lys Gln Val Phe Arg Ser
                405                 410                 415
AGC CTA GAT GAA ATA TGG GCA GGT ACA GTG GCG CAC TTT AAC GAG CGC 1296
Ser Leu Asp Glu Ile Trp Ala Gly Thr Val Ala His Phe Asn Glu Arg
            420                 425                 430
GAC CCT AAG CAA ATC GAA CGC GCA GAG GGT AAC CCT AAG CGT AAA ATG 1344
Asp Pro Lys Gln Ile Glu Arg Ala Glu Gly Asn Pro Lys Arg Lys Met
        435                 440                 445
GCA TTG ATT TTC CGT TGG TAC TTA GGT CTT TCT AGT CGC TGG TCA AAC 1392
Ala Leu Ile Phe Arg Trp Tyr Leu Gly Leu Ser Ser Arg Trp Ser Asn
    450                 455                 460
TCA GGC GAA GTG GGT CGT GAA ATG GAT TAT CAA ATT TGG GCT GGC CCT 1440
Ser Gly Glu Val Gly Arg Glu Met Asp Tyr Gln Ile Trp Ala Gly Pro
465                 470                 475                 480
GCT CTC GGT GCA TTT AAC CAA TGG GCA AAA GGC AGT TAC TTA GAT AAC 1488
Ala Leu Gly Ala Phe Asn Gln Trp Ala Lys Gly Ser Tyr Leu Asp Asn
                485                 490                 495
TAT CAA GAC CGA AAT GCC GTC GAT TTG GCA AAG CAC TTA ATG TAC GGC 1536
Tyr Gln Asp Arg Asn Ala Val Asp Leu Ala Lys His Leu Met Tyr Gly
            500                 505                 510
GCG GCT TAC TTA AAT CGT ATT AAC TCG CTA ACG GCT CAA GGC GTT AAA 1584
Ala Ala Tyr Leu Asn Arg Ile Asn Ser Leu Thr Ala Gln Gly Val Lys
        515                 520                 525
GTG CCA GCA CAG TTA CTT CGC TGG AAG CCA AAC CAA AGA ATG GCC 1629
9Val Pro Ala Gln Leu Leu Arg Trp Lys Pro Asn Gln Arg Met Ala
530 535 540

SEQ ID NO: 9
SEQUENCE LENGTH: 1596
SEQUENCE TYPE: Nucleic acid

STRANDNESS: Double strand
TOPOLOGY: Linear
MOLECULE TYPE: Genomic DNA
ORIGINAL SOURCE: Shewanella putrefaciens SCRC-2874
(FERM BP-1625)

SEQUENCE
GTG CAA CTA CCA TTA ATT GAG GCC TCA TTA GTT AAA TTA TCT GAG CAA 48
Val Gln Leu Pro Leu Ile Glu Ala Ser Leu Val Lys Leu Ser Glu Gln
1               5                   10                  15
GAG CTC ACC TCT TTA AAT TAC GCT TTT CAG CAA ATG AGA AAG CCA CTA 96
Glu Leu Thr Ser Leu Asn Tyr Ala Phe Gln Gln Met Arg Lys Pro Leu
            20                  25                  30
CAA ACC ATT AAT TAC GAC TAT GCG GTG TGG GAC AGA ACC TAC AGC TAT 144
Gln Thr Ile Asn Tyr Asp Tyr Ala Val Trp Asp Arg Thr Tyr Ser Tyr
        35                  40                  45
ATG AAA TCA AAC TCA GCG AGC GCT AAA AGG TAC TAT GAA AAA CAT GAG 192
Met Lys Ser Asn Ser Ala Ser Ala Lys Arg Tyr Tyr Glu Lys His Glu
    50                  55                  60
TAC CCA GAT GAT ACG TTC AAG AGT TTA AAA GTC GAC GGA GTA TTT ATA 240
Tyr Pro Asp Asp Thr Phe Lys Ser Leu Lys Val Asp Gly Val Phe Ile
65                  70                  75                  80
TTC AAC CGT ACA AAT CAG CCA GTT TTT AGT AAA GGT TTT AAT CAT AGA 288
Phe Asn Arg Thr Asn Gln Pro Val Phe Ser Lys Gly Phe Asn His Arg
                85                  90                  95
AAT GAT ATA CCG CTG GTC TTT GAA TTA ACT GAC TTT AAA CAA CAT CCA 336
Asn Asp Ile Pro Leu Val Phe Glu Leu Thr Asp Phe Lys Gln His Pro
            100                 105                 110
CAA AAC ATC GCA TTA TCT CCA CAA ACC AAA CAG GCA CAC CCA CCG GCA 384
Gln Asn Ile Ala Leu Ser Pro Gln Thr Lys Gln Ala His Pro Pro Ala
        115                 120                 125
AGT AAG CCG TTA GAC TCC CCT GAT GAT GTG CCT TCT ACC CAT GGG GTT 432
Ser Lys Pro Leu Asp Ser Pro Asp Asp Val Pro Ser Thr His Gly Val
    130                 135                 140
ATC GCC ACA CGA TAC GGT CCA GCA ATT TAT AGC TCT ACC AGC ATT TTA 480
Ile Ala Thr Arg Tyr Gly Pro Ala Ile Tyr Ser Ser Thr Ser Ile Leu
145                 150                 155                 160

AAA TCT GAT CGT AGC GGC TCC CAA CTT GGT TAT TTA GTC TTC ATT AGG 528
Lys Ser Asp Arg Ser Gly Ser Gln Leu Gly Tyr Leu Val Phe Ile Arg
                165                 170                 175
TTA ATT GAT GAA TGG TTC ATC GCT GAG CTA TCG CAA TAC ACT GCC GCA 576
Leu Ile Asp Glu Trp Phe Ile Ala Glu Leu Ser Gln Tyr Thr Ala Ala
            180                 185                 190
GGT GTT GAA ATC GCT ATG GCT GAT GCC GCA GAC GCA CAA TTA GCG AGA 624
Gly Val Glu Ile Ala Met Ala Asp Ala Ala Asp Ala Gln Leu Ala Arg
        195                 200                 205
TTA GGC GCA AAC ACT AAG CTT AAT AAA GTA ACC GCT ACA TCC GAA CGG 672
Leu Gly Ala Asn Thr Lys Leu Asn Lys Val Thr Ala Thr Ser Glu Arg
    210                 215                 220
TTA ATA ACT AAT GTC GAT GGT AAG CCT CTG TTG AAG TTA GTG CTT TAC 720
Leu Ile Thr Asn Val Asp Gly Lys Pro Leu Leu Lys Leu Val Leu Tyr
225                 230                 235                 240
CAT ACC AAT AAC CAA CCG CCG CCG ATG CTA GAT TAC AGT ATA ATA ATT 768
His Thr Asn Asn Gln Pro Pro Pro Met Leu Asp Tyr Ser Ile Ile Ile
                245                 250                 255
CTA TTA GTT GAG ATG TCA TTT TTA CTG ATC CTC GCT TAT TTC CTT TAC 816
Leu Leu Val Glu Met Ser Phe Leu Leu Ile Leu Ala Tyr Phe Leu Tyr
            260                 265                 270
TCC TAC TTC TTA GTC AGG CCA GTT AGA AAG CTG GCT TCA GAT ATT AAA 864
Ser Tyr Phe Leu Val Arg Pro Val Arg Lys Leu Ala Ser Asp Ile Lys
        275                 280                 285
AAA ATG GAT AAA AGT CGT GAA ATT AAA AAG CTA AGG TAT CAC TAC CCT 912
Lys Met Asp Lys Ser Arg Glu Ile Lys Lys Leu Arg Tyr His Tyr Pro
    290                 295                 300
ATT ACT GAG CTA TTC TTA GTC AGG CCA GTT AGA AAG CTG GCT TCA GAT 960
Ile Thr Glu Leu Phe Leu Val Arg Pro Val Arg Lys Leu Ala Ser Asp
305                 310                 315                 320
ATT AAA AAA ATG GAT AAA AGT CGT GAA ATT AAA AAG CTA AGG TAT CAC 1008
Ile Lys Lys Met Asp Lys Ser Arg Glu Ile Lys Lys Leu Arg Tyr His
                325                 330                 335
TAC CCT ATT ACT GAG CTA GAT AAA TTA ACC AAT ATT CCC AAT CGT CGC 1056
Tyr Pro Ile Thr Glu Leu Asp Lys Leu Thr Asn Ile Pro Asn Arg Arg
            340                 345                 350

GCT TTT GAG CAG CGA CTT GAA ACC TAT TGC CAA CTG CTA GCC CGG CAA 1104
Ala Phe Glu Gln Arg Leu Glu Thr Tyr Cys Gln Leu Leu Ala Arg Gln
        355                 360                 365
CAA ATT GGC TTT ACT CTC ATC ATT GCC GAT GTG GAT CAT TTT AAA GAG 1152
Gln Ile Gly Phe Thr Leu Ile Ile Ala Asp Val Asp His Phe Lys Glu
    370                 375                 380
TAC AAC GAT ACT CTT GGG CAC CTT GCT GGG GAT GAA GCA TTA ATA AAA 1200
Tyr Asn Asp Thr Leu Gly His Leu Ala Gly Asp Glu Ala Leu Ile Lys
385                 390                 395                 400
GTG GCA CAA ACA CTA TCG CAA CAG TTT TAC CGT GCA GAA GAT ATT TGT 1248
Val Ala Gln Thr Leu Ser Gln Gln Phe Tyr Arg Ala Glu Asp Ile Cys
                405                 410                 415
GCC CGT TTT GGT GGT GAA GAA TTT ATT ATG TTA TTT CGA GAC ATA CCT 1296
Ala Arg Phe Gly Gly Glu Glu Phe Ile Met Leu Phe Arg Asp Ile Pro
            420                 425                 430
GAT GAG CCC TTG CAG AGA AAG CTC GAT GCG ATG CTG CAC TCT TTT GCA 1344
Asp Glu Pro Leu Gln Arg Lys Leu Asp Ala Met Leu His Ser Phe Ala
        435                 440                 445
GAG CTC AAC CTA CCT CAT CCA AAC TCA TCA ACC GCT AAT TAC GTT ACT 1392
Glu Leu Asn Leu Pro His Pro Asn Ser Ser Thr Ala Asn Tyr Val Thr
    450                 455                 460
GTG AGC CTT GGG GTT TGC ACA GTT GTT GCT GTT GAT GAT TTT GAA TTT 1440
Val Ser Leu Gly Val Cys Thr Val Val Ala Val Asp Asp Phe Glu Phe
465                 470                 475                 480
AAA AGT GAG TCG CAT ATT ATT GGC AGT CAG GCT GCA TTA ATC GCA GAT 1488
Lys Ser Glu Ser His Ile Ile Gly Ser Gln Ala Ala Leu Ile Ala Asp
                485                 490                 495
AAG GCG CTT TAT CAT GCT AAA GCC TGT GGT CGT AAC CAG TTG TCA AAA 1536
Lys Ala Leu Tyr His Ala Lys Ala Cys Gly Arg Asn Gln Leu Ser Lys
            500                 505                 510
ACT ACT ATT ACT GTT GAT GAG ATT GAG CAA TTA GAA GCA AAT AAA ATC 1584
Thr Thr Ile Thr Val Asp Glu Ile Glu Gln Leu Glu Ala Asn Lys Ile
        515                 520                 525
GGT CAT CAA GCC 1596
Gly His Gln Ala
    530

## Claims

1. Genes coding for a group of eicosapentaenoic acid synthesis enzymes.

2. Genes according to claim 1, derived from a microorganism belonging to the genus Pseudomonas, Alteromonas or Shewanella.

3. Genes according to claim 1 having a nucleotide sequence shown in SEQ ID NO: 1.

4. Gene coding for an entire or a part of the amino acid sequence shown in SEQ ID NO: 2.

5. Gene coding for an entire or a part of the amino acid sequence shown in SEQ ID NO: 3.

6. Gene coding for an entire or a part of the amino acid sequence shown in SEQ ID NO: 4.

7. Gene coding for an entire or a part of the amino acid sequence shown in SEQ ID NO: 5.

8. Gene coding for an entire or a part of the amino acid sequence shown in SEQ ID NO: 6.

9. Gene coding for an entire or a part of the amino acid sequence shown in SEQ ID NO: 7.

10. Gene coding for an entire or a part of the amino acid sequence shown in SEQ ID NO: 8.

11. Gene coding for an entire or a part of the amino acid sequence shown in SEQ ID NO: 9.

12. Plasmid containing genes coding for a group of eicosapentaenoic acid biosynthesis enzymes.

13. Plasmid according to claim 3, wherein the genes coding for a group of eicosapentaenoic acid biosynthesis enzymes are derived from a microorganism belonging to the genus Pseudomonas, Alteromonas or Shewanella.

14. Plasmid containing at least one of the genes according to any one of claims 4 to 11.

15. An organism transformed with a plasmid containing genes coding for eicosapentaenoic acid synthesis enzymes.

16. An organism according to claim 15, wherein the transformed organism is Escherichia coli.

17. A process for production of eicosapentaenoic acid, characterized by culturing an organism transformed with a plasmid containing genes coding for eicosapentaenoic acid synthesis enzymes, and recovering eicosapentaenoic acid from the culture.
